(19) Europäisches Patentamt
European Patent Office
Office européen des brevets

(11) **EP 4 782 431 A1**

(12) **EUROPEAN PATENT APPLICATION**
published in accordance with Art. 153(4) EPC


(43) Date of publication:
**29.07.2026 Bulletin 2026/31**

(21) Application number: **24902786.3**

(22) Date of filing: **10.12.2024**

(51) International Patent Classification (IPC):
***C07D 209/88*** $^{(2006.01)}$ ***A61K 31/403*** $^{(2006.01)}$
***A61P 35/00*** $^{(2006.01)}$ ***A61P 17/00*** $^{(2006.01)}$

(52) Cooperative Patent Classification (CPC):
**A61K 31/403; A61P 1/00; A61P 1/02; A61P 3/00; A61P 9/00; A61P 11/00; A61P 17/00; A61P 19/00; A61P 21/00; A61P 25/00; A61P 31/00; A61P 35/00; A61P 37/00; C07D 209/88**

(86) International application number:
**PCT/CN2024/137995**

(87) International publication number:
**WO 2025/124365 (19.06.2025 Gazette 2025/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.12.2023 CN 202311694967**



(71) Applicant: **Suzhou Merna Therapeutics Co. Ltd**
**Suzhou, Jiangsu 215000 (CN)**

(72) Inventors:
- **PENG, Cheng**
**Suzhou, Jiangsu 215000 (CN)**
- **WANG, Jingfang**
**Suzhou, Jiangsu 215000 (CN)**
- **SONG, Zhidong**
**Suzhou, Jiangsu 215000 (CN)**
- **WANG, Ying**
**Suzhou, Jiangsu 215000 (CN)**
- **TONG, Siyu**
**Suzhou, Jiangsu 215000 (CN)**

(74) Representative: **Ter Meer Steinmeister & Partner Patentanwälte mbB**
**Nymphenburger Straße 4**
**80335 München (DE)**


(54) **CRYSTAL FORM OF RNA M6A REGULATOR, PREPARATION METHOD FOR CRYSTAL FORM AND USE THEREOF**


(57) A crystal form of an RNA m6A regulator of formula (I), a preparation method therefor and use thereof are provided. The crystal form of the RNA m6A regulator of the present disclosure has the beneficial effects of low hygroscopicity and good stability. The preparation method for the crystal form of the RNA m6A regulator of the present disclosure has a simple process, an easily controllable crystallization process, and good reproducibility.


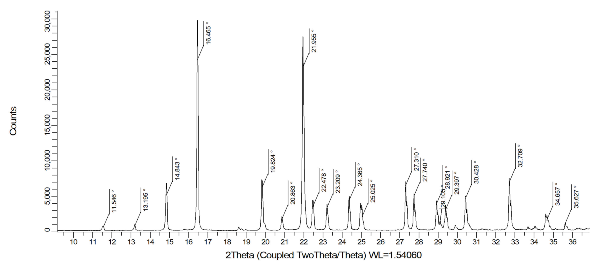


FIG. 36

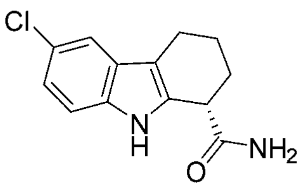

(I)

EP 4 782 431 A1

## Description

### CROSS REFERENCE TO RELATED APPLICATION

The present disclosure claims priority to Chinese patent application No. 202311694967.X, filed with the China National Intellectual Property Administration on December 11, 2023, entitled "CRYSTAL FORM OF RNA M6A REGULATOR, PREPARATION METHOD FOR CRYSTAL FORM AND USE THEREOF", the entire contents of which are incorporated herein by reference.

### TECHNICAL FIELD

The present disclosure belongs to the technical field of medicinal chemistry, and particularly relates to a crystal form of an RNA m6A regulator, a preparation method therefor and use thereof.

### BACKGROUND

A hand-foot syndrome (HFS) and a hand-foot skin reaction (HFSR) are erythematous skin lesions of palms and soles, which are mainly caused by cytotoxic chemotherapeutic drugs and tumor-targeted drugs, and in severe cases, may lead to loss of self-care ability in patients. Main pathological characteristics of the hand-foot syndrome and the hand-foot skin reaction include vacuolar degeneration of basal keratinocytes, infiltration of perivascular lymphocytes in the skin, apoptosis of keratinocytes, and cutaneous edema; and under a microscope, inflammatory changes, vasodilation, edema, and leukocyte infiltration can be observed.

Drugs that can cause the hand-foot syndrome include chemotherapeutic drugs such as capecitabine, liposomal doxorubicin, cytarabine, docetaxel, vinorelbine, continuously infused doxorubicin, and gemcitabine; and drugs that can cause the hand-foot skin reaction include targeted drugs such as sunitinib (Sutent), sorafenib (Nexavar), imatinib (Glivec), and erlotinib (Tarceva). HFS is classified by the World Health Organization (WHO) into 4 grades: Grade 1, dysesthesia, paresthesia, or tingling sensation in hands and feet; Grade 2, discomfort, painless swelling, or erythema when holding objects or walking; Grade 3, painful erythema, swelling of palms and soles, periungual erythema, and swelling; and Grade 4, desquamation, ulceration, blistering, and severe pain. Currently, severe symptoms of the hand-foot syndrome and the hand-foot skin reaction occurring clinically are often only alleviated by superficial skin care or even require discontinuation of medication. Existing treatments only alleviate the symptoms, but failed to address the underlying cause, thereby severely limiting the use of first-line chemotherapeutic drugs for tumors. Therefore, there are significant unmet medical demands. It is required to develop a drug with good efficacy, few side effects, and a low cost for preventing and treating the hand-foot syndrome and the hand-foot skin reaction to meet increasing global medical demands.

m6A methylation modification is the most prevalent RNA modification in mammals, which can regulate multiple signaling pathways and cellular processes (such as growth, development, and diseases), thus exerting critical biological functions (Frye M., et al. Science 2018, 361, 2073-2092; Yang C., et al. Cell Death & Disease 2020, 11, 960; Meyer K.D. & Jaffrey S.R. Nature Review Molecular Cell Biology 2014, 15, 313-326). The m6A methylation modification of mRNA, miRNA, circRNA, and lncRNA is a dynamically reversible process, in which methyltransferases (such as METTL3, METTL14, and METTL16) transfer a methyl group to RNA, while demethylases (FTO and ALKBH5) can remove the methyl group from RNA, thus establishing the regulatory basis of m6A modification. m6A affects processes, such as processing, translation, and degradation, of RNA by recruiting specific binding proteins (such as YTHDF1, YTHDF2, YTHDC1, and IGF2BP), thereby resulting in changes in downstream protein functions and cellular biological behaviors (Hsu P.J., et al. Journal of Biological Chemistry 2019, 294, 19889-19895; Yao Y., et al. FASEB Journal 2019, 33, 7529-7544).

Currently, there are few known direct associations between RNA m6A and skin-related diseases, and most of the m6A-related methyltransferases or demethylases play roles in skin diseases (especially skin tumors). For example, the mRNA expression level of METTL3 and ALKBH5 in tumor tissues of patients with acral melanoma are significantly higher than those in adjacent nontumor tissues, and the mRNA expression level of METTL3 in patients with advanced acral melanoma is significantly higher than that in early-stage patients (Le Zhanghui, Preliminary Study on the Pathogenesis of LncRNA and RNA m6A Methylation in Acral Melanoma, Dissertation, 2019). In the aspect of diagnosis of melanoma, m6A-specific binding proteins YTHDF1 and HNRNPA2B1 can serve as novel biomarkers (Li T. D., et al. Cancer Cell 2020, 20, 239). In the aspect of keratinocytes, it has only been reported currently that long-term and low-dose arsenic exposure can inhibit selective autophagy caused by the m6A demethylases, thereby inducing the occurrence of skin tumors (Cui Y. H., et al. Nature Communications 2021, 12, 2183). In summary, currently, there are no methods for preventing and treating skin diseases by regulating RNA m6A methylation.

In view of the above, the present disclosure is proposed hereby.

**SUMMARY**

Problems to be solved

In view of the unpredictable existing forms and amounts of polymorphic compounds, the present disclosure provides a crystal form of an RNA m6A regulator of formula (I), a preparation method therefor and use thereof.

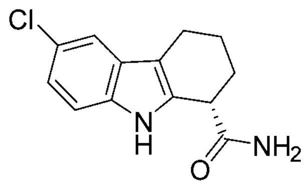

(I)

Technical solutions

The present disclosure provides a crystal form A of a compound of formula (I),

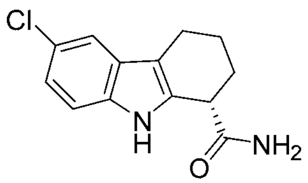

(I) ,

where an X-ray powder diffraction pattern expressed in terms of diffraction angle 2θ has characteristic peaks at 12.673, 18.557, 24.951, 27.029, and 33.461; preferably has characteristic peaks at 6.760, 12.673, 13.507, 18.557, 19.315, 21.594, 21.901, 22.214, 24.951, 27.029, and 33.461; preferably has characteristic peaks at 6.760, 12.673, 13.507, 18.557, 19.315, 20.380, 21.594, 21.901, 22.214, 24.774, 24.951, 26.125, 27.029, 33.461, 35.425, and 35.428; preferably has characteristic peaks at 5.547, 6.760, 12.326, 12.673, 13.507, 14.870, 15.162, 18.557, 19.315, 20.380, 21.183, 21.384, 21.594, 21.901, 22.214, 24.774, 24.951, 26.125, 27.029, 33.461, 35.425, and 35.428; and most preferably, the X-ray powder diffraction pattern expressed in terms of diffraction angle 2θ is as shown in FIG. 21.

The present disclosure provides a crystal form B of a compound of formula (I),

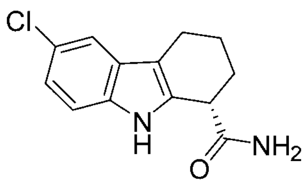

(I) ,

where an X-ray powder diffraction pattern expressed in terms of diffraction angle 2θ has characteristic peaks at 10.834, 13.544, 14.962, 21.722, and 30.594; preferably has characteristic peaks at 10.834, 13.544, 14.962, 21.722, 22.809, 23.822, 25.341, 26.812, 29.852, and 30.594; preferably has characteristic peaks at 6.766, 10.140, 10.834, 13.544, 14.962, 19.915, 20.580, 21.722, 22.809, 23.822, 25.341, 26.812, 27.231, 29.852, and 30.594; and most preferably, the X-ray powder diffraction pattern expressed in terms of diffraction angle 2θ is as shown in FIG. 24.

The present disclosure provides a crystal form C of a compound of formula (I),

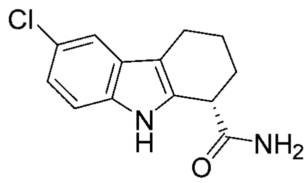

(I) ,

where an X-ray powder diffraction pattern expressed in terms of diffraction angle 2θ has characteristic peaks at 10.532, 11.778, 16.749, 19.015, and 21.131; preferably has characteristic peaks at 10.532, 11.778, 16.749, 17.300, 19.015, 21.131, 22.677, 23.402, 24.765, 26.993, and 28.564; preferably has characteristic peaks at 7.462, 10.095, 10.532, 11.778, 14.049, 14.686, 16.749, 17.300, 17.845, 19.015, 21.131, 22.677, 22.878, 23.402, 24.765, 26.993, and 28.564; and most preferably, the X-ray powder diffraction pattern expressed in terms of diffraction angle 2θ is as shown in FIG. 27.

The present disclosure provides a crystal form D of a compound of formula (I),

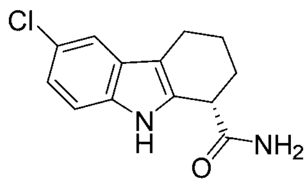

(I) ,

where an X-ray powder diffraction pattern expressed in terms of diffraction angle 2θ has characteristic peaks at 10.667, 17.319, 19.284, 21.379, and 25.317; preferably has characteristic peaks at 10.667, 15.726, 17.319, 18.045, 19.284, 19.959, 21.379, 25.317, 27.193, and 28.917; preferably has characteristic peaks at 10.667, 15.726, 16.493, 17.319, 18.045, 19.284, 19.959, 21.379, 23.201, 24.135, 25.317, 26.476, 27.108, 27.193, and 28.917; preferably has characteristic peaks at 9.632, 10.295, 10.667, 11.977, 15.726, 16.493, 17.319, 18.045, 18.341, 19.284, 19.959, 21.379, 23.201, 24.135, 25.317, 25.572, 26.476, 27.108, 27.193, and 28.917; and most preferably, the X-ray powder diffraction pattern expressed in terms of diffraction angle 2θ is as shown in FIG. 30.

The present disclosure provides a crystal form E of a compound of formula (I),

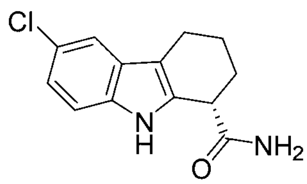

(I) ,

where an X-ray powder diffraction pattern expressed in terms of diffraction angle 2θ has characteristic peaks at 11.989, 16.248, 19.659, 19.973, and 23.014; preferably has characteristic peaks at 9.992, 11.989, 16.248, 19.414, 19.659, 19.973, 21.912, 22.448, 22.632, 23.014, 24.190, and 30.862; preferably has characteristic peaks at 9.992, 11.989, 16.248, 19.414, 19.659, 19.973, 21.175, 21.912, 22.448, 22.632, 23.014, 24.190, 25.095, 26.375, 30.862, 32.739, 34.159, and 37.010; preferably has characteristic peaks at 9.992, 11.989, 14.378, 16.248, 19.414, 19.659, 19.973, 21.175, 21.912, 22.448, 22.632, 23.014, 23.880, 24.190, 25.095, 26.375, 28.530, 30.862, 31.163, 32.174, 32.739, 34.159, and 37.010; and most preferably, the X-ray powder diffraction pattern expressed in terms of diffraction angle 2θ is as shown in FIG. 33.

The present disclosure provides a crystal form F of a compound of formula (I),

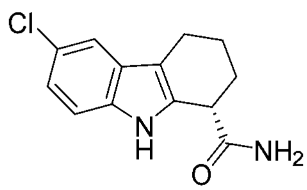

(I) ,

where an X-ray powder diffraction pattern expressed in terms of diffraction angle 2θ has characteristic peaks at 16.465, 19.824, 21.955, 27.310, and 32.709; preferably has characteristic peaks at 14.843, 16.465, 19.824, 21.955, 24.365, 27.310, 27.740, 28.921, 30.428, and 32.709; preferably has characteristic peaks at 11.546, 13.195, 14.843, 16.465, 19.824, 21.955, 22.478, 23.209, 24.365, 27.310, 27.740, 28.921, 29.397, 30.428, and 32.709; and most preferably, the X-ray powder diffraction pattern expressed in terms of diffraction angle 2θ is as shown in FIG. 36.

The present disclosure further provides a crystal form G of a compound of formula (I),

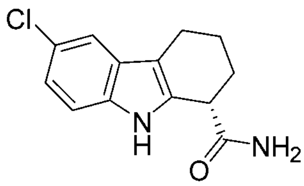

(I) ,

where an X-ray powder diffraction pattern expressed in terms of diffraction angle 2θ has characteristic peaks at 16.482, 19.844, 21.972, 27.762, 30.447, and 32.731; preferably has characteristic peaks at 14.858, 16.482, 19.844, 21.972, 24.388, 24.992, 27.333, 27.762, 28.938, 30.447, and 32.731; preferably has characteristic peaks at 11.539, 13.207, 14.858, 15.780, 16.482, 19.844, 21.972, 22.490, 24.388, 24.992, 27.333, 27.762, 28.938, 29.412, 30.447, and 32.731; and most preferably, the X-ray powder diffraction pattern expressed in terms of diffraction angle 2θ is as shown in FIG. 39.

Technical effects

The crystal form A, the crystal form B, the crystal form C, the crystal form D, the crystal form E, the crystal form F, and the crystal form G of the compound of formula (I) provided by the present disclosure have advantages in aspects such as physicochemical properties, preparation processability, and bioavailability, and for example, have advantages in at least one of aspects such as melting point, solubility, hygroscopicity, purification effect, stability, adhesiveness, compressibility, flowability, *in vivo* and *in vitro* dissolution, and biological effectiveness. The free crystal forms of the compound of formula (I) in the present disclosure have good physical and chemical self-stability, a high crystal form yield when prepared from the same starting materials, and obvious advantages in aspects such as solubility, hygroscopicity, stability, mechanical stability, flowability, compressibility, and adhesiveness, thus providing new and better options for the development of RNA m6A regulator drugs and having great significance.

**BRIEF DESCRIPTION OF THE DRAWINGS**

FIG. 1 describes exemplary results of a cellular m6A level and an NAP1L2 m6A level determined by nanopore sequencing after administration of sorafenib, capecitabine, docetaxel, and osimertinib to HaCaT keratinocytes for 24 hours.

FIG. 2 describes exemplary results of a cellular m6A level and an NAP1L2 m6A level in skin tissues of hind limb toes of mice in Example 3.

FIG. 3 describes exemplary results of an NAP1L2 mRNA expression amount determined by reverse transcription-polymerase chain reaction (RT-PCR) after administration of a recombinant human METTL3 protein and a recombinant human FTO protein or transfection with METTL3 siRNA and FTO siRNA to HaCaT keratinocytes for 24 hours.

FIG. 4 describes exemplary results of mRNA and protein expression amounts of matrix metalloproteinases determined by RT-PCR and Western blot after administration of a recombinant human NAP1L2 protein, sorafenib, capecitabine, docetaxel, and osimertinib or transfection with NAP1L2 siRNA to HaCaT keratinocytes for 24 hours.

FIG. 5 describes exemplary results of mRNA and protein expression amounts of keratinocyte differentiation markers KRT1, KRT10, Loricrin, and Involucrin determined by RT-PCR and Western blot after administration of a recombinant human HBEGF protein and an HBEGF antibody or transfection with METTL3 and NAP1L2 siRNA to HaCaT keratinocytes for 24 hours.

FIG. 6 describes exemplary results of a cellular m6A methylation inhibition level determined by liquid chromatography-mass spectrometry (LC-MS) after administration of a compound of formula (I), nicotinamide, and UZH2 to THP-1 cells for 24 hours.

FIG. 7 describes exemplary results of mRNA expression amounts of keratinocyte differentiation markers KRT1, KRT10, Loricrin, and Involucrin determined by RT-PCR after administration of a recombinant human HBEGF protein and the compound of formula (I) to HaCaT keratinocytes for 24 hours.

FIG. 8 describes exemplary stratum corneum thickness measurement results of paw plantar skin of rats in Example 10.

FIG. 9 describes exemplary histopathological results of epithelial blisters, inflammatory cell infiltration, and skin tissue congestion situations of paw plantar skin of rats after tissue staining in Example 10.

FIG. 10 describes exemplary histopathological scoring results of the epithelial blisters, the inflammatory cell infiltration, and the skin tissue congestion situations of the paw plantar skin of the rats after tissue staining in Example 10.

FIG. 11 describes exemplary results of the skin of hind limb toes of mice in Example 11.

FIG. 12 describes exemplary results of the degree of swelling of hind limb toes of mice in Example 11.

FIG. 13 describes exemplary histopathological results of skin tissues of hind limb toes of mice in Example 11.

FIG. 14 describes exemplary immunohistochemical results of cytokine IL-8 in skin tissues of hind limb toes of mice in capecitabine modeling series groups in Example 11.

FIG. 15 describes exemplary immunohistochemical results of cytokine IL-6 in skin tissues of hind limb toes of mice in docetaxel modeling series groups in Example 11.

FIG. 16 describes exemplary results of the skin of hind limb toes of mice in Example 12.

FIG. 17 describes exemplary results of the degree of swelling of hind limb toes of mice in Example 12.

FIG. 18 describes exemplary histopathological results of skin tissues of hind limb toes of mice in Example 12.

FIG. 19 describes exemplary immunohistochemical results of cytokine IL-1β in skin tissues of hind limb toes of mice in sorafenib modeling series groups in Example 12.

FIG. 20 describes exemplary immunohistochemical results of cytokine IL-1β in skin tissues of hind limb toes of mice in osimertinib modeling series groups in Example 12.

FIG. 21 is an X-ray powder diffraction (XRPD) pattern of a crystal form A of the compound of formula (I).

FIG. 22 is a thermogravimetric analysis (TGA) spectrogram of the crystal form A of the compound of formula (I).

FIG. 23 is a differential scanning calorimetry (DSC) spectrogram of the crystal form A of the compound of formula (I).

FIG. 24 is an XRPD pattern of a crystal form B of the compound of formula (I).

FIG. 25 is a TGA spectrogram of the crystal form B of the compound of formula (I).

FIG. 26 is a DSC spectrogram of the crystal form B of the compound of formula (I).

FIG. 27 is an XRPD pattern of a crystal form C of the compound of formula (I).

FIG. 28 is a TGA spectrogram of the crystal form C of the compound of formula (I).

FIG. 29 is a DSC spectrogram of the crystal form C of the compound of formula (I).

FIG. 30 is an XRPD pattern of a crystal form D of the compound of formula (I).

FIG. 31 is a TGA spectrogram of the crystal form D of the compound of formula (I).

FIG. 32 is a DSC spectrogram of the crystal form D of the compound of formula (I).

FIG. 33 is an XRPD pattern of a crystal form E of the compound of formula (I).

FIG. 34 is a TGA spectrogram of the crystal form E of the compound of formula (I).

FIG. 35 is a DSC spectrogram of the crystal form E of the compound of formula (I).

FIG. 36 is an XRPD pattern of a crystal form F of the compound of formula (I).

FIG. 37 is a TGA spectrogram of the crystal form F of the compound of formula (I).

FIG. 38 is a DSC spectrogram of the crystal form F of the compound of formula (I).

FIG. 39 is an XRPD pattern of a crystal form G of the compound of formula (I).

FIG. 40 is a TGA spectrogram of the crystal form G of the compound of formula (I).

FIG. 41 is a DSC spectrogram of the crystal form G of the compound of formula (I).

## DETAILED DESCRIPTION OF THE EMBODIMENTS

To make the technical solutions and beneficial effects of the present disclosure more apparent and understandable, the following provides a detailed description by the way of listing specific examples. The drawings are not necessarily drawn to scale, and local features may be enlarged or reduced to more clearly show details of the local features. Unless otherwise defined, technical and scientific terms used herein have the same meanings as those in the technical field to which the present disclosure belongs.

The present disclosure provides a crystal form A of a compound of formula (I),

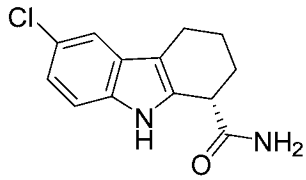

(I)

,

where an X-ray powder diffraction pattern expressed in terms of diffraction angle 2θ has characteristic peaks at 12.673, 18.557, 24.951, 27.029, and 33.461; preferably has characteristic peaks at 6.760, 12.673, 13.507, 18.557, 19.315, 21.594, 21.901, 22.214, 24.951, 27.029, and 33.461; preferably has characteristic peaks at 6.760, 12.673, 13.507, 18.557, 19.315, 20.380, 21.594, 21.901, 22.214, 24.774, 24.951, 26.125, 27.029, 33.461, 35.425, and 35.428; preferably has characteristic peaks at 5.547, 6.760, 12.326, 12.673, 13.507, 14.870, 15.162, 18.557, 19.315, 20.380, 21.183, 21.384, 21.594, 21.901, 22.214, 24.774, 24.951, 26.125, 27.029, 33.461, 35.425, and 35.428; and most preferably, the X-ray powder diffraction pattern expressed in terms of diffraction angle 2θ is as shown in FIG. 21.

The present disclosure further provides a method for preparing the crystal form A of the compound of formula (I), including: mixing the compound of formula (I) with a solvent 1, stirring, and filtering.

In some embodiments, the solvent 1 is selected from an alcohol solvent.

In some embodiments, the solvent 1 is selected from a $C_{1-4}$ alcohol.

In some embodiments, the solvent 1 is selected from one or more of methanol, ethanol, and isopropanol.

In some embodiments, the solvent 1 is selected from ethanol.

In some embodiments, the preparation method of the present disclosure further includes steps such as centrifugation, washing, or drying.

The present disclosure further provides a crystal form B of a compound of formula (I),

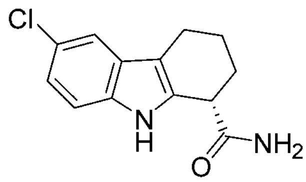

(I)

,

where an X-ray powder diffraction pattern expressed in terms of diffraction angle 2θ has characteristic peaks at 10.834, 13.544, 14.962, 21.722, and 30.594; preferably has characteristic peaks at 10.834, 13.544, 14.962, 21.722, 22.809, 23.822, 25.341, 26.812, 29.852, and 30.594; preferably has characteristic peaks at 6.766, 10.140, 10.834, 13.544, 14.962, 19.915, 20.580, 21.722, 22.809, 23.822, 25.341, 26.812, 27.231, 29.852, and 30.594; and most preferably, the X-ray powder diffraction pattern expressed in terms of diffraction angle 2θ is as shown in FIG. 24.

The present disclosure further provides a method for preparing the crystal form B of the compound of formula (I), including: dissolving the compound of formula (I) in a solvent 2, and adding an antisolvent 3 to precipitate a crystal.

In some embodiments, the solvent 2 is selected from an alcohol solvent.

In some embodiments, the solvent 2 is selected from a $C_{1-4}$ alcohol.

In some embodiments, the solvent 2 is selected from one or more of methanol, ethanol, and isopropanol.

In some embodiments, the solvent 2 is selected from methanol.

In some embodiments, the antisolvent 3 is selected from water.

In some embodiments, the preparation method of the present disclosure further includes steps such as centrifugation, washing, or drying.

The present disclosure further provides a crystal form C of a compound of formula (I),

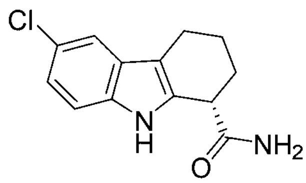

(I)

,

where an X-ray powder diffraction pattern expressed in terms of diffraction angle 2θ has characteristic peaks at 10.532, 11.778, 16.749, 19.015, and 21.131; preferably has characteristic peaks at 10.532, 11.778, 16.749, 17.300, 19.015, 21.131, 22.677, 23.402, 24.765, 26.993, and 28.564; preferably has characteristic peaks at 7.462, 10.095, 10.532, 11.778, 14.049, 14.686, 16.749, 17.300, 17.845, 19.015, 21.131, 22.677, 22.878, 23.402, 24.765, 26.993, and 28.564; and most preferably, the X-ray powder diffraction pattern expressed in terms of diffraction angle 2θ is as shown in FIG. 27.

The present disclosure further provides a method for preparing the crystal form C of the compound of formula (I), including: dissolving the compound of formula (I) in a solvent 4, and adding an antisolvent 5 to precipitate a crystal.

In some embodiments, the solvent 4 is selected from an ester solvent.

In some embodiments, the solvent 4 is selected from one or more of ethyl acetate, methyl acetate, butyl acetate, and isobutyl acetate.

In some embodiments, the solvent 4 is selected from ethyl acetate.

In some embodiments, the antisolvent 5 is selected from an alkane solvent.

In some embodiments, the antisolvent 5 is selected from one or more of n-hexane, cyclohexane, pentane, dimethylpentane, and n-heptane.

In some embodiments, the antisolvent 5 is selected from n-heptane.

In some embodiments, the preparation method of the present disclosure further includes steps such as centrifugation, washing, or drying.

The present disclosure further provides a crystal form D of a compound of formula (I),

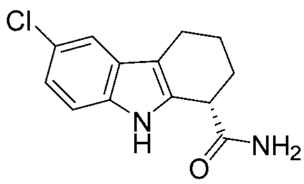

(I) ,

where an X-ray powder diffraction pattern expressed in terms of diffraction angle 2θ has characteristic peaks at 10.667, 17.319, 19.284, 21.379, and 25.317; preferably has characteristic peaks at 10.667, 15.726, 17.319, 18.045, 19.284, 19.959, 21.379, 25.317, 27.193, and 28.917; preferably has characteristic peaks at 10.667, 15.726, 16.493, 17.319, 18.045, 19.284, 19.959, 21.379, 23.201, 24.135, 25.317, 26.476, 27.108, 27.193, and 28.917; preferably has characteristic peaks at 9.632, 10.295, 10.667, 11.977, 15.726, 16.493, 17.319, 18.045, 18.341, 19.284, 19.959, 21.379, 23.201, 24.135, 25.317, 25.572, 26.476, 27.108, 27.193, and 28.917; and most preferably, the X-ray powder diffraction pattern expressed in terms of diffraction angle 2θ is as shown in FIG. 30.

The present disclosure further provides a method for preparing the crystal form D of the compound of formula (I), including: mixing the compound of formula (I) with a solvent 6, heating for dissolution, cooling, and filtering.

In some embodiments, the solvent 6 is selected from a ketone solvent.

In some embodiments, the solvent 6 is selected from acetone, butanone, pentanone, hexanone, and cyclohexanone.

In some embodiments, the solvent 6 is selected from acetone.

In some embodiments, the preparation method of the present disclosure further includes steps such as centrifugation, washing, or drying.

The present disclosure further provides a crystal form E of a compound of formula (I),

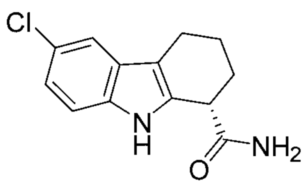

(I) ,

where an X-ray powder diffraction pattern expressed in terms of diffraction angle 2θ has characteristic peaks at 11.989, 16.248, 19.659, 19.973, and 23.014; preferably has characteristic peaks at 9.992, 11.989, 16.248, 19.414, 19.659, 19.973, 21.912, 22.448, 22.632, 23.014, 24.190, and 30.862; preferably has characteristic peaks at 9.992, 11.989, 16.248, 19.414, 19.659, 19.973, 21.175, 21.912, 22.448, 22.632, 23.014, 24.190, 25.095, 26.375, 30.862, 32.739, 34.159, and 37.010; preferably has characteristic peaks at 9.992, 11.989, 14.378, 16.248, 19.414, 19.659, 19.973, 21.175, 21.912, 22.448,

22.632, 23.014, 23.880, 24.190, 25.095, 26.375, 28.530, 30.862, 31.163, 32.174, 32.739, 34.159, and 37.010; and most preferably, the X-ray powder diffraction pattern expressed in terms of diffraction angle 2θ is as shown in FIG. 33.

The present disclosure further provides a method for preparing the crystal form E of the compound of formula (I), including: mixing the compound of formula (I) with a solvent 7, stirring, and filtering.

In some embodiments, the solvent 7 is selected from an ether solvent.

In some embodiments, the solvent 7 is selected from one or more of ethyl ether, propyl ether, butyl ether, anisole, petroleum ether, isopropyl ether, and 1,4-dioxane.

In some embodiments, the solvent 7 is selected from 1,4-dioxane.

In some embodiments, the preparation method of the present disclosure further includes steps such as centrifugation, washing, or drying.

The present disclosure further provides a crystal form F of a compound of formula (I),

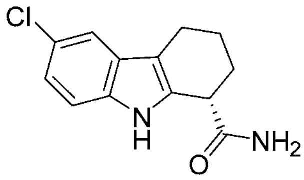

(I) ,

where an X-ray powder diffraction pattern expressed in terms of diffraction angle 2θ has characteristic peaks at 16.465, 19.824, 21.955, 27.310, and 32.709; preferably has characteristic peaks at 14.843, 16.465, 19.824, 21.955, 24.365, 27.310, 27.740, 28.921, 30.428, and 32.709; preferably has characteristic peaks at 11.546, 13.195, 14.843, 16.465, 19.824, 21.955, 22.478, 23.209, 24.365, 27.310, 27.740, 28.921, 29.397, 30.428, and 32.709; and most preferably, the X-ray powder diffraction pattern expressed in terms of diffraction angle 2θ is as shown in FIG. 36.

The present disclosure further provides a method for preparing the crystal form F of the compound of formula (I), including: mixing the compound of formula (I) with a solvent 8, heating for dissolution, cooling, and filtering.

In some embodiments, the solvent 8 is selected from an alcohol solvent.

In some embodiments, the solvent 8 is selected from a $C_{1-4}$ alcohol.

In some embodiments, the solvent 8 is selected from methanol, ethanol, and isopropanol.

In some embodiments, the solvent 8 is selected from ethanol.

In some embodiments, the preparation method of the present disclosure further includes steps such as centrifugation, washing, or drying.

The present disclosure further provides a crystal form G of a compound of formula (I),

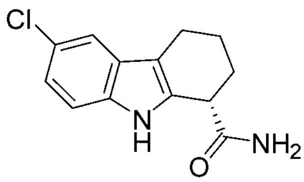

(I) ,

where an X-ray powder diffraction pattern expressed in terms of diffraction angle 2θ has characteristic peaks at 16.482, 19.844, 21.972, 27.762, 30.447, and 32.731; preferably has characteristic peaks at 14.858, 16.482, 19.844, 21.972, 24.388, 24.992, 27.333, 27.762, 28.938, 30.447, and 32.731; preferably has characteristic peaks at 11.539, 13.207, 14.858, 15.780, 16.482, 19.844, 21.972, 22.490, 24.388, 24.992, 27.333, 27.762, 28.938, 29.412, 30.447, and 32.731; and most preferably, the X-ray powder diffraction pattern expressed in terms of diffraction angle 2θ is as shown in FIG. 39.

The present disclosure further provides a method for preparing the crystal form G of the compound of formula (I), including: mixing the compound of formula (I) with a solvent 9 and a solvent 10, stirring for dissolution, continually adding the solvent 10 and adding a seed crystal, stirring, continually adding the solvent 10, stirring, and filtering.

In some embodiments, the solvent 9 is selected from a ketone solvent.

In some embodiments, the solvent 9 is selected from one or more of acetone, butanone, pentanone, hexanone, and cyclohexanone.

In some embodiments, the solvent 9 is selected from acetone.

In some embodiments, the solvent 10 is selected from water.

In some embodiments, the preparation method of the present disclosure further includes steps such as

centrifugation, washing, or drying.

The present disclosure further provides a pharmaceutical composition prepared from the aforementioned crystal form A of the compound of formula (I), the aforementioned crystal form B of the compound of formula (I), the aforementioned crystal form C of the compound of formula (I), the aforementioned crystal form D of the compound of formula (I), the aforementioned crystal form E of the compound of formula (I), the aforementioned crystal form F of the compound of formula (I), or the aforementioned crystal form G of the compound of formula (I).

The present disclosure further provides a pharmaceutical composition, which includes the aforementioned crystal form A of the compound of formula (I), the aforementioned crystal form B of the compound of formula (I), the aforementioned crystal form C of the compound of formula (I), the aforementioned crystal form D of the compound of formula (I), the aforementioned crystal form E of the compound of formula (I), the aforementioned crystal form F of the compound of formula (I), or the aforementioned crystal form G of the compound of formula (I), and optionally a pharmaceutically acceptable excipient.

The "pharmaceutically acceptable excipient" refers to a pharmaceutically acceptable material, mixture, or vehicle related to the consistency of a dosage form or the pharmaceutical composition. Suitable pharmaceutically acceptable excipients are different according to selected dosage forms. Furthermore, the pharmaceutically acceptable excipients may be selected according to their specific functions in the composition.

In some embodiments, the pharmaceutically acceptable excipient includes excipients of the following types: a diluent, a filler, a penetration enhancer, a binder, a disintegrant, a lubricant, a glidant, a granulating agent, a coating agent, a wetting agent, a solvent, a cosolvent, a suspending aid, an emulsifier, a flavoring agent, a taste-masking agent, a colorant, an anti-caking agent, a humectant, a chelating agent, a plasticizer, a tackifier, an antioxidant, a preservative, a stabilizer, a surfactant, and a buffer.

In some embodiments, the pharmaceutical composition is a solid preparation.

In some embodiments, the pharmaceutical composition is a film and a coated film.

In some embodiments, the pharmaceutical composition is an ointment.

In some embodiments, the pharmaceutical composition is a plaster.

In some embodiments, the pharmaceutical composition is a cream.

In some embodiments, the solid preparation is a capsule.

In some embodiments, the pharmaceutical composition is a tablet.

In some embodiments, the ointment contains the compound of formula (I) in an amount of 0-50% by weight.

The present disclosure further provides a method for preparing a pharmaceutical composition, which includes a step of mixing the aforementioned crystal form A of the compound of formula (I), the aforementioned crystal form B of the compound of formula (I), the aforementioned crystal form C of the compound of formula (I), the aforementioned crystal form D of the compound of formula (I), the aforementioned crystal form E of the compound of formula (I), the aforementioned crystal form F of the compound of formula (I), or the aforementioned crystal form G of the compound of formula (I) with a pharmaceutically acceptable excipient.

The present disclosure further provides use of the aforementioned crystal form A of the compound of formula (I), the aforementioned crystal form B of the compound of formula (I), the aforementioned crystal form C of the compound of formula (I), the aforementioned crystal form D of the compound of formula (I), the aforementioned crystal form E of the compound of formula (I), the aforementioned crystal form F of the compound of formula (I), the aforementioned crystal form G of the compound of formula (I), the aforementioned composition, or a composition prepared by the aforementioned method in preparation of a drug for treating and/or preventing a disease related to RNA m6A regulation.

In some embodiments, the disease related to RNA m6A regulation is a disease or pain related to endocrine and metabolic diseases, neurological diseases, tumors, cardiovascular diseases, infections, immune system diseases, urogenital diseases, dermatological and musculoskeletal diseases, respiratory diseases, genetic diseases and malformations, digestive system diseases, stomatognathic diseases, and vascular and lymphatic system diseases.

In some embodiments, the disease related to RNA m6A regulation is a hand-foot syndrome, a hand-foot skin reaction, a cancer, or a dermatological disease.

In the present disclosure, the "$2\theta$ or $2\theta$ angle" refer to the diffraction angle, where $\theta$ is a Bragg angle, in the unit of ° or degree; and an error margin for each characteristic peak at $2\theta$ is $\pm 0.2$ degrees (including cases where numbers with more than 1 decimal place are rounded), which may be -0.20, - 0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, a precipitation manner includes, but is not limited to, stirring, cooling, volatilization, slurrying, and precipitation.

The "slurrying" is a commonly used noun in the field of pharmaceutical preparation, generally referring to mechanical or fluidic processing of a solid pharmaceutical raw material to disperse or suspend a solid drug in a solvent.

In some embodiments, a slurrying time is 5-30 h.

According to the description of hygroscopicity characteristics and the definition of hygroscopic weight gain in the

"9103 Guidelines for the Hygroscopicity of Pharmaceuticals" in Part IV of the 2020 Edition of the Chinese Pharmacopoeia,

deliquescence represents absorption of a sufficient amount of water to form a liquid;
high hygroscopicity represents a hygroscopic weight gain not less than 15%;
hygroscopicity represents a hygroscopic weight gain less than 15% but not less than 2%;
slight hygroscopicity represents a hygroscopic weight gain less than 2% but not less than 0.2%; and
no hygroscopicity or almost no hygroscopicity represents a hygroscopic weight gain less than 0.2%.

In the present disclosure, the "differential scanning calorimetry or DSC" refers to measuring a temperature difference and a heat flow difference between a sample and a reference during a heating or constant temperature process of the sample to characterize all physical changes and chemical changes related to a thermal effect and obtain phase transition information of the sample.

In the present disclosure, a drying temperature is generally 25°C-100°C, preferably 40°C-70°C, and the drying may be drying under ambient pressure or drying under reduced pressure.

The methods of the present disclosure are described below through specific examples. It should be understood that these examples are used to illustrate basic principles, main features, and advantages of the present disclosure, and the scope of the present disclosure is not limited to the following examples. Implementation conditions used in the examples may be further adjusted according to specific requirements, and unspecified implementation conditions usually are conditions in conventional experiments.

Explanations of abbreviations used in the present disclosure are as follows:

XRPD: X-ray powder diffraction;
DSC: differential scanning calorimetry;
TGA: thermogravimetric analysis;
DVS: dynamic vapor sorption; and
HPLC: high performance liquid chromatography.
Detection instruments and methods
X-ray powder diffraction (XRPD)

Crystal form analysis of a sample was performed using a Bruker D8 ADVANCE X-ray powder diffractometer. A 2θ scanning angle of the sample was 3°-40°, a scanning step size was 0.02°, and a scanning time per step was 0.12 s/step. An optical tube voltage and a current were 40 kV and 40 mA, respectively. During sample preparation, an appropriate amount of the sample was placed on a sample tray and flattened with a tool such as a glass slide to ensure a smooth and even surface.

Thermogravimetric analysis (TGA)

TA Instruments TGA Discovery 5500 was used for analysis of a sample. The sample was placed in a tared aluminum tray, automatically weighed by the system, and then heated to a specified temperature at a rate of 10°C/min under the protection of nitrogen.

Differential scanning calorimetry (DSC)

TA Instruments Discovery 2500 was used for analysis of a sample. 0.5-1.5 mg of the sample was weighed, placed in a sample tray, and then heated to a specified temperature at a rate of 10°C/min under the protection of nitrogen (50 ml/min).

Dynamic vapor sorption analysis (DVS)

ProUmid SPSx-1μ Advance was used for analysis of a sample. The sample to be tested was approximately in an amount of 5-50 mg. A temperature in a test chamber was controlled at 25±1°C, a relative humidity cycle was 40-95-0-95-40%, a step size was 10%, an equilibrium time was 240 minutes per step, and mass data was recorded every 20 seconds.

High performance liquid chromatography (HPLC)

An Agilent 1260 infinityII binary pump was used for solubility and stability testing.

**Example 1: Preparation of the compound of formula (I)**

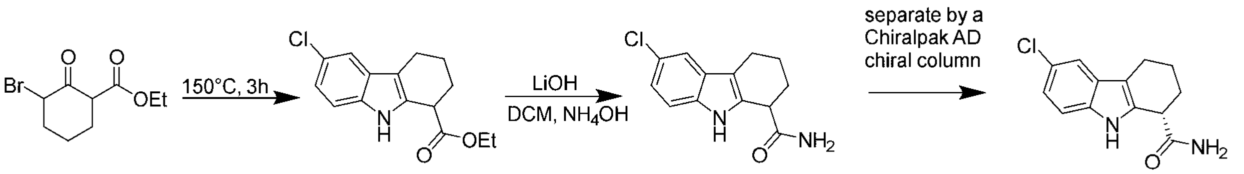


compound **1**

compound of formula (**I**)

Ethyl 3-bromo-2-oxocyclohexane-1-formate (20 mg, 0.08 mmol) and 4-chloroaniline (25 mg, 0.2 mmol) were mixed and then heated to 150°C for a reaction for 3 hours. The reaction solution was cooled to room temperature, diluted with 100 mL of dichloromethane, washed 3 times with 100 mL of 1 N HCl, and washed 1 time with 100 mL of saturated $NaHCO_3$. The organic layer was dried over anhydrous $Na_2SO_4$, concentrated under vacuum, and separated and purified by silica gel column chromatography to obtain ethyl 6-chloro-2,3,4,9-tetrahydro-1H-carbazole-1-formate (16 mg). 10 mg of the ethyl 6-chloro-2,3,4,9-tetrahydro-1H-carbazole-1-formate (0.036 mmol) was weighed and dissolved in 10 mL of ethanol, 2 mL of a 2 M LiOH solution was added, and the mixture was stirred at room temperature for a reaction for 1 hour. After rotary evaporation, the mixture was diluted with 20 mL of water, adjusted to a pH of 2, and then extracted 3 times with 50 mL of dichloromethane. The organic phases were combined, dried, and concentrated, followed by the addition of ammonia water and heating at 60°C for a reaction for 24 hours. The mixture was separated and purified by silica gel column chromatography to obtain 6-chloro-2,3,4,9-tetrahydro-1H-carbazole-1-carboxamide as a white solid (compound 1, 7.6 mg), which was separated by a Chiralpak AD chiral column to obtain (S)-6-chloro-2,3,4,9-tetrahydro-1H-carbazole-carboxamide (the compound of formula (I)). LCMS $[M+H]^+$ of the compound of formula (I): 249. $^1H$-NMR (400 MHz, DMSO-$d_6$): $\delta$ 10.79 (s, 1H), 7.37-7.39 (m, 2H), 7.28 (d, $J$ = 8.0 Hz, 1H), 7.08 (s, 1H), 6.98-7.01 (m, 1H), 3.64-3.67 (m, 1H), 2.58-2.61 (m, 2H), 1.66-2.04 (m, 4H).

**Example 2:** Changes in an mRNA methylation level of drug-induced keratinocytes

$1*10^8$ HaCaT cells were seeded in culture dishes, into which 50 nM drugs (including sorafenib, capecitabine, docetaxel, and osimertinib) were added, respectively. The cells were cultured in an incubator containing 5% $CO_2$ at 37°C for 24 hours and washed with PBS after culture media were removed. Centrifugation was performed, the supernatant was discarded, and then, 400 $\mu$L of an RLT cell lysis buffer (QIAGEN, Cat.#79216) was added. A portion of samples were selected, into which 400 $\mu$L of 70% ethanol was added. The mixture was evenly stirred and then transferred to an RNeasy mini column, and RNA was extracted using an RNeasy mini kit (QIAGEN, Cat.#74104). 2 $\mu$L of an RNA sample was selected and dissolved in 98 $\mu$L of a 10 mM Tris-HCl buffer solution, and the absorbance at 260 nm was measured using a Nanodrop (Thermo Fisher Scientific) to determine the RNA concentration.

40 $\mu$g of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a template to synthesize a complementary strand, a terminal end of the reverse transcription adapter RTA was ligated with a sequencing adapter (RNA adapter) to form a final sequencing library, and quantitative detection was performed using a Qubit™ dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

Results are shown in FIG. 1. Compared with a blank control group, the m6A methylation levels of HaCaT cells were 5.05-fold, 6.01-fold, 3.97-fold and 5.13-fold higher than that of the control group after the addition of the sorafenib, the capecitabine, the docetaxel, and the osimertinib, respectively. This indicates that antitumor drugs (including the sorafenib, the capecitabine, the docetaxel, and the osimertinib) induced the abnormal m6A methylation of mRNA of human keratinocytes. It is further found through bioinformatics analysis that the m6A methylation level of NAP1L2 had a significant difference, mainly showing in the m6A methylation of a UGAGGACUCA fragment.

**Example 3:** Changes in an mRNA methylation level in drug-induced mouse models of adverse skin reactions

After adaptive feeding for 7 days, male ICR mice (5-6 weeks old, with a body weight of approximately 35 g) were randomly divided into groups, with 6 mice in each group, including a blank control group, a sorafenib group, a capecitabine group, a docetaxel group, and an osimertinib group. The groups were respectively administered corresponding modeling drugs by gavage (sorafenib at 100 mg/kg, capecitabine at 200 mg/kg, docetaxel at 25 mg/kg, and osimertinib at 10 mg/kg) once daily. After continuous administration for 30 days, the mice were sacrificed, and skin tissues of hind limb toes of the mice were collected. A small amount of a tissue sample was selected, placed in a mortar containing liquid nitrogen, and ground into powder. A single-phase lysis buffer was added. After being placed at room temperature for 5 minutes, the sample was centrifuged (12,000 rpm) for 5 minutes. 1 mL of the supernatant was collected, 200 μL of chloroform was added, and the mixture was evenly mixed by shaking, placed at room temperature for 15 minutes, and centrifuged (12,000 rpm, 4°C) for 15 minutes. An aqueous phase on an upper layer was sucked, added into an equal volume of isopropanol, placed at -20°C for 1 hour, and then centrifuged (12,000 rpm, 4°C) to discard the supernatant. 400 μL of 70% ethanol was added to a precipitate, the mixture was evenly stirred and then transferred to an RNeasy mini column, and RNA was extracted using an RNeasy mini kit (QIAGEN, Cat.#74104). 2 μL of an RNA sample was selected and dissolved in 98 μL of a 10 mM Tris-HCl buffer solution, and the absorbance at 260 nm was measured using a Nanodrop (Thermo Fisher Scientific) to determine the RNA concentration.

40 μg of an RNA sample was selected for enrichment of mRNA (PolyA+RNA) using an NEBNext Poly(A) mRNA magnetic isolation module (NEB, Cat.#E7490), and then, a 3' end of the above enriched mRNA (PolyA+RNA) molecule was ligated with reverse transcription adapter RTA using a direct RNA sequencing kit (Oxford Nanopore Technologies, Cat.#SQK-RNA002). A reverse transcription reaction was performed with the mRNA (PolyA+RNA) molecule as a template to synthesize a complementary strand, a terminal end of the reverse transcription adapter RTA was ligated with a sequencing adapter (RNA adapter) to form a final sequencing library, and quantitative detection was performed using a Qubit™ dsDNA HS assay kit (Invitrogen, Cat.#LOT2133187).

After quality inspection of the library, the prepared sequencing library was loaded onto a PromethION flow cell chip (Oxford Nanopore Technologies, Cat.#FLO-MIN106D), and placed in a PromethION sequencer (Oxford Nanopore Technologies) for sequencing by selecting a matched sequencing mode. Basecalling was performed on sequencing data through guppy, and m6A methylation analysis was performed on original fast5 data.

Results are shown in FIG. 2. Compared with a positive control group, the m6A methylation levels of skin tissues of hind limb toes of the mice in the drug modeling groups were significantly increased. The m6A methylation level of NAP1L2 in the drug addition groups had a significant difference from the blank control group.

**Example 4:** Regulation of an mRNA expression level of NAP1L2 by m6A

1*10$^8$ HaCaT cells were seeded in culture dishes, into which a recombinant protein of m6A methyltransferase METTL3 (Abcam, Cat.#ab271611) and a recombinant protein of m6A demethylase FTO (Abcam, Cat.#ab271525) were added, or METTL3 siRNA (sh-METTL3, GeneChem) and FTO siRNA (sh-FTO, GeneChem) were transfected, respectively. The cells were placed in an incubator containing 5% carbon dioxide for culture at 37°C for 24 hours, washed with PBS, and then collected by high-speed centrifugation. Total RNA was extracted using Trizol reagent (Sigma Aldrich). 1 μg of RNA was reverse-transcribed into cDNA using a cDNA reverse transcription kit (Transgene Biotech, Cat.#AT311-03). 1.25 μL of a primer (Beyotime, Cat.#QH18721S), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of NAP1L2.

Results are shown in FIG. 3. When the m6A methyltransferase METTL3 was added to the HaCaT keratinocytes or the function of the m6A demethylase FTO was inhibited (via sh-FTO), the mRNA expression level of NAP1L2 was significantly increased; and when the m6A demethylase FTO was added to the HaCaT keratinocytes or a function of the m6A methyltransferase METTL3 was inhibited (via sh-METTL3), the mRNA expression level of NAP1L2 was significantly decreased. This indicates that m6A methylation regulated the mRNA expression amount of NAP1L2.

**Example 5:** Regulation of the expression of matrix metalloproteinases (MMPs) in human HaCaT keratinocytes by NAP1L2

1*10$^8$ HaCaT cells were seeded in culture dishes, into which a recombinant human NAP1L2 protein (Abcam, Cat.#ab117213), sorafenib, capecitabine, docetaxel, and osimertinib were added, or NAP1L2 siRNA (sh-NAP1L2, GeneChem) was transfected, respectively. The cells were placed in an incubator containing 5% carbon dioxide for culture at 37°C for 24 hours, washed with PBS, and then collected by high-speed centrifugation. An RLT lysis buffer was added to the above collected cells, the mixture was shaken at 4°C for half an hour and centrifuged to collect the

supernatant, and the expression of matrix metalloproteinases (MMPs) was measured by Western blot.

Total RNA was extracted from the above collected cells using Trizol reagent (Sigma Aldrich). 1 μg of RNA was reverse-transcribed into cDNA using a cDNA reverse transcription kit (Transgene Biotech, Cat.#AT311-03). 1.25 μL of a primer (SinoBiological, Cat.#HP100168&HP100367), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of the matrix metalloproteinases.

Results are shown in FIG. 4. After the recombinant NAP1L2 protein was added to the HaCaT keratinocytes, the mRNA and protein expression amounts of matrix metalloproteinases MMP2 and MMP9 were significantly increased; and when NAP1L2 siRNA was added to the HaCaT keratinocytes to inhibit the expression of NAP1L2, the mRNA and protein expression amounts of the matrix metalloproteinases MMP2 and MMP9 were significantly decreased. Numerous studies have confirmed that abnormal expression of matrix metalloproteinases is closely associated with various skin-related diseases (Kumper M. et al., Am. J. Physiol. Cell. Physiol. 2022, 323(4): 1290-1303).

**Example 6:** Regulation of the expression of human keratinocyte differentiation markers by m6A

$1*10^8$ HaCaT cells were seeded in culture dishes, into which a recombinant HBEGF protein (Thermo Fisher Scientific, Cat.#100-47-1mg) was added. The cells were placed in an incubator containing 5% carbon dioxide for culture at 37°C for 24 hours, into which an HBEGF antibody (Invitrogen, Cat.#406316) was added, or METTL3 siRNA (sh-METTL3, GeneChem) and NAP1L2 siRNA (sh-NAP1L2, GeneChem) were transfected. The cells were placed in an incubator containing 5% carbon dioxide for culture at 37°C for 24 hours, washed with PBS, and then collected by high-speed centrifugation. An RLT lysis buffer was added to the above collected cells, the mixture was shaken at 4°C for half an hour and centrifuged to collect the supernatant, and the protein expression amounts of keratinocyte differentiation markers KRT1, KRT10, Loricrin, and Involucrin were measured by Western blot.

Total RNA was extracted using Trizol reagent (Sigma Aldrich). 1 μg of RNA was reverse-transcribed into cDNA using a cDNA reverse transcription kit (Transgene Biotech, Cat.#AT311-03). 1.25 μL of a primer (with a primer sequence shown in Table 2), 10 μL of an iTag Universal SYBR Green supermix (Bio-Rad, Cat.#172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of the keratinocyte differentiation markers KRT1, KRT10, Loricrin, and Involucrin.

Results are shown in FIG. 5. After the recombinant HBEGF protein was added to the HaCaT keratinocytes, the mRNA and protein expression amounts of the keratinocyte differentiation markers KRT1, KRT10, Loricrin, and Involucrin were significantly increased, indicating that the HaCaT keratinocytes were in a highly differentiated state; however, when the HBEGF antibody or METTL3 or NAP1L2 siRNA was added to inhibit an m6A methylation function in the cells, the mRNA and protein expression amounts of the keratinocyte differentiation markers KRT1, KRT10, Loricrin, and Involucrin are significantly decreased. This indicates that m6A regulated the expression of the keratinocyte differentiation markers, thereby regulating the differentiation of the keratinocytes.

**Example 7:** Impacts of compounds on an m6A methylation level of THP-1 cells

$1*10^8$ THP-1 cells were seeded in culture dishes, into which 4 nM and 400 nM compound of formula (I), 4 nM and 4 μM nicotinamide, and 5 μM m6A methyltransferase inhibitor UZH2 were added, respectively. The cells were cultured in an incubator containing 5% $CO_2$ at 37°C for 24 hours. After discarding culture media and washing with PBS, the cells were centrifuged, and the supernatant was discarded. 400 μL of an RLT cell lysis buffer was added, and the cells were stored at -80°C. A portion of samples were selected, into which 400 μL of 70% ethanol was added and evenly stirred. 700 μL of a sample was transferred to an RNeasy centrifugation column and centrifuged for 15 seconds (8,000 g, 25°C). Then, 700 μL of an RW1 buffer solution was added and centrifuged for 15 seconds (8,000 g, 25°C), followed by the addition of 500 μL of an RPE buffer solution and centrifugation for 16 seconds (8,000 g, 25°C). The above steps were repeated twice, followed by centrifugation for 2 minutes to completely remove the eluent. 30 μL of nuclease-free water was added to a chromatographic column, and the cells were incubated for 5 minutes and then centrifuged for 2 minutes (12,000 g, 25°C). The steps were repeated 3 times. 2 μL of an RNA sample was selected and dissolved in 98 μL of a 10 mM Tris buffer solution, and the absorbance at 260 nm was measured using a Nanodrop to determine the RNA concentration.

50 μg of total RNA was selected and dissolved in 100 μL of nuclease-free water. Oligo (dT) magnetic beads were resuspended, 50 μL of the magnetic beads were transferred to a 1.5 mL test tube, 500 μL of a binding buffer solution was added, the mixture was allowed to stand, and the supernatant was removed. 100 μL of an RNA sample was added to 200 μL of a magnetic bead suspension for mixing, the mixture was stirred and incubated in a Thermomixer at 25°C for 5 minutes, the supernatant was discarded, and then 200 μL of a washing buffer solution was added. The steps were repeated 2 times, and then centrifugation was performed for 10 seconds (2,000 g, 25°C). 50 μL of an elution buffer solution

was added and evenly mixed, the mixture was heated to 75°C, the supernatant was transferred to a new 1.5 mL test tube and purified using an RNA Clean & Concentrator kit, and the RNA concentration was determined.

20 μL (approximately 200 ng) of an mRNA sample and 20 μL of a Nuclease P1 digestion premix (including 0.5 μL of 2 unit/μL Nuclease P1, 0.4 μL of 5 M NaCl, 2 μL of 0.1 M $ZnCl_2$, and 17.1 μL of PCR-grade water) were added into each test tube, and the mixture was stirred and incubated in a Thermomixer at 37°C for 2 hours. 2 μL of a 2 M $NH_4HCO_3$ solution and 1 unit of alkaline phosphatase were added, and the mixture was stirred and incubated in a Thermomixer at 37°C for 2 hours. 1 μL of a 1.2 M HCl neutralization solution was added, and centrifugation was performed for 30 minutes (16,000 g, 4°C). 20 μL of the supernatant was collected, an m6A methylation fragment was analyzed by LC-MS, and the m6A inhibition rate was calculated.

m6A inhibition rate (%)=(measured ion peak area-blank control)/(positive ion peak area-blank control)*100.

Results are shown in FIG. 6. The compound of formula (I) can significantly inhibit the m6A methylation level of mRNA of the cells in a concentration-dependent manner. At a concentration of 400 nM, the compound of formula (I) exhibited m6A inhibition activity comparable to that of the m6A methyltransferase inhibitor UZH2 at a concentration of 5 μM. However, nicotinamide exhibits no inhibitory effect on the m6A methylation level of mRNA of the cells at either a low concentration (4 nM) or a high concentration (4 μM).

**Example 8:** Impacts of compounds on an m6A methylation level of human keratinocytes

HaCaT cells were cultured in DMEM culture medium containing 10% fetal bovine serum, 100 U/mL penicillin, and 100 μg/mL streptomycin. Culture dishes were placed in an incubator containing 5% $CO_2$ for culture at 37°C for 24 hours. An equal volume of DMSO was added in control group 1, 50 nM sorafenib, capecitabine, docetaxel, and osimertinib were respectively added in control groups A, B, C, and D, and 400 nM test compounds or nicotinamide were respectively added in sample groups A1, A2, B1, B2, C1, C2, D1, and D2 after the addition of 50 nM corresponding compounds. The cells were continuously cultured for 24 hours, washed with PBS, and then collected by high-speed centrifugation. Total RNA was extracted using Trizol reagent (Sigma Aldrich), and RNA m6A methylation of a sample was quantitatively detected using an EpiQuik m6A RNA methylation quantification kit. The concentrations of positive control samples for a standard curve were 0.01 ng/μl, 0.02 ng/μl, 0.05 ng/μl, 0.1 ng/μl, 0.2 ng/μl, and 0.5 ng/μl. The absorbance at 450 nm was read using a microplate reader (Tecan GENios). A quantitative calculation formula for m6A is as follows:

m6A (ng)=(absorbance of sample well–absorbance of background well)/slope of standard curve

m6A (%)=m6A (ng)/RNA amount of sample (ng)*100%.

Results are shown in Table 1. The treatment with the sorafenib, the capecitabine, the docetaxel, and the osimertinib can significantly increase the m6A methylation level of human HaCaT keratinocytes (as shown in Table 1). The compound of formula (I) can effectively inhibit an abnormal increase in the RNA m6A level of human keratinocytes caused by chemotherapeutic drugs and multikinase inhibitors.

Table 1 Fold changes in the m6A methylation level relative to control group 1. A specific calculation method is: fold change=measured m6A amount/measured m6A amount of control 1.

| Group | Drug/compound | m6A fold change | Group | Drug/compound | m6A fold change |
|---|---|---|---|---|---|
| A | Sorafenib | 4.5 | B | Capecitabine | 6.7 |
| A1 | Compound of formula (I) | 1.4 | B1 | Compound of formula (I) | 1.1 |
| A2 | Nicotinamide | 4.7 | B2 | Nicotinamide | 4.5 |
| C | Docetaxel | 5.6 | D | Osimertinib | 4.8 |
| C1 | Compound of formula (I) | 1.0 | D1 | Compound of formula (I) | 1.2 |
| C2 | Nicotinamide | 5.4 | D2 | Nicotinamide | 5.0 |

**Example 9:** Inhibitory effects of compounds on the differentiation of HaCaT keratinocytes

Human HaCaT keratinocytes were cultured in DMEM culture medium containing 10% fetal bovine serum, 100 U/mL penicillin, and 100 μg/mL streptomycin. The cells were seeded into a 96-well plate at a density of $2*10^6$ cells per square centimeter and placed in an incubator containing 5% carbon dioxide for culture at 37°C for 24 hours. An equal volume of DMSO was added into control well 1, a 2.5 ng/mL recombinant human HBEGF protein (Abcam, ab205523) was added into control well 2, and a 2.5 ng/mL recombinant human HBEGF protein and corresponding concentrations of test compounds were added into sample wells. The cells were continuously cultured for 24 hours, washed with PBS, and then collected by high-speed centrifugation. Total RNA was extracted using Trizol reagent (Sigma Aldrich). 1 μg of RNA was reverse-transcribed into cDNA using a cDNA reverse transcription kit (Transgene Biotech, AT311-03). 1.25 μL of a primer (with a primer sequence shown in Table 2), 10 μL of an iTag Universal SYBR Green supermix (Bio-Rad, 172-5125), and an appropriate amount of DEPC ultrapure water were added to formulate 20 μL of a reaction solution to perform an RT-PCR reaction. Upon completion of the reaction, the reaction solution was subjected to agarose gel electrophoresis to determine the mRNA expression level of keratinocyte differentiation markers KRT1, KRT10, Loricrin, and Involucrin.

Table 2 Sequence information of some primers

| | | |
|---|---|---|
| KRT1 | Forward primer | 5'-GTCAAGTCCTCTGGTGGCAG-3' |
| | Reverse primer | 5'-AAGGCTGGGACAAATCGACC-3' |
| KRT10 | Forward primer | 5'-CCCGGGTGTTGATCTGACTC-3' |
| | Reverse primer | 5'-CCAGGCTTCAGCATCTTTGC-3' |
| Loricrin | Forward primer | 5'-TCATGATGCTACCCGAGGTTTG-3' |
| | Reverse primer | 5'-CAGAACTAGATGCAGCCGGAGA-3' |
| Involucrin | Forward primer | 5'-TCGCTCCTCAAGACTGTTCCTCC-3' |
| | Reverse primer | 5'-CAGGCAGTCCCTTTACAGCA-3' |

As shown in FIG. 7, after the recombinant human HBEGF protein was added for induction, the mRNA expression level of the HaCaT differentiation markers KRT1, KRT10, Loricrin, and Involucrin were significantly increased. However, after the compound of formula (I) was added, the mRNA expression level of the HaCaT differentiation markers KRT1, KRT10, Loricrin, and Involucrin were significantly inhibited.

**Example 10:** Inhibitory effects of compounds on the differentiation of keratinocytes in rat models with a hand-foot skin reaction

After adaptive feeding for one week, SD rats (with a body weight of approximately 200 g) were randomly divided into groups with balanced body weight, with 12 rats in each group. Modeling drugs (including sorafenib, erlotinib, afatinib, and osimertinib) were respectively dissolved in a solution containing 5% of DMSO, 45% of PEG400, and 50% of $H_2O$, and the modeling drugs were diluted to required concentrations and then administered once daily by gavage at doses shown in Table 3. After administration by gavage for 1 hour, 0.05 g of ointments containing different mass proportions of test compounds were evenly applied to left paws of the rats, a blank matrix ointment was applied to right paws of the rats to serve as a self-control, and no ointment was applied to the rats in a blank control group. After drug application, four limbs were continuously immobilized for 2 hours, then wiped with clean water to remove the residual drugs, removed from immobilization, and restored to free movement. The modeling drugs, the blank matrix ointment, and the ointments containing the test compounds were all administered once daily. After continuous administration for 30 days, the rats were euthanized, and paw plantar skin tissues were collected, fixed in 10% neutral formalin, cut into 5 μm sections, dehydrated, and then embedded in paraffin. The ointments for the test compounds were prepared by mixing the compounds, white beeswax, white petrolatum, and light liquid paraffin in specific ratios (weight ratios of 3 ointments were respectively 1:18:58:23, 3:18:57:22, and 10:20:40:30). The blank matrix ointment was prepared by mixing white beeswax, white petrolatum, and light liquid paraffin in a specific ratio (weight ratio of 18:60:22).

Tissue staining: After dewaxing and rehydration, the paw plantar skin tissue sections of the above rats were stained in a hematoxylin solution for several minutes, cleaned, and placed in 1% acid alcohol for soaking until the sections faded to light blue-red. After cleaning with running water for 5 minutes, the sections were stained with eosin for 2-3 minutes, cleaned to remove excess dyes, dehydrated, then permeabilized by adding xylene for several minutes, and sealed and fixed with a neutral resin. The stratum corneum was observed under an optical microscope, and the thickness of the epidermal stratum corneum was measured using Dmetrix software.

Immunohistochemical staining: After dewaxing and hydration, the paw plantar skin tissue sections of the above rats were incubated with 3% $H_2O_2$ at room temperature for 30 minutes and blocked with 10% goat serum for 30 minutes after antigen retrieval. A KRT1 antibody (Abcam, ab93652) and a Loricrin antibody (Abcam, ab183646) were added dropwise, the sections were incubated overnight at 4°C, and an HRP secondary antibody (ZSGB-BIO, PV-6001) and a DAB kit (ZSG-BIO, ZLI9017) were added for color development. The sections were re-stained with hematoxylin, cleaned, and sealed and fixed with a neutral resin, and the expression amounts of KRT1 and Loricrin were observed under an optical microscope.

Criteria for determining successful modeling of the above rat models are as follows: (i) symptoms, such as erythema, swelling, desquamation, ulcers, or blisters, occur at paw sites; and/or (ii) the stratum corneum thickness in tissue staining is significantly higher than that of normal rats; and/or (iii) markers, such as KRT1, KRT5, and Loricrin, are significant increased. An incidence rate calculation method involves a proportion of animals in each group meeting the above criteria for determining successful modeling, that is, incidence rate = (number of successfully modeled rats/total number of rats in the group)*100%.

Pathological scoring criteria for tissue staining of the above rats are as follows: no blisters, recorded as 0 point; the number of blisters being 1-3, recorded as 1 point; the number of blisters being 4-6, recorded as 2 points; the number of blisters being 7-9, recorded as 3 points; and the number of blisters being more than 10, recorded as 4 points; an inflammation area accounting for less than 10% of a total section area, recorded as 0 point; the inflammation area accounting for 10-25% of the total section area, recorded as 1 point; the inflammation area accounting for 25-50% of the total section area, recorded as 2 points; the inflammation area accounting for 50-75% of the total section area, recorded as 3 points; and the inflammation area accounting for more than 75% of the total section area, recorded as 4 points; an congestion area accounting for less than 10% of a total section area, recorded as 0 point; the congestion area accounting for 10-25% of the total section area, recorded as 1 point; the congestion area accounting for 25-50% of the total section area, recorded as 2 points; the congestion area accounting for 50-75% of the total section area, recorded as 3 points; and the congestion area accounting for more than 75% of the total section area, recorded as 4 points. Scoring was performed using a double-blind scoring system. After completion of statistics, data for each group was presented in the form of mean + SEM (N=12).

Table 3 Doses of drugs used in rat models and experimental results

| **Serial number** | **Modeling drug** | **Dose** | **Compound and its mass proportion in an ointment** | | **Incidence rate** | |
|---|---|---|---|---|---|---|
| | | | Left paw | Right paw | Left paw | Right paw |
| 1 | Sorafenib | 100 mg/kg | Compound of formula (I), 1% | None | 2/12 (17%) | 9/12 (75%) |
| 2 | Erlotinib | 70 mg/kg | Compound of formula (I), 1% | None | 3/12 (25%) | 10/12 (83%) |
| 3 | Afatinib | 50 mg/kg | Compound of formula (I), 1% | None | 3/12 (25%) | 9/12 (75%) |
| 4 | Osimertinib | 60 mg/kg | Compound of formula (I), 1% | None | 2/12 (17%) | 8/12 (67%) |
| 5 | Sorafenib | 100 mg/kg | Compound of formula (I), 3% | None | 2/12 (17%) | 4/12 (33%) |
| 6 | Erlotinib | 70 mg/kg | Compound of formula (I), 3% | None | 2/12 (17%) | 5/12 (42%) |
| 7 | Afatinib | 50 mg/kg | Compound of formula (I), 3% | None | 2/12 (17%) | 4/12 (33%) |
| 8 | Osimertinib | 60 mg/kg | Compound of formula (I), 3% | None | 2/12 (17%) | 4/12 (33%) |
| 9 | Sorafenib | 100 mg/kg | Compound of formula (I), 10% | None | 0/12 (0%) | 4/12 (33%) |
| 10 | Erlotinib | 70 mg/kg | Compound of formula (I), 10% | None | 1/12 (8%) | 4/12 (33%) |
| 11 | Afatinib | 50 mg/kg | Compound of formula (I), 10% | None | 0/12 (0%) | 3/12 (25%) |
| 12 | Osimertinib | 60 mg/kg | Compound of formula (I), 10% | None | 0/12 (0%) | 3/12 (25%) |
| 13 | Sorafenib | 100 mg/kg | None | None | 9/12 (75%) | |
| 14 | Erlotinib | 70 mg/kg | None | None | 10/12 (83.3%) | |
| 15 | Afatinib | 50 mg/kg | None | None | 8/12 (66.7%) | |
| 16 | Osimertinib | 60 mg/kg | None | None | 9/12 (75%) | |

As shown in Table 3, when the sorafenib, the erlotinib, the afatinib, and the osimertinib were respectively administered once daily by gavage at doses of 100 mg/kg, 70 mg/kg, 50 mg/kg, and 60 mg/kg to the SD rats, successful

modeling rates for the hand-foot skin reaction were 75%, 83.3%, 66.7%, and 75%, respectively. The compound of formula (I) at a low dose (such as the compound at a mass content of 1%) can significantly decrease the incidence rate of hand-foot skin reaction at a drug application site. The compound of formula (I) at a high mass content (such as the compound at a mass content of 3% and 10%) can significantly decrease the incidence rate of hand-foot skin reaction in all paws of the rats.

According to stratum corneum thickness measurement results shown in FIG. 8, in the successfully modeled rats using the sorafenib, the erlotinib, the afatinib, and the osimertinib, the stratum corneum thickness of the paw plantar skin was significantly higher than that of normal rats. The compound of formula (I) can effectively decrease the stratum corneum thickness of the paw plantar skin of the modeled rats.

According to tissue staining results shown in FIG. 9, due to skin toxicity caused by the modeling drugs, blisters can be formed under the epidermis and in the epidermis of the paw plantar skin of the rats, inflammatory cell infiltration occurs in the dermis, and congestion symptoms occur in skin tissues. The compound of formula (I) can effectively alleviate the formation of blisters in the epidermis and subepidermis caused by the above modeling drugs, inhibit the inflammatory cell infiltration in the dermis, and significantly alleviate the skin congestion phenomenon.

According to pathological scoring results shown in FIG. 10, the modeling drugs can cause the formation of blisters under the epidermis and in the epidermis at paw plantar sites of the rats, inflammatory cell infiltration occurs in the dermis, and congestion symptoms occur in skin tissues. The compound of formula (I) can effectively alleviate the formation of blisters in the epidermis and subepidermis caused by the above modeling drugs, inhibit the inflammatory cell infiltration in the dermis, and significantly alleviate the skin congestion phenomenon.

Immunohistochemical staining results also showed that in the successfully modeled rats using the sorafenib, the erlotinib, the afatinib, and the osimertinib, KRT1 and Loricrin were significantly increased in tissues. However, with the addition of compounds, KRT1 and Loricrin were significantly decreased in the tissues. In summary, the compound of formula (I) can effectively inhibit the differentiation of keratinocytes both *in vitro* and *in vivo*, thus exhibiting potential for treatment of a hyperkeratosis-related disease.

**Example 11:** Effect of the compound of formula (I) in a chemotherapeutic drug-induced mouse model with hand-foot syndrome

After adaptive feeding for 7 days, SPF-grade ICR mice (male, 5-6 weeks old, with a body weight of approximately 35 g) were randomly divided into groups, including a blank control group (6 mice), a capecitabine modeling group (6 mice), a docetaxel modeling group (6 mice), a capecitabine + compound of formula (I) group (6 mice), a docetaxel + compound of formula (I) group (6 mice), a capecitabine + nicotinamide group (6 mice), and a docetaxel + nicotinamide group (6 mice). The groups were respectively administered corresponding modeling drugs by oral gavage (capecitabine at 200 mg/kg and docetaxel at 25 mg/kg) once daily; and the blank control group was administered an equivalent volume of normal saline. After administration with the chemotherapeutic drugs for 2 weeks, the ointments containing the compound of formula (I) (at a compound content of 3%) used in Example 10 were respectively applied topically in the groups, or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind feet of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3%

hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of $Na_2HPO_4$, and 1 M HCl, pH 7.4). The sections were permeabilized with 0.5% Triton-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin adhesive (Solarbio, Catalog No. 20230725), and inspected under a microscope.

Mouse skin appearance results are shown in FIG. 11. After modeling with the capecitabine and the docetaxel for 14 days, the skin of the hind limb toes of the mice had obvious lesions. Compared with the normal control group, the skin of the hind limb toes of the mice in the capecitabine group and the docetaxel group had obvious erythema, rhagades, and blisters.

The degree of swelling of the hind limb toes of the mice is shown in FIG. 12. After continuous oral administration of the modeling drugs including the capecitabine and the docetaxel for 14 days, swelling of the hind limb toes of the mice was caused. Topical application of the ointments containing the compound of formula (I) to the hind limb toes of the mice can significantly alleviate the swelling of the toes caused by the modeling drugs ($p<0.05$). However, it was not observed that oral administration of nicotinamide at 100 mg/kg significantly alleviated the swelling of the hind limb toes of the mice.

Pathological staining results of toe skin tissues are shown in FIG. 13. Compared with the normal control group, after continuous oral administration of the modeling drugs including the capecitabine and the docetaxel for 14 days, the epidermal layer and the stratum corneum in the skin tissues of the hind limb toes of the mice were significantly thickened, with increased granular cells in the basal layer and infiltration of inflammatory cells observed. Topical application of the ointments containing the compound of formula (I) to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of nicotinamide at 100 mg/kg mildly alleviated the thickening of the epidermal layer and the stratum corneum in the skin tissues of the hind limb toes of the mice, with slightly increased granular cells in the basal layer and infiltration of inflammatory cells still observed.

Immunohistochemical results of the capecitabine modeling series groups are shown in FIG. 14. Compared with the normal control group, after continuous oral administration of the modeling drug capecitabine for 14 days, cytokine IL-8 in the skin tissues of the hind limb toes of the mice was significantly increased ($p<0.05$). This indicates that oral administration of the capecitabine resulted in skin inflammation of the mice, which is consistent with a clinical trial observation result. Topical application of the ointments containing the compound of formula (I) to the hind limb toes of the mice can significantly decrease the expression level of cytokine IL-8 in the skin tissues of the hind limb toes of the mice ($p<0.05$). However, oral administration of the nicotinamide at 100 mg/kg didn't have a significant impact on the expression level of cytokine IL-8 in the skin tissues of the hind limb toes of the mice.

Immunohistochemical results of the docetaxel modeling series groups are shown in FIG. 15. Compared with the normal control group, after continuous oral administration of the modeling drug docetaxel for 14 days, cytokine IL-6 in the skin tissues of the hind limb toes of the mice was significantly increased ($p<0.05$). This indicates that oral administration of the docetaxel resulted in skin inflammation of the mice, which is consistent with a clinical trial observation result. Topical application of the ointments containing the compound of formula (I) to the hind limb toes of the mice can significantly decrease the expression level of cytokine IL-6 in the skin tissues of the hind limb toes of the mice ($p<0.05$). However, oral administration of the nicotinamide at 100 mg/kg didn't have a significant impact on the expression level of cytokine IL-6 in the skin tissues of the hind limb toes of the mice.

**Example 12:** Effect of the compound of formula (I) in a kinase inhibitor-induced mouse model with hand-foot skin reaction

After adaptive feeding for 7 days, SPF-grade ICR mice (male, 5-6 weeks old, with a body weight of approximately 35 g) were randomly divided into groups, including a blank control group (6 mice), a sorafenib modeling group (6 mice), an

osimertinib modeling group (6 mice), a sorafenib + compound of formula (I) group (6 mice), an osimertinib + compound of formula (I) group (6 mice), a sorafenib + nicotinamide group (6 mice), and an osimertinib + nicotinamide group (6 mice). The groups were respectively administered corresponding modeling drugs by oral gavage (sorafenib at 100 mg/kg and osimertinib at 10 mg/kg) once daily; and the blank control group was administered an equivalent volume of normal saline. After administration with the chemotherapeutic drugs for 2 weeks, the ointments containing the compound of formula (I) (at a compound content of 3%) used in Example 10 were respectively applied topically in the groups, or nicotinamide was orally administered (100 mg/kg) once daily continuously for 16 days.

Measurement of the degree of swelling of toes: The degree of swelling of hind limb toes of the mice was measured using a toe swelling tester (KW-7C, Nanjing Calvin Biotechnology Co., Ltd.) before administration with the modeling drugs, after administration with the modeling drugs for 2 weeks, and before sacrifice, respectively. Before measurement, ankle joints of hind feet of the mice were marked at same positions before administration, after administration for 2 weeks, and before sacrifice. During measurement, water was properly located at the same level as the marked positions, and numerical values were recorded after becoming stable.

Pathological staining of skin tissues: Skin tissue samples were selected from hind limb toes of the mice and placed in 4% paraformaldehyde for fixation at room temperature for 4 hours. Then, the tissues were taken out and rinsed with running water for several hours. The samples were subjected to dehydration treatment with 70%, 80%, and 90% ethanol solutions, followed by treatment with a mixed solution of equal amounts of absolute alcohol and xylene for 15 minutes, and then permeabilized twice with a xylene solution for 15 minutes each time until the samples became transparent. The samples were placed in a mixture of half xylene and half paraffin for soaking for 15 minutes, and then, paraffin I and paraffin II were added for wax penetration for one hour, respectively. After embedding with paraffin, the samples were sectioned, baked, dewaxed, and hydrated. Then, the hydrated sections were placed in a hematoxylin solution (ZSGB-BIO, article No. 23041001) for staining for 3 minutes, and a differentiation solution of hydrochloric acid in ethanol was added for differentiation for 15 seconds. After rinsing with water, a Scott bluing solution (Servicebio, article No. 20230801) was added for bluing for 15 seconds. After rinsing with running water, the sections were stained with an eosin solution (Solarbio, article No. 33535) for 3 minutes, rinsed with running water, dehydrated, permeabilized, sealed, and inspected under a microscope.

Immunohistochemical staining: After baking, dewaxing, and hydration, paraffin sections of skin tissues of hind limb toes of the mice were subjected to antigen retrieval using a 0.2 M citric acid buffer solution (containing 1.534 g of citric acid, 3.1 g of sodium citrate, and 100 mL of distilled water, pH 4.7). Endogenous hydrogen peroxide was removed using 3% hydrogen peroxide (Shandong Annjet High-Tech Disinfection Technology Co., Ltd., article No. 20290902), and the sections were incubated at room temperature for 10 minutes and then fully rinsed with a PBS buffer solution (containing 8 g of NaCl, 0.2 g of KCl, 1.44 g of $Na_2HPO_4$, and 1 M HCl, pH 7.4). The sections were permeabilized with 0.5% Triton-X-100 (AmyJet Scientific, article No. A-CSH436-100 mL), respectively permeabilized with mouse anti-IL-1β and rabbit IL-8 for 10 minutes, and not permeabilized with rabbit anti-IL-6. After blocking with a 5% BSA antigen, 1/150 diluted mouse anti-IL-1β (Affinity Biosciences, article No. BF8021), 1/200 diluted rabbit anti-IL-8 (Affinity Biosciences, article No. DF6998), or 1/150 diluted rabbit anti-IL-6 (Affinity Biosciences, article No. DF6087) was added dropwise onto each slide, and the slides were placed in a wet box for incubation at 4°C overnight. After the overnight incubation, the wet box was taken out and allowed to stand at room temperature for 45 minutes. The slides were rinsed 3 times with a PBS buffer solution for 15 minutes each time. For the rabbit anti-IL-8 and rabbit anti-IL-6 indicators, 1/100 diluted horseradish peroxidase-labeled goat anti-rabbit IgG (H+L) (ZSGB-BIO, article No. 234750811) was added. For the mouse anti-IL-1β indicator, 1/100 diluted horseradish peroxidase-labeled goat anti-mouse IgG (H+L) (ZSGB-BIO, article No. 226700804) was added. The slides were incubated at 37°C for 30 minutes and then fully rinsed with a PBS buffer solution. DAB (CWBIO, article No. 13723) was added for color development for 3-5 minutes, and the sections were cleaned with a PBS buffer solution for 1 minute, re-stained with hematoxylin (ZSGB-BIO, article No. 23041001) for 3 minutes, differentiated with hydrochloric acid in alcohol, blued, and washed with water. The sections were sequentially placed in 80% ethanol, 95% ethanol, anhydrous ethanol, and anhydrous ethanol II for rapid dehydration, permeabilized with xylene I and xylene II, sealed with a neutral resin adhesive (Solarbio, Catalog No. 20230725), and inspected under a microscope.

Results are shown in FIG. 16. After modeling with the sorafenib and the osimertinib for 14 days, the skin of the hind limb toes of the mice had obvious lesions. Compared with the normal control group, the skin of the hind limb toes of the mice in the sorafenib group and the osimertinib group had obvious erythema and rhagades.

The degree of swelling of the hind limb toes of the mice is shown in FIG. 17. After continuous oral administration of the modeling drugs including the sorafenib and the osimertinib for 14 days, significant swelling of the hind limb toes of the mice was caused. Topical application of the ointments containing the compound of formula (I) to the hind limb toes of the mice can effectively alleviate the swelling of the toes caused by the modeling drugs. However, it was not observed that oral administration of the nicotinamide at 100 mg/kg significantly alleviated the swelling of the hind limb toes of the mice.

Pathological staining results of toe skin tissues are shown in FIG. 18. Compared with the normal control group, after continuous oral administration of the modeling drugs including the sorafenib and the osimertinib for 14 days, the epidermal layer and the stratum corneum in the skin tissues of the hind limb toes of the mice were significantly thickened,

with increased granular cells in the basal layer and infiltration of inflammatory cells observed. Topical application of the ointments containing the compound of formula (I) to the hind limb toes of the mice can effectively alleviate the phenomenon of thickening of the epidermal layer and the stratum corneum in the skin tissues, with the morphology of basal layer cells restored to normal and no infiltration of inflammatory cells observed. However, oral administration of the nicotinamide at 100 mg/kg didn't significantly alleviate the thickening of the epidermal layer and the stratum corneum in the skin tissues of the hind limb toes of the mice, with slightly restored morphology of basal layer cells and infiltration of inflammatory cells still observed.

Immunohistochemical results of the sorafenib modeling series groups are shown in FIG. 19. Compared with the normal control group, after continuous oral administration of the modeling drug sorafenib for 14 days, cytokine IL-1β in the skin tissues of the hind limb toes of the mice was significantly increased ($p<0.05$). This indicates that oral administration of the sorafenib resulted in skin inflammation of the mice, which is consistent with a clinical trial observation result. Topical application of the ointments containing the compound of formula (I) to the hind limb toes of the mice can significantly decrease the expression level of cytokine IL-1β in the skin tissues of the hind limb toes of the mice ($p<0.05$). However, oral administration of the nicotinamide at 100 mg/kg didn't have a significant impact on the expression level of cytokine IL-1β in the skin tissues of the hind limb toes of the mice.

Immunohistochemical results of the osimertinib modeling series groups are shown in FIG. 20. Compared with the normal control group, after continuous oral administration of the modeling drug osimertinib for 14 days, cytokine IL-1β in the skin tissues of the hind limb toes of the mice was significantly increased ($p<0.05$). This indicates that oral administration of the osimertinib resulted in skin inflammation of the mice, which is consistent with a clinical trial observation result. Topical application of the ointments containing the compound of formula (I) to the hind limb toes of the mice can significantly decrease the expression level of cytokine IL-1β in the skin tissues of the hind limb toes of the mice ($p<0.05$). However, oral administration of the nicotinamide at 100 mg/kg didn't have a significant impact on the expression level of cytokine IL-1β in the skin tissues of the hind limb toes of the mice.

**Example 13: Preparation of the crystal form A of the compound of formula (I)**

50 mg of the compound of formula (I) was weighed and added into ethanol (0.4 mL), and the mixture was suspended under magnetic stirring at a rate of 300-400 rpm at 25°C for 1 week. A resulting suspension was centrifuged and filtered using a 0.45 μm nylon filter membrane centrifuge tube at 14,000 rpm to obtain a solid. X-ray powder diffraction detection showed that the product was the crystal form A, with an XRPD pattern as shown in FIG. 21 and positions of characteristic peaks as shown in Table 4. A DSC spectrogram showed endothermic peaks $T_{onset}$ at 155.80°C, 163.89°C, and 164.69°C.

Table 4 XRPD diffraction peak data of the crystal form A of the compound of formula (I)

| 2θ(°) | D(Å) | Relative intensity (%) |
|---|---|---|
| 5.547 | 15.9188 | 5.60 |
| 6.760 | 13.0654 | 24.30 |
| 12.326 | 7.17498 | 5.60 |
| 12.673 | 6.97938 | 59.80 |
| 13.507 | 6.55005 | 34.60 |
| 14.870 | 5.95264 | 7.20 |
| 15.162 | 5.83872 | 19.40 |
| 16.615 | 5.33133 | 8.60 |
| 17.413 | 5.08876 | 12.00 |
| 18.267 | 4.85267 | 9.80 |
| 18.557 | 4.77762 | 100.00 |
| 19.104 | 4.64202 | 6.40 |
| 19.315 | 4.59162 | 26.10 |
| 20.081 | 4.41828 | 11.80 |
| 20.380 | 4.35409 | 23.00 |
| 20.727 | 4.28194 | 15.50 |

(continued)

| 2θ(°) | D(Å) | Relative intensity (%) |
|---|---|---|
| 21.183 | 4.19086 | 16.40 |
| 21.384 | 4.15182 | 19.30 |
| 21.594 | 4.11207 | 43.10 |
| 21.901 | 4.05497 | 27.90 |
| 22.214 | 3.99865 | 30.80 |
| 22.779 | 3.90064 | 14.50 |
| 23.010 | 3.86198 | 10.80 |
| 23.976 | 3.70853 | 7.70 |
| 24.774 | 3.59091 | 23.60 |
| 24.951 | 3.56579 | 47.90 |
| 25.376 | 3.50709 | 16.00 |
| 26.125 | 3.40825 | 23.50 |
| 26.735 | 3.33183 | 8.80 |
| 27.029 | 3.29622 | 54.50 |
| 27.710 | 3.2167 | 12.70 |
| 28.518 | 3.12742 | 9.80 |
| 28.975 | 3.07909 | 12.60 |
| 33.461 | 2.67588 | 45.40 |
| 35.425 | 2.53186 | 20.30 |
| 35.428 | 2.53167 | 20.10 |

**Example 14: Preparation of the crystal form B of the compound of formula (I)**

50 mg of the compound of formula (I) was weighed and added into methanol (0.4 mL) to be fully dissolved at an ambient temperature, and a resulting solution or suspension was filtered through a 0.45 μm nylon filter membrane syringe type filter to obtain a clear solution. Water (1.6 mL) was slowly added into the resulting clear solution. The resulting suspension was centrifuged and filtered using a 0.45 μm nylon filter membrane centrifuge tube at 14,000 rpm to obtain a solid. X-ray powder diffraction detection showed that the product was the crystal form B, with an XRPD pattern as shown in FIG. 24 and positions of characteristic peaks as shown in Table 5. A DSC spectrogram showed a melting point $T_{onset}$ at 166.07°C and an endothermic peak $T_{onset}$ at 171.93°C.

Table 5 XRPD diffraction peak data of the crystal form B of the compound of formula (I)

| 2θ(°) | D(Å) | Relative intensity (%) |
|---|---|---|
| 6.766 | 13.0531 | 4.50 |
| 10.140 | 8.71686 | 2.10 |
| 10.834 | 8.1594 | 63.20 |
| 13.544 | 6.53253 | 52.50 |
| 14.962 | 5.91634 | 21.80 |
| 18.584 | 4.77061 | 1.70 |
| 18.696 | 4.7424 | 4.40 |
| 19.915 | 4.45463 | 6.00 |
| 20.385 | 4.35316 | 2.00 |

(continued)

| 2θ(°) | D(Å) | Relative intensity (%) |
|---|---|---|
| 20.580 | 4.3122 | 9.50 |
| 21.722 | 4.08808 | 100.00 |
| 22.809 | 3.89564 | 9.60 |
| 23.822 | 3.73228 | 16.90 |
| 25.341 | 3.51185 | 10.40 |
| 26.657 | 3.3414 | 6.70 |
| 26.812 | 3.32242 | 14.50 |
| 27.231 | 3.27224 | 8.00 |
| 29.852 | 2.99059 | 18.00 |
| 30.121 | 2.96457 | 6.10 |
| 30.594 | 2.91977 | 24.50 |
| 31.300 | 2.85552 | 3.70 |
| 35.465 | 2.52912 | 5.30 |
| 35.706 | 2.51257 | 4.10 |

**Example 15: Preparation of the crystal form C of the compound of formula (I)**

50 mg of the compound of formula (I) was weighed and added into ethyl acetate (0.8 mL) to be fully dissolved at an ambient temperature, and a resulting solution or suspension was filtered through a 0.45 μm nylon filter membrane syringe type filter to obtain a clear solution. N-heptane (4 mL) was slowly added into the resulting clear solution. The resulting suspension was centrifuged and filtered using a 0.45 μm nylon filter membrane centrifuge tube at 14,000 rpm to obtain a solid. X-ray powder diffraction detection showed that the product was the crystal form C, with an XRPD pattern as shown in FIG. 27 and positions of characteristic peaks as shown in Table 6. A DSC spectrogram showed a melting point $T_{onset}$ at 150.71°C.

Table 6 XRPD diffraction peak data of the crystal form C of the compound of formula (I)

| 2θ(°) | D(Å) | Relative intensity (%) |
|---|---|---|
| 7.462 | 11.8377 | 2.80 |
| 10.095 | 8.75505 | 1.80 |
| 10.532 | 8.39262 | 100.00 |
| 11.778 | 7.50792 | 19.10 |
| 14.049 | 6.29889 | 1.40 |
| 14.686 | 6.0271 | 2.50 |
| 16.749 | 5.28889 | 19.60 |
| 17.300 | 5.1216 | 16.90 |
| 17.845 | 4.96644 | 5.40 |
| 19.015 | 4.66361 | 21.20 |
| 20.502 | 4.3284 | 2.10 |
| 21.131 | 4.20098 | 46.20 |
| 22.677 | 3.91807 | 5.30 |
| 22.878 | 3.88405 | 5.20 |
| 23.402 | 3.79827 | 5.50 |

(continued)

| 2θ(°) | D(Å) | Relative intensity (%) |
|---|---|---|
| 23.676 | 3.75497 | 3.00 |
| 24.207 | 3.67376 | 5.00 |
| 24.765 | 3.59214 | 11.90 |
| 26.164 | 3.40322 | 3.30 |
| 26.993 | 3.3005 | 7.90 |
| 27.385 | 3.25421 | 2.90 |
| 28.171 | 3.16513 | 5.20 |
| 28.564 | 3.12245 | 16.30 |
| 30.021 | 2.97416 | 3.80 |

**Example 16: Preparation of the crystal form D of the compound of formula (I)**

Approximately 50 mg of the compound of formula (I) was weighed and added into acetone (0.6 mL) to be fully dissolved at 50°C, and a resulting solution or a dilute suspension was filtered through a 0.45 μm nylon filter membrane syringe type filter to obtain a clear solution. The resulting clear solution was cooled to 5°C at a cooling rate of 0.1°C/min. The resulting suspension was centrifuged and filtered using a 0.45 μm nylon filter membrane centrifuge tube at 14,000 rpm to obtain a solid. X-ray powder diffraction detection showed that the product was the crystal form D, with an XRPD pattern as shown in FIG. 30 and positions of characteristic peaks as shown in Table 7. A DSC spectrogram showed an endothermic peak $T_{onset}$ at 164.75°C, an exothermic peak $T_{onset}$ at 168.22°C, and an endothermic peak $T_{onset}$ at 171.36°C.

Table 7 XRPD diffraction peak data of the crystal form D of the compound of formula (I)

| 2θ(°) | D(Å) | Relative intensity (%) |
|---|---|---|
| 9.632 | 9.17516 | 2.40 |
| 10.295 | 8.58569 | 3.80 |
| 10.667 | 8.28673 | 100.00 |
| 11.977 | 7.38346 | 9.80 |
| 14.232 | 6.21809 | 3.10 |
| 14.374 | 6.15719 | 6.70 |
| 15.726 | 5.63071 | 20.00 |
| 16.277 | 5.44115 | 8.10 |
| 16.493 | 5.37055 | 14.00 |
| 17.319 | 5.11619 | 41.60 |
| 17.649 | 5.02121 | 3.80 |
| 18.045 | 4.91194 | 18.20 |
| 18.341 | 4.83341 | 10.30 |
| 18.997 | 4.6679 | 6.10 |
| 19.284 | 4.59905 | 30.70 |
| 19.602 | 4.52504 | 5.00 |
| 19.959 | 4.44499 | 25.10 |
| 21.379 | 4.15278 | 57.60 |
| 22.453 | 3.95662 | 3.70 |
| 22.668 | 3.91948 | 5.40 |

(continued)

| 2θ(°) | D(Å) | Relative intensity (%) |
|---|---|---|
| 23.201 | 3.83073 | 11.60 |
| 23.390 | 3.80017 | 5.10 |
| 24.135 | 3.68454 | 14.20 |
| 24.269 | 3.66453 | 8.30 |
| 24.688 | 3.60321 | 2.80 |
| 25.317 | 3.51508 | 61.90 |
| 25.572 | 3.48065 | 9.00 |
| 26.113 | 3.40974 | 3.00 |
| 26.476 | 3.36378 | 14.70 |
| 27.108 | 3.28682 | 10.40 |
| 27.193 | 3.27671 | 18.80 |
| 27.538 | 3.23648 | 5.50 |
| 27.883 | 3.19718 | 5.30 |
| 28.532 | 3.12591 | 7.70 |
| 28.917 | 3.08519 | 25.90 |
| 29.998 | 2.97637 | 8.80 |
| 31.852 | 2.80729 | 5.20 |
| 32.326 | 2.7672 | 5.70 |
| 35.469 | 2.52887 | 6.70 |

**Example 17: Preparation of the crystal form E of the compound of formula (I)**

50 mg of the compound of formula (I) was weighed and added into 1,4-dioxane (0.4 mL), and the mixture was suspended under magnetic stirring at a rate of 300-400 rpm at 25°C for 1 week. A resulting suspension was centrifuged and filtered using a 0.45 μm nylon filter membrane centrifuge tube at 14,000 rpm to obtain a solid. X-ray powder diffraction detection showed that the product was the crystal form E, with an XRPD pattern as shown in FIG. 33 and positions of characteristic peaks as shown in Table 8. A DSC spectrogram showed an endothermic peak $T_{onset}$ at 161.98°C and an endothermic peak $T_{onset}$ at 169.77°C.

Table 8 XRPD diffraction peak data of the crystal form E of the compound of formula (I)

| 2θ(°) | D(Å) | Relative intensity (%) |
|---|---|---|
| 9.992 | 8.84557 | 44.40 |
| 11.989 | 7.37618 | 61.50 |
| 14.378 | 6.1555 | 9.20 |
| 16.248 | 5.45099 | 99.80 |
| 18.329 | 4.8364 | 5.40 |
| 19.177 | 4.62449 | 2.30 |
| 19.414 | 4.56862 | 23.70 |
| 19.659 | 4.51218 | 68.50 |
| 19.973 | 4.44191 | 65.00 |
| 21.175 | 4.19246 | 20.80 |
| 21.912 | 4.05304 | 44.10 |

(continued)

| 2θ(°) | D(Å) | Relative intensity (%) |
|---|---|---|
| 22.448 | 3.95743 | 55.40 |
| 22.632 | 3.92576 | 24.70 |
| 23.014 | 3.8614 | 100.00 |
| 23.880 | 3.72327 | 19.30 |
| 24.190 | 3.67623 | 48.40 |
| 25.095 | 3.54572 | 19.80 |
| 25.316 | 3.51523 | 14.60 |
| 25.656 | 3.46945 | 9.30 |
| 26.375 | 3.37645 | 19.60 |
| 28.171 | 3.16509 | 7.30 |
| 28.530 | 3.12609 | 19.30 |
| 29.795 | 2.99619 | 10.20 |
| 30.099 | 2.96662 | 7.90 |
| 30.862 | 2.89505 | 38.90 |
| 31.163 | 2.8677 | 16.20 |
| 31.516 | 2.83645 | 12.90 |
| 32.174 | 2.77992 | 15.20 |
| 32.739 | 2.73321 | 22.80 |
| 33.462 | 2.67579 | 4.50 |
| 34.159 | 2.62273 | 19.60 |
| 34.955 | 2.56482 | 6.70 |
| 35.635 | 2.51745 | 6.80 |
| 35.892 | 2.49998 | 4.90 |
| 36.271 | 2.47472 | 4.90 |
| 37.010 | 2.42697 | 20.10 |
| 37.278 | 2.41017 | 6.70 |
| 38.806 | 2.31869 | 7.30 |

**Example 18: Preparation of the crystal form F of the compound of formula (I)**

Approximately 50 mg of the compound of formula (I) was weighed and added into ethanol (0.5 mL) to be fully dissolved at 50°C, and a resulting solution or a dilute suspension was filtered through a 0.45 μm nylon filter membrane syringe type filter to obtain a clear solution. The resulting clear solution was cooled to 5°C at a cooling rate of 0.1°C/min. The resulting suspension was centrifuged and filtered using a 0.45 μm nylon filter membrane centrifuge tube at 14,000 rpm to obtain a solid. X-ray powder diffraction detection showed that the product was the crystal form F of the compound of formula (I), with an XRPD pattern as shown in FIG. 36 and positions of characteristic peaks as shown in Table 9. A DSC spectrogram showed a melting point $T_{onset}$ at 187.84°C.

Table 9 XRPD diffraction peak data of the crystal form F of the compound of formula (I)

| 2θ(°) | D(Å) | Relative intensity (%) |
|---|---|---|
| 11.546 | 7.65812 | 1.80 |
| 13.195 | 6.70469 | 2.50 |

(continued)

| 2θ(°) | D(Å) | Relative intensity (%) |
|---|---|---|
| 14.843 | 5.96344 | 20.70 |
| 16.465 | 5.37969 | 100.00 |
| 19.824 | 4.47504 | 25.00 |
| 20.863 | 4.25439 | 6.50 |
| 21.955 | 4.04528 | 95.40 |
| 22.478 | 3.95227 | 13.80 |
| 23.209 | 3.82936 | 13.10 |
| 24.365 | 3.65031 | 17.20 |
| 25.025 | 3.55551 | 7.70 |
| 27.310 | 3.26294 | 26.10 |
| 27.740 | 3.21329 | 19.80 |
| 28.921 | 3.08475 | 15.40 |
| 29.105 | 3.06569 | 4.40 |
| 29.397 | 3.0359 | 12.10 |
| 30.428 | 2.9353 | 18.30 |
| 32.709 | 2.73564 | 28.10 |
| 34.657 | 2.58617 | 5.60 |
| 35.627 | 2.51795 | 4.00 |

**Example 19: Preparation of the crystal form F of the compound of formula (I)**

Approximately 300 mg of the compound of formula (I) was weighed and added into 7.6 mL of acetonitrile to be fully dissolved at 50°C, and a resulting solution was filtered through a 0.45 μm nylon filter membrane syringe type filter to obtain a clear solution. The obtained clear solution was subjected to heat preservation at 50°C for 30 minutes, and then cooled slowly from 50°C to 5°C at a rate of 0.1°C/min. After cooling began, approximately 5 mg of an F crystal was added into a saturated solution to serve as a seed crystal and then stirred for 2 minutes to obtain a suspension. The resulting suspension was stirred at 5°C for one day. The resulting suspension was centrifuged using a 0.45 μm nylon filter membrane centrifuge tube at 14,000 rpm at 5°C, and then placed in an oven for vacuum drying at 25°C for 2 hours to obtain 141 mg of a dry white powder of the F crystal, with a yield of 47%. X-ray powder diffraction detection showed that the product was the crystal form F of the compound of formula (I).

**Example 20: Preparation of the crystal form G of the compound of formula (I)**

100 g of the compound of formula (I) was weighed, added into 1 L of acetone and 300 mL of water, and stirred for dissolution at a temperature controlled at approximately 20-30°C. After clarification, 200 mL of water was added dropwise into the clarified solution until a seed crystal point (the reaction solution became turbid) was reached, and a seed crystal (crystal form F) was added and stirred at 20-30°C for 1 hour. Subsequently, 1 L of water was continuously supplemented, the mixture was stirred for 2 hours and filtered, a filter cake was rinsed with 500 mL of water to obtain a wet product, and the wet product was dried under vacuum at 40-50°C to obtain a product. X-ray powder diffraction detection showed that the product was the crystal form G of the compound of formula (I), with an XRPD pattern as shown in FIG. 39 and positions of characteristic peaks as shown in Table 10. A DSC spectrogram showed an endothermic peak $T_{onset}$ at 144.92°C and a melting point $T_{onset}$ at 187.21°C.

Table 10 XRPD diffraction peak data of the crystal form G of the compound of formula (I)

| 2θ(°) | D(Å) | Relative intensity (%) |
|---|---|---|
| 11.539 | 7.66285Å | 1.80 |

(continued)

| 2θ(°) | D(Å) | Relative intensity (%) |
|---|---|---|
| 13.207 | 6.69841Å | 2.20 |
| 14.858 | 5.95755Å | 16.10 |
| 15.780 | 5.61143Å | 1.20 |
| 16.482 | 5.37407Å | 94.50 |
| 18.635 | 4.75764Å | 2.00 |
| 19.844 | 4.47043Å | 31.30 |
| 20.882 | 4.2506Å | 7.10 |
| 21.037 | 4.2195Å | 1.60 |
| 21.972 | 4.04207Å | 100.00 |
| 22.490 | 3.95013Å | 10.50 |
| 23.228 | 3.82629Å | 7.10 |
| 24.388 | 3.64686Å | 12.60 |
| 24.992 | 3.56009Å | 11.80 |
| 27.333 | 3.26026 | 16.30 |
| 27.762 | 3.21079 | 20.30 |
| 28.938 | 3.08295 | 11.00 |
| 29.124 | 3.0637 | 2.60 |
| 29.412 | 3.03433 | 9.90 |
| 29.920 | 2.98401 | 2.10 |
| 30.447 | 2.93349 | 21.30 |
| 31.313 | 2.8543 | 1.00 |
| 32.731 | 2.73381 | 33.60 |
| 33.705 | 2.65707 | 1.30 |
| 34.060 | 2.63015 | 1.40 |
| 34.649 | 2.58677 | 7.30 |
| 35.647 | 2.51659 | 3.40 |
| 36.257 | 2.47568 | 1.60 |

**Example 21: Competitive suspension tests of the crystal form B, the crystal form F, and the crystal form G of the compound of formula (I)**

To investigate a relative stability relationship among the crystal form B, the crystal form F, and the crystal form G of the compound of formula (I), competitive suspension tests were conducted in different solvents. Results are shown in Table 11.

Approximately 2 mg of the crystal form B, 2 mg of the crystal form F, and 2 mg of the crystal form G were weighed and added into 0.2 mL of saturated solutions of the selected solvents. Resulting suspensions were suspended at 5°C, 25°C, and 50°C for 3 days, respectively. The resulting suspensions were centrifuged, and solid portions were detected by XRPD.

Table 11 Competitive suspension test results of the crystal form B, the crystal form F, and the crystal form G

| Initial crystal forms: the crystal form B, the crystal form F, and the crystal form G | | | | |
|---|---|---|---|---|
| Experiment number | Solvent | XRPD | | |
| | | 5°C, 3 days | 25°C, 3 days | 50°C, 3 days |
| 1 | Isopropanol | Crystal form F | Crystal form F | Crystal form F |
| 2 | Isopropyl acetate | Crystal form F | Crystal form F | Crystal form F |
| 3 | Acetonitrile | Crystal form F | Crystal form F | Crystal form F |

The competitive suspension slurrying tests showed that the crystal forms B, F, and G were all converted into the crystal form F in isopropanol, isopropyl acetate, and acetonitrile. This indicates that the crystal form F was more stable than the crystal forms B and G within a temperature range of 5°C to 50°C.

**Example 22: Stability of the crystal form F of the compound of formula (I)**

A certain amount of the crystal form F of the compound of formula (I) was weighed and placed in an open container at 25°C/92%RH for 1 week. A certain amount of the crystal form F of the compound of formula (I) was weighed and placed in a closed container at 60°C for 1 week. The stability of samples under these conditions was detected by XRPD and HPLC, and whether the samples had color changes was observed. Results are shown in Table 12.

Table 12 Stability test results of the crystal form F of the compound of formula (I)

| Crystal form | Condition | | Purity (%) | Degradation (%) | Color | Crystal form |
|---|---|---|---|---|---|---|
| Crystal form F | Initial | | 99.8 | -- | -- | F |
| | 25°C/92%RH open | 1 week | 99.8 | None | No change | F |
| | 60°C, closed | 1 week | 99.8 | None | No change | F |

As can be seen from the stability test results in Table 12, in the aspect of physical stability, the crystal form F was not changed under both the test conditions. In the aspect of chemical stability, the crystal form F showed no degradation under both the conditions. During the stability test process, the samples of the crystal form F showed no obvious color change.

**Example 23: Hygroscopicity of the crystal form F of the compound of formula (I)**

At 25°C, water absorption and dehydration behaviors of the crystal form F of the compound of formula (I) were studied by DVS testing. A DVS cycle was 40-0-95-0-40%RH. XRPD detection was performed on samples after the DVS testing to determine whether crystal form transformation occurred. Results are shown in Table 13.

Table 13 Water adsorption and desorption test results of the crystal form F of the compound of formula (I)

| **Method** | 40-0-95-0-40%RH, each step equilibrated for 240 min, 25°C | | | |
|---|---|---|---|---|
| Relative humidity at **25°C** | **1st desorption Percentage of weight change** | **1st adsorption Percentage of weight change** | **2nd desorption Percentage of weight change** | **2nd adsorption Percentage of weight change** |
| 0% | 0.1 | 0.1 | 0.3 | 0.3 |
| 10% | 0.3 | 0.2 | 0.3 | 0.2 |
| 20% | 0.1 | 0.2 | 0.4 | 0.2 |
| 30% | 0.1 | 0.1 | 0.1 | 0.0 |
| 40% | 0.2 | 0.2 | 0.4 | 0.0 |
| 50% | // | 0.2 | 0.2 | // |
| 60% | // | 0.2 | 0.2 | // |

(continued)

| Method | 40-0-95-0-40%RH, each step equilibrated for 240 min, 25°C | | | |
|---|---|---|---|---|
| Relative humidity at **25° C** | **1st desorption Percentage of weight change** | **1st adsorption Percentage of weight change** | **2nd desorption Percentage of weight change** | **2nd adsorption Percentage of weight change** |
| 70% | // | 0.2 | 0.2 | // |
| 80% | // | 0.1 | 0.0 | // |
| 90% | // | 0.3 | 0.1 | // |
| 95% | // | 0.1 | 0.1 | // |
| **Hygroscopicity** | | | | |
| **Conclusion** | No hygroscopicity | | | |
| **XRPD after DVS testing** | | | | |
| | Crystal form F | | | |
| Note: "//": not tested<br>no or almost no hygroscopicity: water absorption amount < 0.2%<br>slight hygroscopicity: water absorption amount ≥ 0.2% but < 2%<br>hygroscopicity: water absorption amount ≥ 2% but < 15%<br>high hygroscopicity: water absorption amount ≥ 15%<br>deliquescence: absorption of a sufficient amount of water to form a liquid<br>water absorption amount = water absorption amount at specific RH (80% to 95%) - water absorption amount at 40%RH<br>based on the description of hygroscopicity characteristics and the definition of hygroscopic weight gain in the "9103 Guidelines for the Hygroscopicity of Pharmaceuticals" in Part IV of the 2020 edition of the Chinese Pharmacopoeia | | | | |

**Example 24: Stability of the crystal form A of the compound of formula (I)**

A certain amount of the crystal form A of the compound of formula (I) was weighed and placed in an open container at 25°C/92.5%RH for 1 week. A certain amount of the crystal form A of the compound of formula (I) was weighed and placed in a closed container at 60°C for 1 week. The stability of samples under these conditions was detected by XRPD and HPLC, and whether the samples had color changes was observed. Results are shown in Table 14.

Table 14 Stability test results of the crystal form A of the compound of formula (I)

| Crystal form | Condition | | Purity (%) | Degradation (%) | Color | Crystal form |
|---|---|---|---|---|---|---|
| Crystal form A | Initial | | 99.8 | -- | -- | A |
| | 25°C/92.5%RH, open | 1 week | 99.8 | None | No change | A |
| | 60°C, closed | 1 week | 99.8 | None | No change | A |

As can be seen from the stability test results in Table 14, in the aspect of physical stability, the crystal form A was not changed under both the test conditions. In the aspect of chemical stability, the crystal form A showed no degradation under both the conditions. During the stability test process, the samples of the crystal form A showed no obvious color change.

**Example 25: Hygroscopicity of the crystal form A of the compound of formula (I)**

At 25°C, water absorption and dehydration behaviors of the crystal form A of the compound of formula (I) were studied by DVS testing. A DVS cycle was 40-0-95-0-40%RH. XRPD detection was performed on samples after the DVS testing to determine whether crystal form transformation occurred. Results are shown in Table 15.

Table 15 Water adsorption and desorption test results of the crystal form A of the compound of formula (I)

| **Method** | 40-0-95-0-40%RH, each step equilibrated for 240 min, 25°C | | | |
|---|---|---|---|---|
| Relative humidity at **25°C** | **1st desorption Percentage of weight change** | **1st adsorption Percentage of weight change** | **2nd desorption Percentage of weight change** | **2nd adsorption Percentage of weight change** |
| 0% | 0.3 | 0.3 | 0.2 | 0.2 |
| 10% | 0.3 | 0.1 | 0.3 | 0.3 |
| 20% | 0.1 | 0.3 | 0.2 | 0.3 |
| 30% | 0.1 | 0.3 | 0.3 | 0.1 |
| 40% | 0.3 | 0.1 | 0.4 | 0.3 |
| 50% | // | 0.0 | 0.1 | // |
| 60% | // | 0.4 | 0.4 | // |
| 70% | // | 0.4 | 0.4 | // |
| 80% | // | 0.5 | 0.2 | // |
| 90% | // | 0.5 | 0.6 | // |
| 95% | // | 0.7 | 0.7 | // |
| **Hygroscopicity** | | | | |
| **Conclusion** | Slight hygroscopicity, water absorption of 0.6% from 40% to 95%RH | | | |
| **XRPD after DVS testing** | | | | |
| | Crystal form A | | | |
| **Method** | 40-0-95-0-40%RH, each step equilibrated for 240 min, 25°C | | | |
| Relative humidity at **25°C** | **1st desorption Percentage of weight change** | **1st adsorption Percentage of weight change** | **2nd desorption Percentage of weight change** | **2nd adsorption Percentage of weight change** |
| Note: "//": not tested<br>no or almost no hygroscopicity: water absorption amount < 0.2%<br>slight hygroscopicity: water absorption amount ≥ 0.2% but < 2%<br>hygroscopicity: water absorption amount ≥ 2% but < 15%<br>high hygroscopicity: water absorption amount ≥ 15%<br>deliquescence: absorption of a sufficient amount of water to form a liquid<br>water absorption amount = water absorption amount at specific RH (80% to 95%) - water absorption amount at 40%RH<br>based on the description of hygroscopicity characteristics and the definition of hygroscopic weight gain in the "9103 Guidelines for the Hygroscopicity of Pharmaceuticals" in Part IV of the 2020 edition of the Chinese Pharmacopoeia | | | | |

**Example 26: Stability of the crystal form C of the compound of formula (I)**

A certain amount of the crystal form C of the compound of formula (I) was weighed and placed in an open container at 25°C/92.5%RH for 1 week. A certain amount of the crystal form C of the compound of formula (I) was weighed and placed in a closed container at 60°C for 1 week. The stability of samples under these conditions was detected by XRPD and HPLC, and whether the samples had color changes was observed. Results are shown in Table 16.

Table 16 Stability test results of the crystal form C of the compound of formula (I)

| Crystal form | Condition | | Purity (%) | Degradation (%) | Color | Crystal form |
|---|---|---|---|---|---|---|
| Crystal form C | Initial | | 99.7 | -- | -- | C |
| | 25°C/92.5%RH, open | 1 week | 99.1 | 0.6 | No change | F |
| | 60°C, closed | 1 week | 99.6 | 0.1 | Changed | F |

As can be seen from the stability test results in Table 16, in the aspect of physical stability, the crystal form C was changed into the crystal form F under both the test conditions. In the aspect of chemical stability, the crystal form C was slightly degraded under both the conditions. During the stability test process, the sample of the crystal form C had a color change at 60°C.

**Example 27: Stability of the crystal form D of the compound of formula (I)**

A certain amount of the crystal form D of the compound of formula (I) was weighed and placed in an open container at 25°C/92.5%RH for 1 week. A certain amount of the crystal form D of the compound of formula (I) was weighed and placed in a closed container at 60°C for 1 week. The stability of samples under these conditions was detected by XRPD and HPLC, and whether the samples had color changes was observed. Results are shown in Table 17.

Table 17 Stability test results of the crystal form D of the compound of formula (I)

| Crystal form | Condition | | Purity (%) | Degradation (%) | Color | Crystal form |
|---|---|---|---|---|---|---|
| Crystal form D | Initial | | 99.8 | -- | -- | D |
| | 25°C/92.5%RH, open | 1 week | 99.2 | 0.6 | No change | F |
| | 60°C, closed | 1 week | 98.7 | 1.1 | Changed | D+F |

As can be seen from the stability test results in Table 17, in the aspect of physical stability, the crystal form D was changed into the crystal form F at 25°C/92.5%RH, and was changed into a mixed crystal form of the crystal forms D and F at 60°C. In the aspect of chemical stability, the crystal form D was degraded by 0.6% and 1.1% under both the conditions, respectively. During the stability test process, the sample of the crystal form D had a color change at 60°C.

**Example 28: Stability of the crystal form E of the compound of formula (I)**

A certain amount of the crystal form E of the compound of formula (I) was weighed and placed in an open container at 25°C/92.5%RH for 1 week. A certain amount of the crystal form E of the compound of formula (I) was weighed and placed in a closed container at 60°C for 1 week. The stability of samples under these conditions was detected by XRPD and HPLC, and whether the samples had color changes was observed. Results are shown in Table 18.

Table 18 Stability test results of the crystal form E of the compound of formula (I)

| Crystal form | Condition | | Purity (%) | Degradation (%) | Color | Crystal form |
|---|---|---|---|---|---|---|
| Crystal form E | Initial | | 99.4 | -- | -- | E |
| | 25°C/92.5%RH, open | 1 week | 99.2 | 0.2 | No change | F |
| | 60°C, closed | 1 week | 96.4 | 3.0 | Changed | E+F |

As can be seen from the stability test results in Table 18, in the aspect of physical stability, the crystal form E was changed into the crystal form F at 25°C/92.5%RH, and was changed into a mixed crystal form of the crystal forms E and F at 60°C. In the aspect of chemical stability, the crystal form E was degraded by 0.2% and 3.0% under both the conditions, respectively. During the stability test process, the sample of the crystal form E had a color change at 60°C.

It should be understood that the above examples are all exemplary and are not intended to encompass all possible embodiments covered by the claims. Without departing from the scope of the present disclosure, various modifications and alterations may also be made based on the above examples. Similarly, various technical features of the above examples may also be arbitrarily combined to form other examples of the present disclosure that may not have been explicitly described. Therefore, the above examples merely express several embodiments of the present disclosure and

do not limit the scope of protection of the present disclosure.

## Claims

1. A crystal form A of a compound of formula (I),

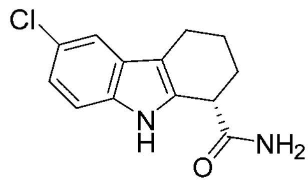

(I) ,

wherein an X-ray powder diffraction pattern expressed in terms of diffraction angle 2θ has characteristic peaks at 12.673, 18.557, 24.951, 27.029, and 33.461; preferably has characteristic peaks at 6.760, 12.673, 13.507, 18.557, 19.315, 21.594, 21.901, 22.214, 24.951, 27.029, and 33.461; preferably has characteristic peaks at 6.760, 12.673, 13.507, 18.557, 19.315, 20.380, 21.594, 21.901, 22.214, 24.774, 24.951, 26.125, 27.029, 33.461, 35.425, and 35.428; preferably has characteristic peaks at 5.547, 6.760, 12.326, 12.673, 13.507, 14.870, 15.162, 18.557, 19.315, 20.380, 21.183, 21.384, 21.594, 21.901, 22.214, 24.774, 24.951, 26.125, 27.029, 33.461, 35.425, and 35.428; and most preferably, the X-ray powder diffraction pattern expressed in terms of diffraction angle 2θ is as shown in FIG. 21.

2. A crystal form B of a compound of formula (I),

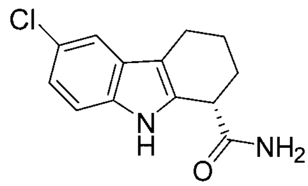

(I) ,

wherein an X-ray powder diffraction pattern expressed in terms of diffraction angle 2θ has characteristic peaks at 10.834, 13.544, 14.962, 21.722, and 30.594; preferably has characteristic peaks at 10.834, 13.544, 14.962, 21.722, 22.809, 23.822, 25.341, 26.812, 29.852, and 30.594; preferably has characteristic peaks at 6.766, 10.140, 10.834, 13.544, 14.962, 19.915, 20.580, 21.722, 22.809, 23.822, 25.341, 26.812, 27.231, 29.852, and 30.594; and most preferably, the X-ray powder diffraction pattern expressed in terms of diffraction angle 2θ is as shown in FIG. 24.

3. A crystal form C of a compound of formula (I),

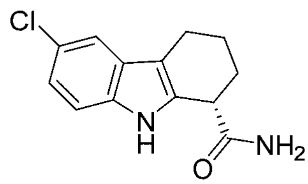

(I) ,

wherein an X-ray powder diffraction pattern expressed in terms of diffraction angle 2θ has characteristic peaks at 10.532, 11.778, 16.749, 19.015, and 21.131; preferably has characteristic peaks at 10.532, 11.778, 16.749, 17.300, 19.015, 21.131, 22.677, 23.402, 24.765, 26.993, and 28.564; preferably has characteristic peaks at 7.462, 10.095, 10.532, 11.778, 14.049, 14.686, 16.749, 17.300, 17.845, 19.015, 21.131, 22.677, 22.878, 23.402, 24.765, 26.993, and 28.564; and most preferably, the X-ray powder diffraction pattern expressed in terms of diffraction angle 2θ is as shown in FIG. 27.

**4.** A crystal form D of a compound of formula (I),

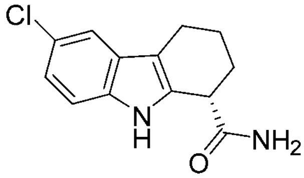

(I)

,

wherein an X-ray powder diffraction pattern expressed in terms of diffraction angle 2θ has characteristic peaks at 10.667, 17.319, 19.284, 21.379, and 25.317; preferably has characteristic peaks at 10.667, 15.726, 17.319, 18.045, 19.284, 19.959, 21.379, 25.317, 27.193, and 28.917; preferably has characteristic peaks at 10.667, 15.726, 16.493, 17.319, 18.045, 19.284, 19.959, 21.379, 23.201, 24.135, 25.317, 26.476, 27.108, 27.193, and 28.917; preferably has characteristic peaks at 9.632, 10.295, 10.667, 11.977, 15.726, 16.493, 17.319, 18.045, 18.341, 19.284, 19.959, 21.379, 23.201, 24.135, 25.317, 25.572, 26.476, 27.108, 27.193, and 28.917; and most preferably, the X-ray powder diffraction pattern expressed in terms of diffraction angle 2θ is as shown in FIG. 30.

**5.** A crystal form E of a compound of formula (I),

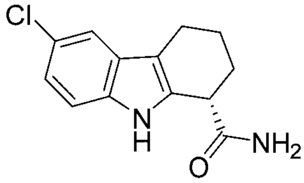

(I)

,

wherein an X-ray powder diffraction pattern expressed in terms of diffraction angle 2θ has characteristic peaks at 11.989, 16.248, 19.659, 19.973, and 23.014; preferably has characteristic peaks at 9.992, 11.989, 16.248, 19.414, 19.659, 19.973, 21.912, 22.448, 22.632, 23.014, 24.190, and 30.862; preferably has characteristic peaks at 9.992, 11.989, 16.248, 19.414, 19.659, 19.973, 21.175, 21.912, 22.448, 22.632, 23.014, 24.190, 25.095, 26.375, 30.862, 32.739, 34.159, and 37.010; preferably has characteristic peaks at 9.992, 11.989, 14.378, 16.248, 19.414, 19.659, 19.973, 21.175, 21.912, 22.448, 22.632, 23.014, 23.880, 24.190, 25.095, 26.375, 28.530, 30.862, 31.163, 32.174, 32.739, 34.159, and 37.010; and most preferably, the X-ray powder diffraction pattern expressed in terms of diffraction angle 2θ is as shown in FIG. 33.

**6.** A crystal form F of a compound of formula (I),

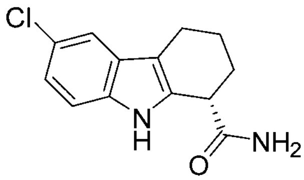

(I)

,

wherein an X-ray powder diffraction pattern expressed in terms of diffraction angle 2θ has characteristic peaks at 16.465, 19.824, 21.955, 27.310, and 32.709; preferably has characteristic peaks at 14.843, 16.465, 19.824, 21.955, 24.365, 27.310, 27.740, 28.921, 30.428, and 32.709; preferably has characteristic peaks at 11.546, 13.195, 14.843, 16.465, 19.824, 21.955, 22.478, 23.209, 24.365, 27.310, 27.740, 28.921, 29.397, 30.428, and 32.709; and most preferably, the X-ray powder diffraction pattern expressed in terms of diffraction angle 2θ is as shown in FIG. 36.

**7.** A crystal form G of a compound of formula (I),

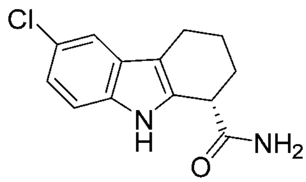

(I)

,

wherein an X-ray powder diffraction pattern expressed in terms of diffraction angle 2θ has characteristic peaks at 16.482, 19.844, 21.972, 27.762, 30.447, and 32.731; preferably has characteristic peaks at 14.858, 16.482, 19.844, 21.972, 24.388, 24.992, 27.333, 27.762, 28.938, 30.447, and 32.731; preferably has characteristic peaks at 11.539, 13.207, 14.858, 15.780, 16.482, 19.844, 21.972, 22.490, 24.388, 24.992, 27.333, 27.762, 28.938, 29.412, 30.447, and 32.731; and most preferably, the X-ray powder diffraction pattern expressed in terms of diffraction angle 2θ is as shown in FIG. 39.

**8.** The crystal form according to any one of claims 1-7, wherein the 2θ angle has an error margin of ±0.20 degrees.

**9.** A method for preparing the crystal form A of the compound of formula (I) according to claim 1, comprising: mixing the compound of formula (I) with a solvent 1, stirring, and filtering; wherein
preferably, the solvent 1 is selected from an alcohol solvent, preferably a $C_{1-4}$ alcohol, more preferably one or more of methanol, ethanol, and isopropanol, and most preferably ethanol.

**10.** A method for preparing the crystal form B of the compound of formula (I) according to claim 2, comprising: dissolving the compound of formula (I) in a solvent 2, and adding an antisolvent 3 to precipitate a crystal; wherein

preferably, the solvent 2 is selected from an alcohol solvent, preferably a $C_{1-4}$ alcohol, more preferably one or more of methanol, ethanol, and isopropanol, and most preferably methanol; and
preferably, the antisolvent 3 is selected from water.

**11.** A method for preparing the crystal form C of the compound of formula (I) according to claim 3, comprising: dissolving the compound of formula (I) in a solvent 4, and adding an antisolvent 5 to precipitate a crystal; wherein

preferably, the solvent 4 is selected from an ester solvent, preferably one or more of ethyl acetate, methyl acetate, butyl acetate, and isobutyl acetate, and more preferably ethyl acetate; and
preferably, the antisolvent 5 is selected from an alkane solvent, preferably one or more of n-hexane, cyclohexane, pentane, dimethylpentane, and n-heptane, and more preferably n-heptane.

**12.** A method for preparing the crystal form D of the compound of formula (I) according to claim 4, comprising: mixing the compound of formula (I) with a solvent 6, heating for dissolution, cooling, and filtering; wherein
preferably, the solvent 6 is selected from a ketone solvent, preferably one or more of acetone, butanone, pentanone, hexanone, and cyclohexanone, and more preferably acetone.

**13.** A method for preparing the crystal form E of the compound of formula (I) according to claim 5, comprising: mixing the compound of formula (I) with a solvent 7, stirring, and filtering; wherein
preferably, the solvent 7 is selected from an ether solvent, preferably one or more of ethyl ether, propyl ether, butyl ether, anisole, petroleum ether, isopropyl ether, and 1,4-dioxane, and more preferably 1,4-dioxane.

**14.** A method for preparing the crystal form F of the compound of formula (I) according to claim 6, comprising: mixing the compound of formula (I) with a solvent 8, heating for dissolution, cooling, and filtering; wherein
preferably, the solvent 8 is selected from an alcohol solvent, preferably a $C_{1-4}$ alcohol, more preferably one or more of methanol, ethanol, and isopropanol, and most preferably ethanol.

**15.** A method for preparing the crystal form G of the compound of formula (I) according to claim 7, comprising: mixing the compound of formula (I) with a solvent 9 and a solvent 10, stirring for dissolution, continually adding the solvent 10 and adding a seed crystal, stirring, continually adding the solvent 10, stirring, and filtering; wherein

preferably, the solvent 9 is selected from a ketone solvent, preferably one or more of acetone, butanone,

pentanone, hexanone, and cyclohexanone, and more preferably acetone; and
preferably, the solvent 10 is selected from water.

**16.** A pharmaceutical composition prepared from the crystal form A of the compound of formula (I) according to claim 1, the crystal form B of the compound of formula (I) according to claim 2, the crystal form C of the compound of formula (I) according to claim 3, the crystal form D of the compound of formula (I) according to claim 4, the crystal form E of the compound of formula (I) according to claim 5, the crystal form F of the compound of formula (I) according to claim 6, or the crystal form G of the compound of formula (I) according to claim 7.

**17.** A pharmaceutical composition, comprising the crystal form A of the compound of formula (I) according to claim 1, the crystal form B of the compound of formula (I) according to claim 2, the crystal form C of the compound of formula (I) according to claim 3, the crystal form D of the compound of formula (I) according to claim 4, the crystal form E of the compound of formula (I) according to claim 5, the crystal form F of the compound of formula (I) according to claim 6, or the crystal form G of the compound of formula (I) according to claim 7, and optionally a pharmaceutically acceptable excipient.

**18.** A method for preparing a pharmaceutical composition, comprising a step of mixing the crystal form A of the compound of formula (I) according to claim 1, the crystal form B of the compound of formula (I) according to claim 2, the crystal form C of the compound of formula (I) according to claim 3, the crystal form D of the compound of formula (I) according to claim 4, the crystal form E of the compound of formula (I) according to claim 5, the crystal form F of the compound of formula (I) according to claim 6, or the crystal form G of the compound of formula (I) according to claim 7 with a pharmaceutically acceptable excipient.

**19.** Use of the crystal form A of the compound of formula (I) according to claim 1, the crystal form B of the compound of formula (I) according to claim 2, the crystal form C of the compound of formula (I) according to claim 3, the crystal form D of the compound of formula (I) according to claim 4, the crystal form E of the compound of formula (I) according to claim 5, the crystal form F of the compound of formula (I) according to claim 6, the crystal form G of the compound of formula (I) according to claim 7, the composition according to claims 16 or 17, or a composition prepared by the method according to claim 18 in preparation of a drug for treating and/or preventing a disease related to RNA m6A regulation.

**20.** The use according to claim 19, wherein the disease related to RNA m6A regulation is a disease or pain related to endocrine and metabolic diseases, neurological diseases, tumors, cardiovascular diseases, infections, immune system diseases, urogenital diseases, dermatological and musculoskeletal diseases, respiratory diseases, genetic diseases and malformations, digestive system diseases, stomatognathic diseases, and vascular and lymphatic system diseases, and preferably is a hand-foot syndrome, a hand-foot skin reaction, a cancer, or a dermatological disease.

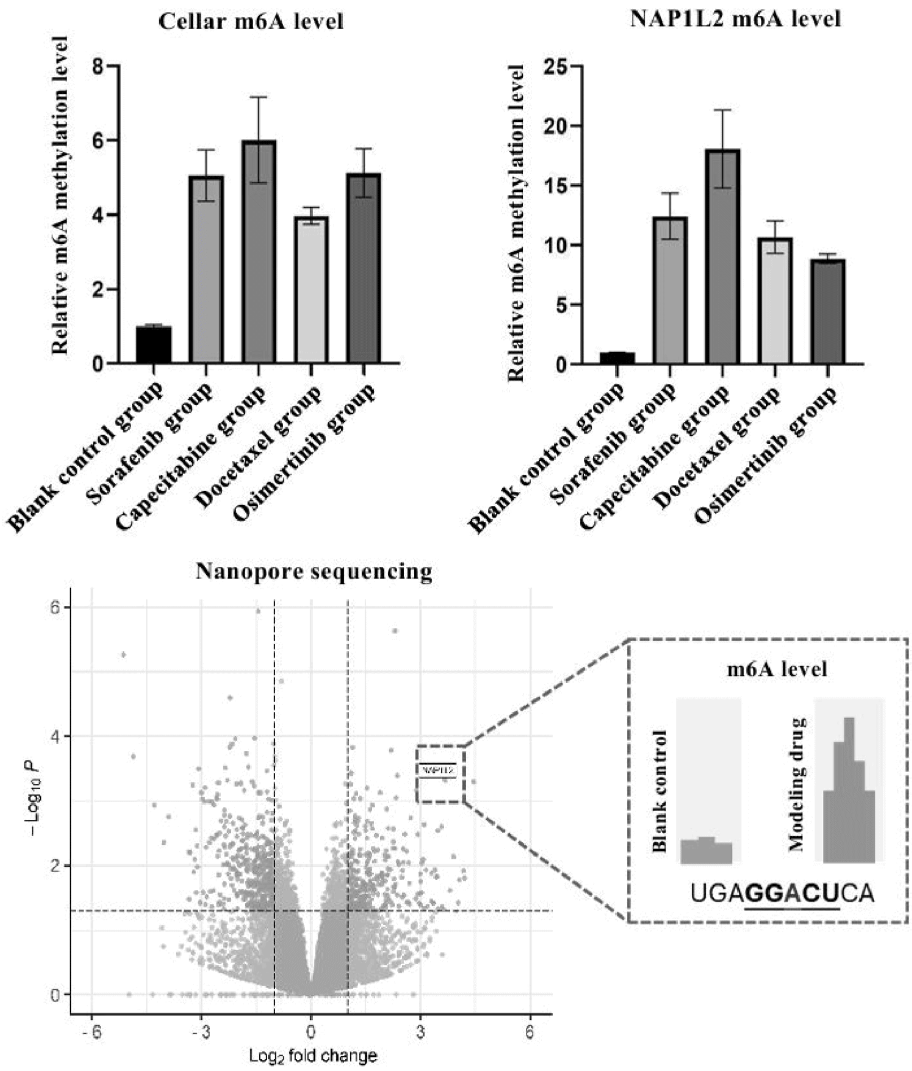
Cellar m6A level
Relative m6A methylation level
Blank control group
Sorafenib group
Capecitabine group
Docetaxel group
Osimertinib group
NAP1L2 m6A level
Nanopore sequencing
$-Log_{10}$ P
$Log_2$ fold change
m6A level
Blank control
Modeling drug
UGAGGACUCA

FIG. 1

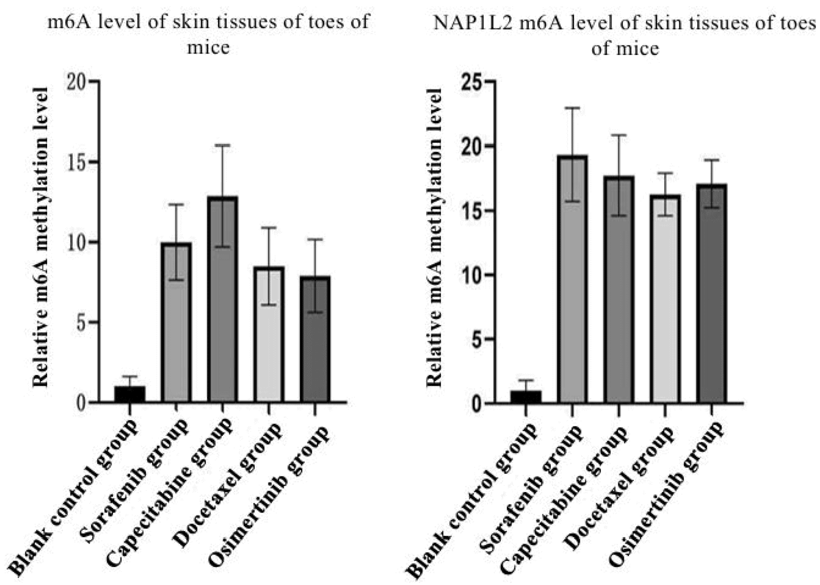
m6A level of skin tissues of toes of mice
NAP1L2 m6A level of skin tissues of toes of mice
Relative m6A methylation level
Blank control group
Sorafenib group
Capecitabine group
Docetaxel group
Osimertinib group

FIG. 2

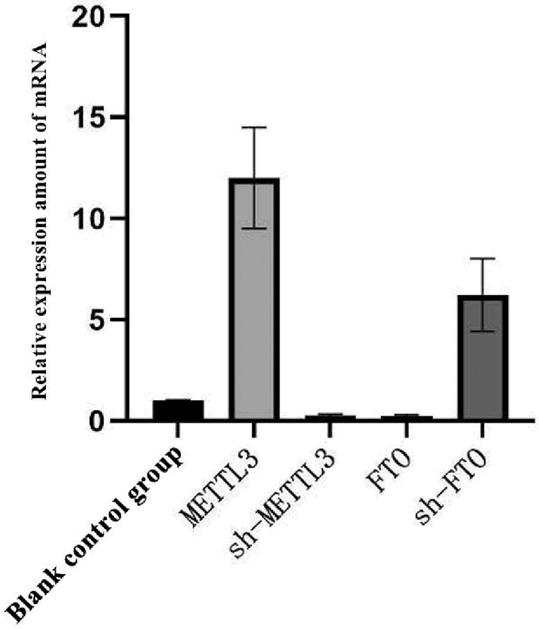
Relative expression amount of mRNA
20
15
10
5
0
Blank control group
METTL3
sh-METTL3
FTO
sh-FTO

FIG. 3

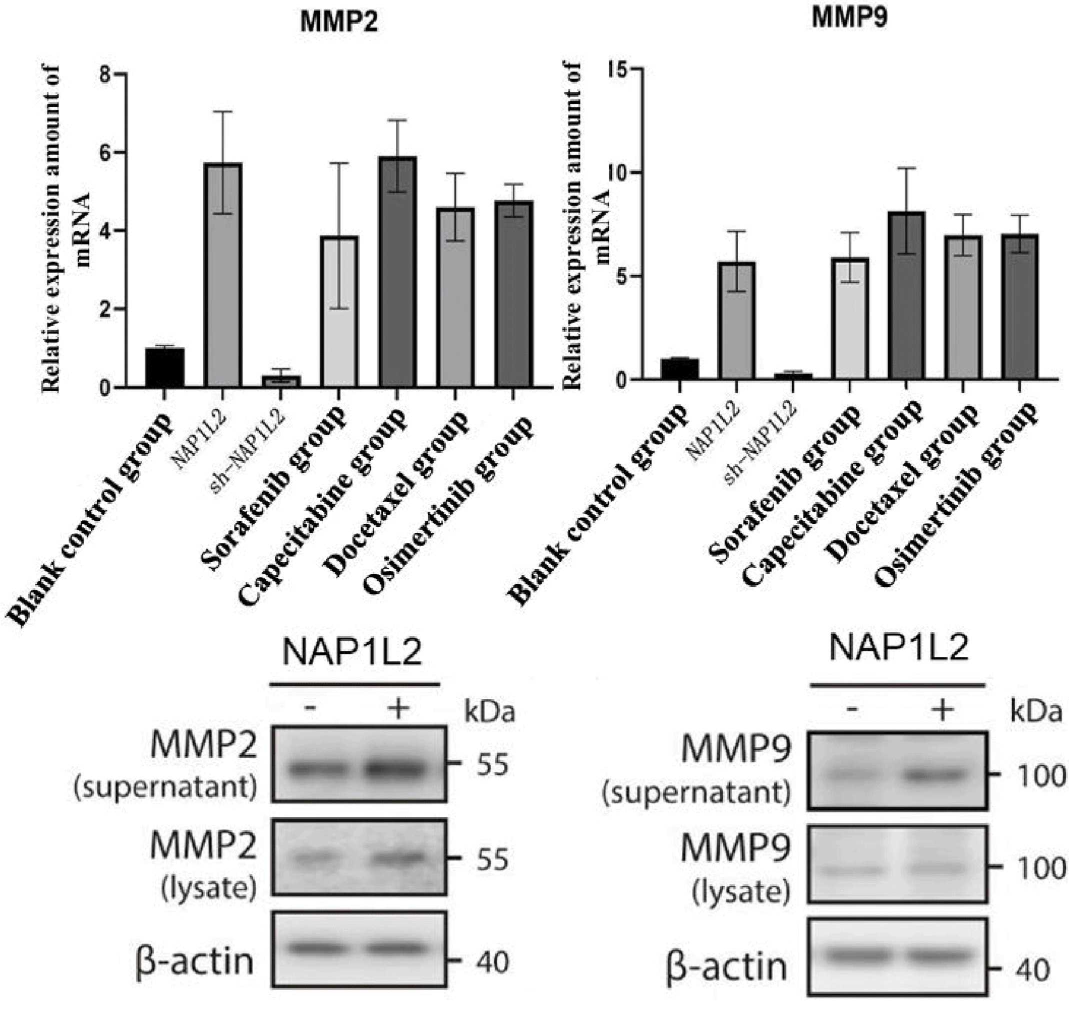
MMP2
MMP9
Relative expression amount of mRNA
Blank control group
NAP1L2
sh-NAP1L2
Sorafenib group
Capecitabine group
Docetaxel group
Osimertinib group
NAP1L2
-
+
kDa
MMP2 (supernatant)
55
MMP2 (lysate)
55
β-actin
40
MMP9 (supernatant)
100
MMP9 (lysate)
100
β-actin
40

FIG. 4

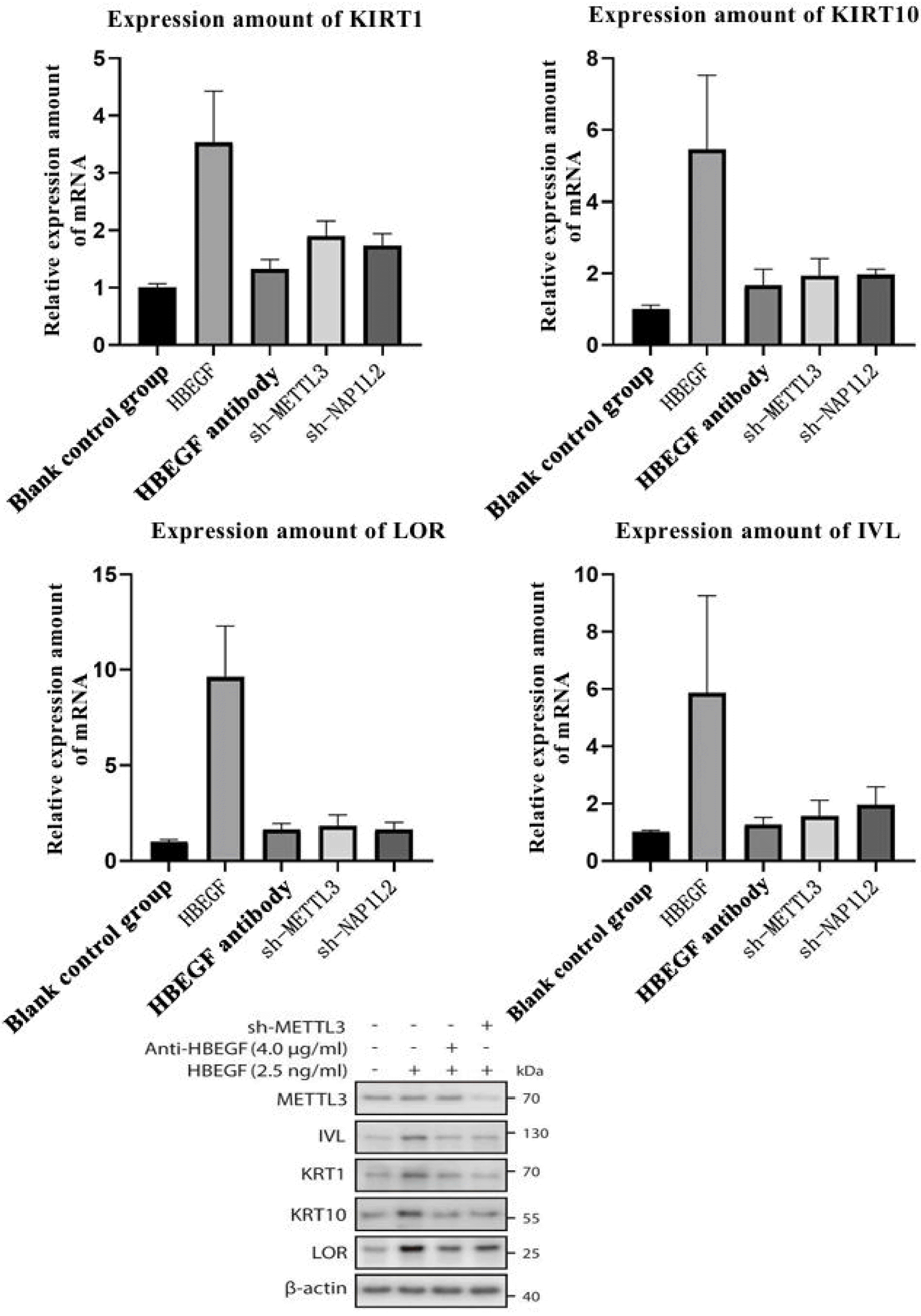
Expression amount of KIRT1
Relative expression amount of mRNA
5
4
3
2
1
0
Blank control group
HBEGF
HBEGF antibody
sh-METTL3
sh-NAP1L2
Expression amount of KIRT10
Relative expression amount of mRNA
8
6
4
2
0
Blank control group
HBEGF
HBEGF antibody
sh-METTL3
sh-NAP1L2
Expression amount of LOR
Relative expression amount of mRNA
15
10
5
0
Blank control group
HBEGF
HBEGF antibody
sh-METTL3
sh-NAP1L2
Expression amount of IVL
Relative expression amount of mRNA
10
8
6
4
2
0
Blank control group
HBEGF
HBEGF antibody
sh-METTL3
sh-NAP1L2
sh-METTL3 - - - +
Anti-HBEGF (4.0 μg/ml) - - + -
HBEGF (2.5 ng/ml) - + + +
kDa
METTL3
70
IVL
130
KRT1
70
KRT10
55
LOR
25
β-actin
40

FIG. 5

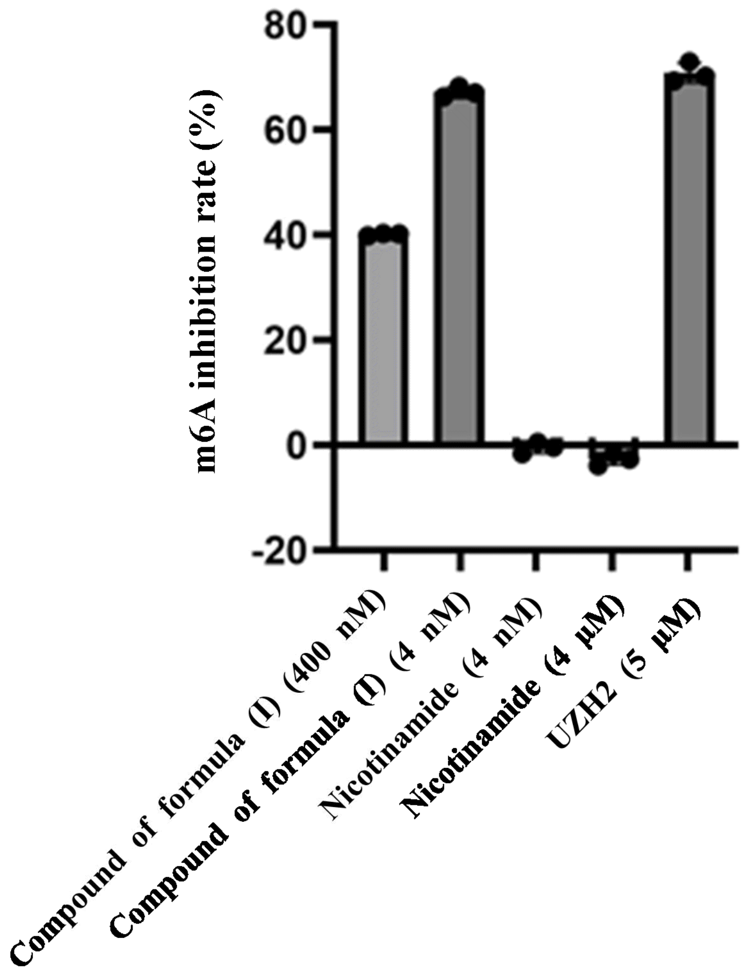
m6A inhibition rate (%)
80
60
40
20
0
-20
Compound of formula (I) (400 nM)
Compound of formula (I) (4 nM)
Nicotinamide (4 nM)
Nicotinamide (4 μM)
UZH2 (5 μM)

FIG.6

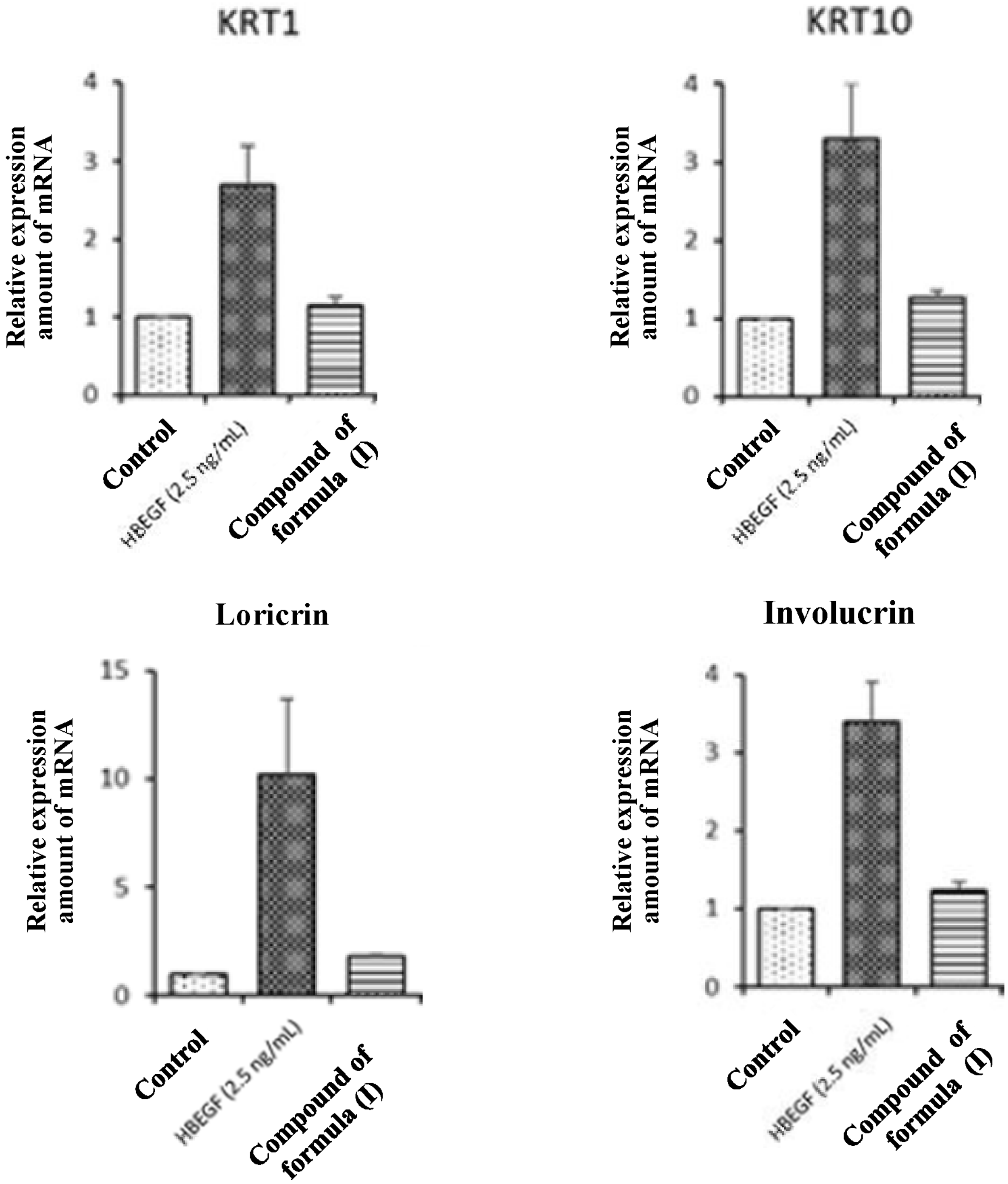
KRT1
Relative expression amount of mRNA
0
1
2
3
4
Control
HBEGF (2.5 ng/mL)
Compound of formula (I)
KRT10
Relative expression amount of mRNA
0
1
2
3
4
Control
HBEGF (2.5 ng/mL)
Compound of formula (I)
Loricrin
Relative expression amount of mRNA
0
5
10
15
Control
HBEGF (2.5 ng/mL)
Compound of formula (I)
Involucrin
Relative expression amount of mRNA
0
1
2
3
4
Control
HBEGF (2.5 ng/mL)
Compound of formula (I)

FIG. 7

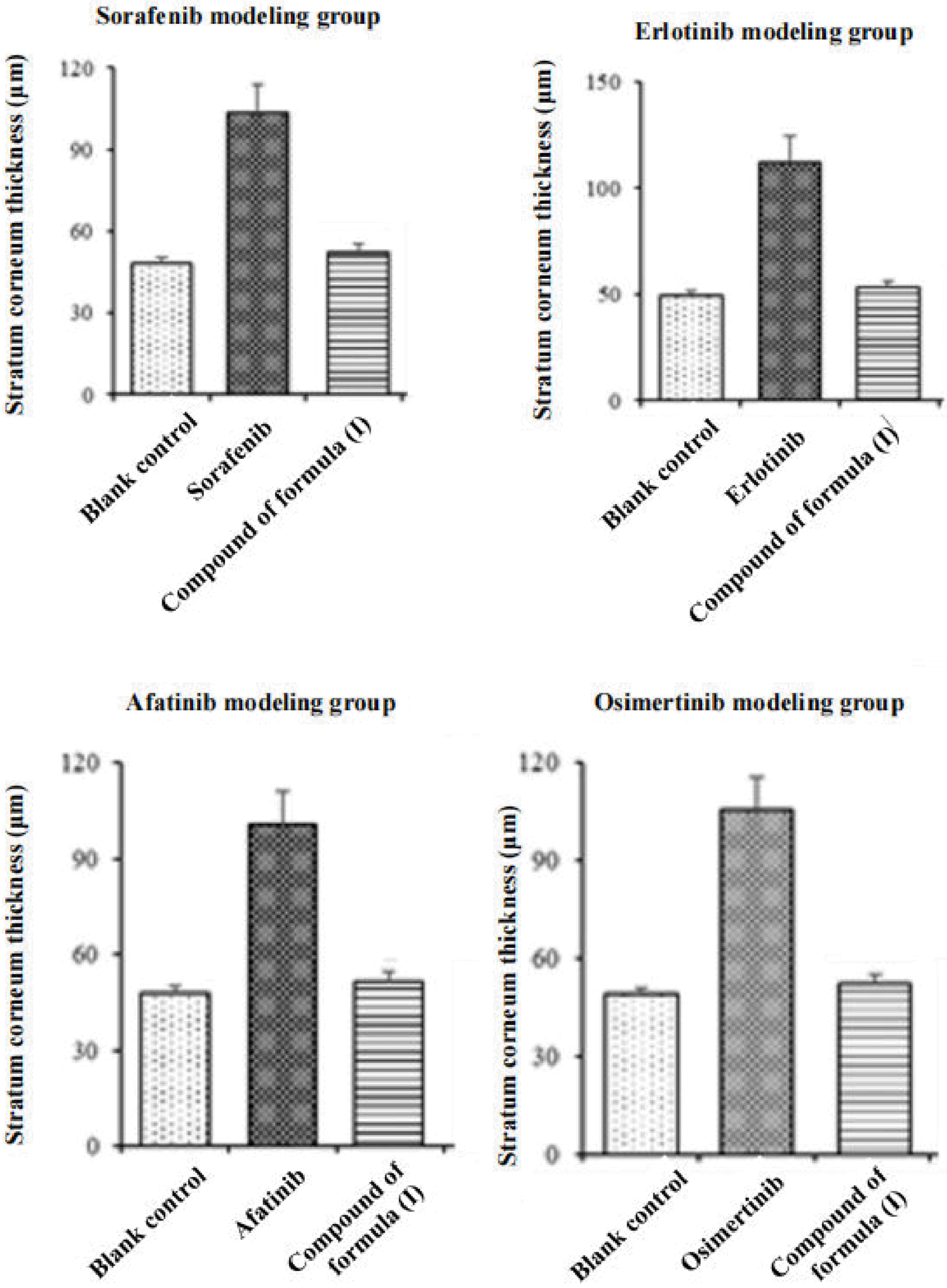
Sorafenib modeling group
Stratum corneum thickness (μm)
120
90
60
30
0
Blank control
Sorafenib
Compound of formula (I)
Erlotinib modeling group
Stratum corneum thickness (μm)
150
100
50
0
Blank control
Erlotinib
Compound of formula (I)
Afatinib modeling group
Stratum corneum thickness (μm)
120
90
60
30
0
Blank control
Afatinib
Compound of formula (I)
Osimertinib modeling group
Stratum corneum thickness (μm)
120
90
60
30
0
Blank control
Osimertinib
Compound of formula (I)

FIG. 8

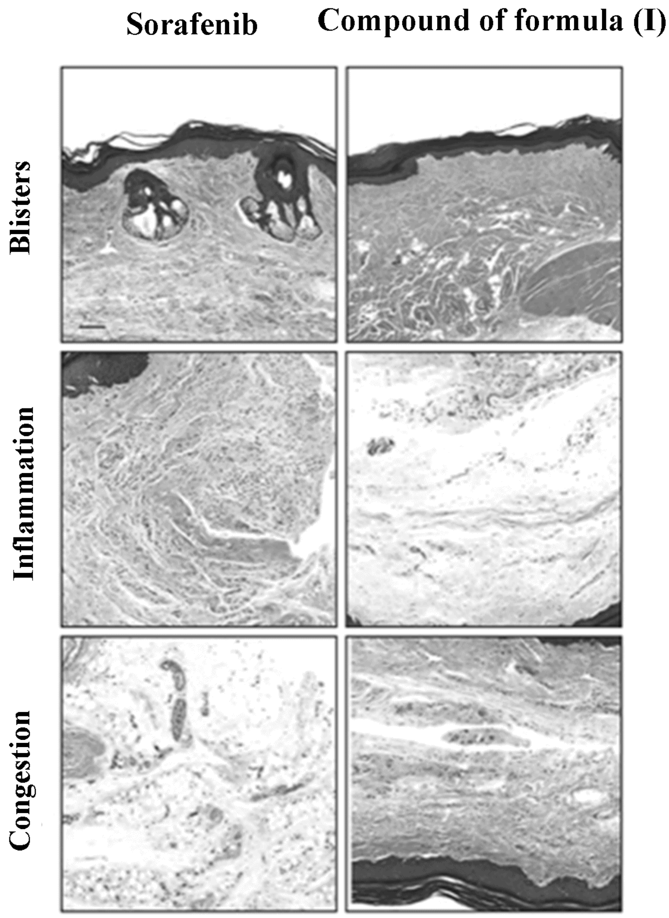
Sorafenib
Compound of formula (I)
Blisters
Inflammation
Congestion

FIG. 9

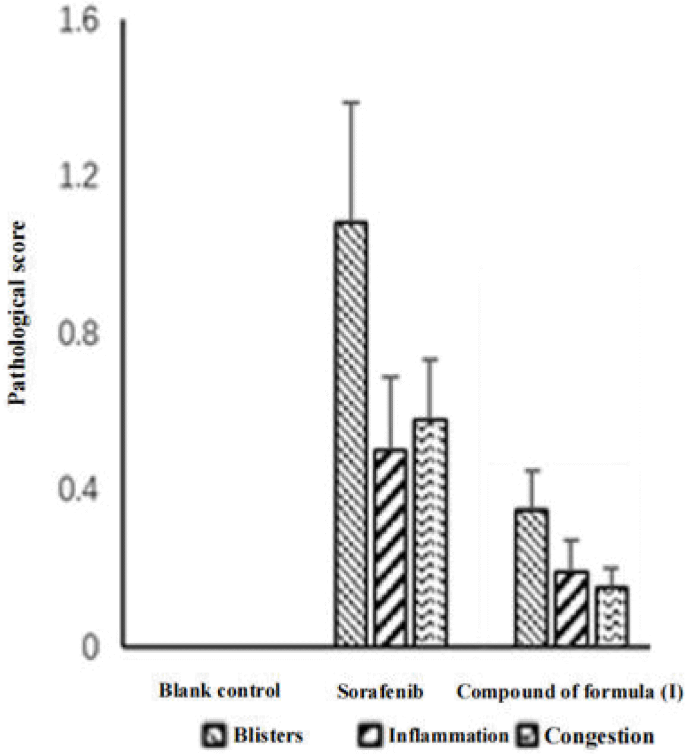
1.6
1.2
0.8
0.4
0
Pathological score
Blank control
Sorafenib
Compound of formula (I)
Blisters
Inflammation
Congestion

FIG.10

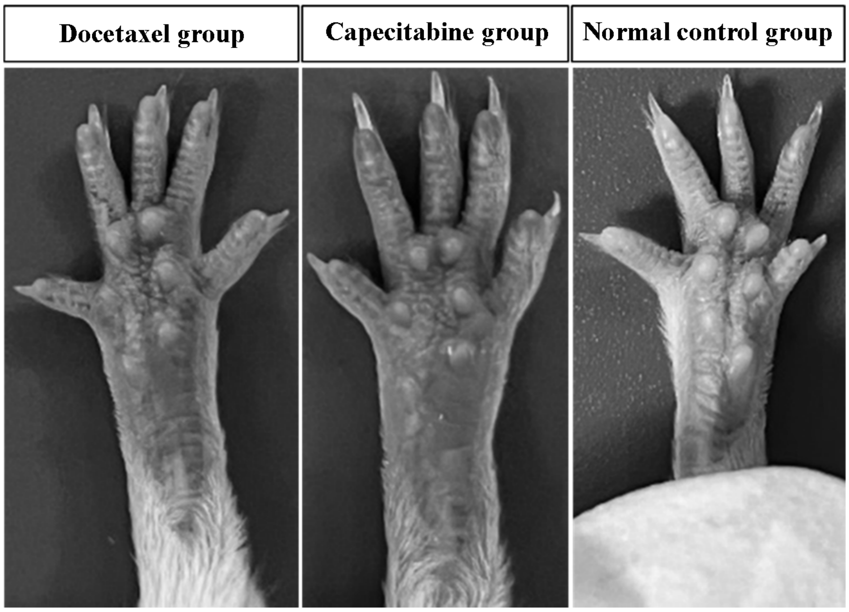


FIG. 11

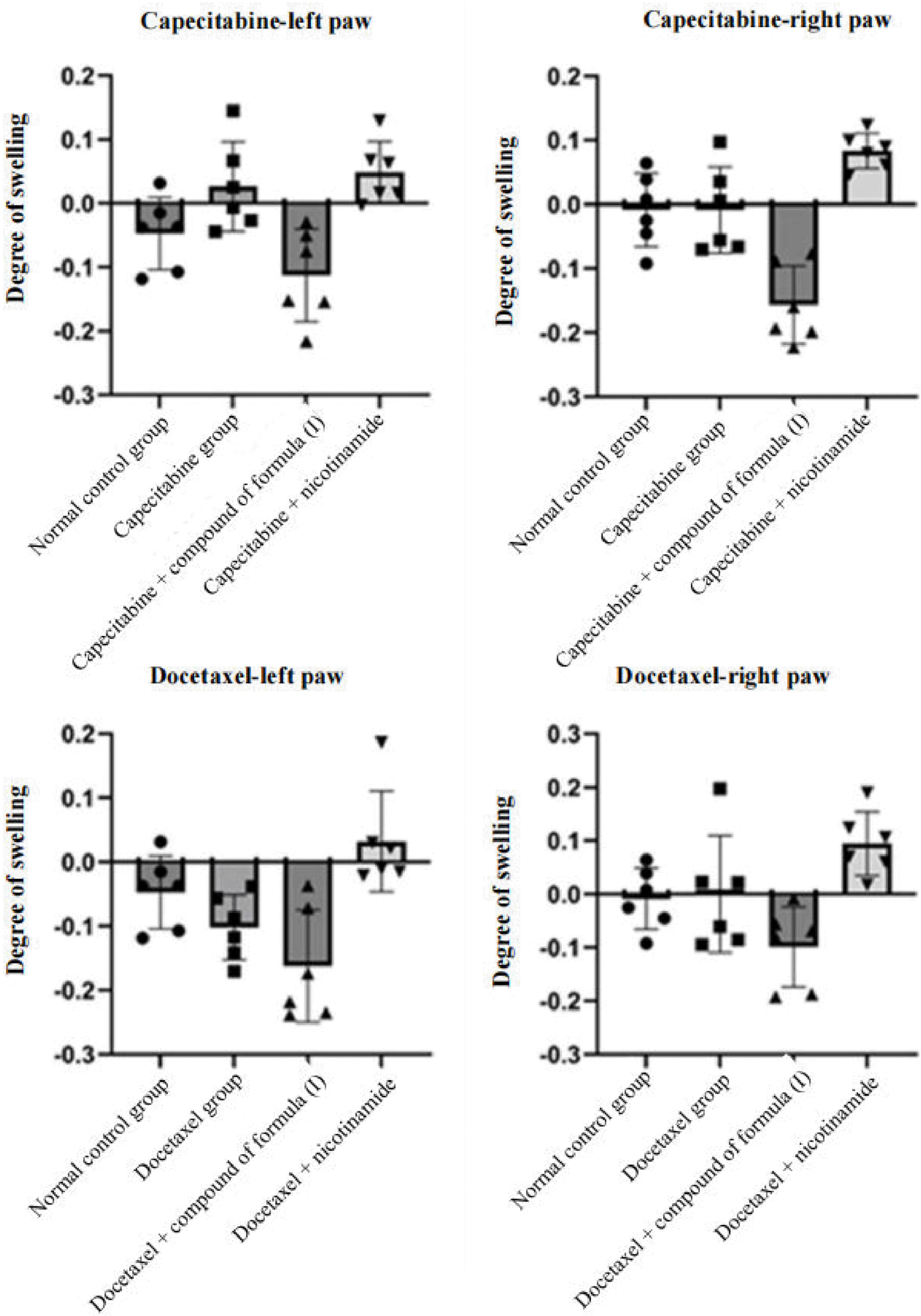
Capecitabine-left paw
Degree of swelling
0.2
0.1
0.0
-0.1
-0.2
-0.3
Normal control group
Capecitabine group
Capecitabine + compound of formula (I)
Capecitabine + nicotinamide
Capecitabine-right paw
Degree of swelling
0.2
0.1
0.0
-0.1
-0.2
-0.3
Normal control group
Capecitabine group
Capecitabine + compound of formula (I)
Capecitabine + nicotinamide
Docetaxel-left paw
Degree of swelling
0.2
0.1
0.0
-0.1
-0.2
-0.3
Normal control group
Docetaxel group
Docetaxel + compound of formula (I)
Docetaxel + nicotinamide
Docetaxel-right paw
Degree of swelling
0.3
0.2
0.1
0.0
-0.1
-0.2
-0.3
Normal control group
Docetaxel group
Docetaxel + compound of formula (I)
Docetaxel + nicotinamide

FIG. 12

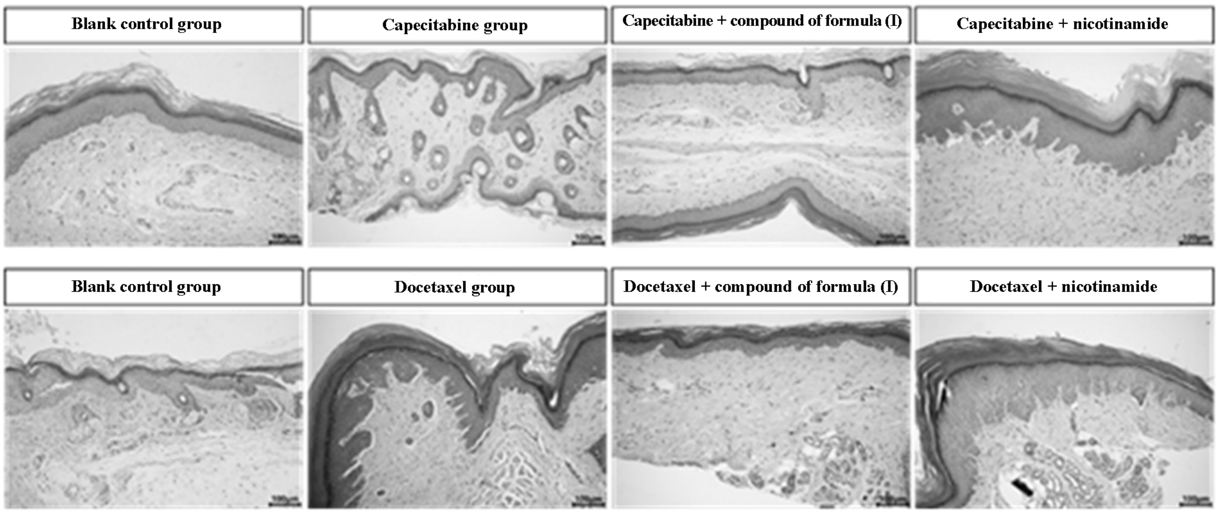
Blank control group
Capecitabine group
Capecitabine + compound of formula (I)
Capecitabine + nicotinamide
Blank control group
Docetaxel group
Docetaxel + compound of formula (I)
Docetaxel + nicotinamide

FIG. 13

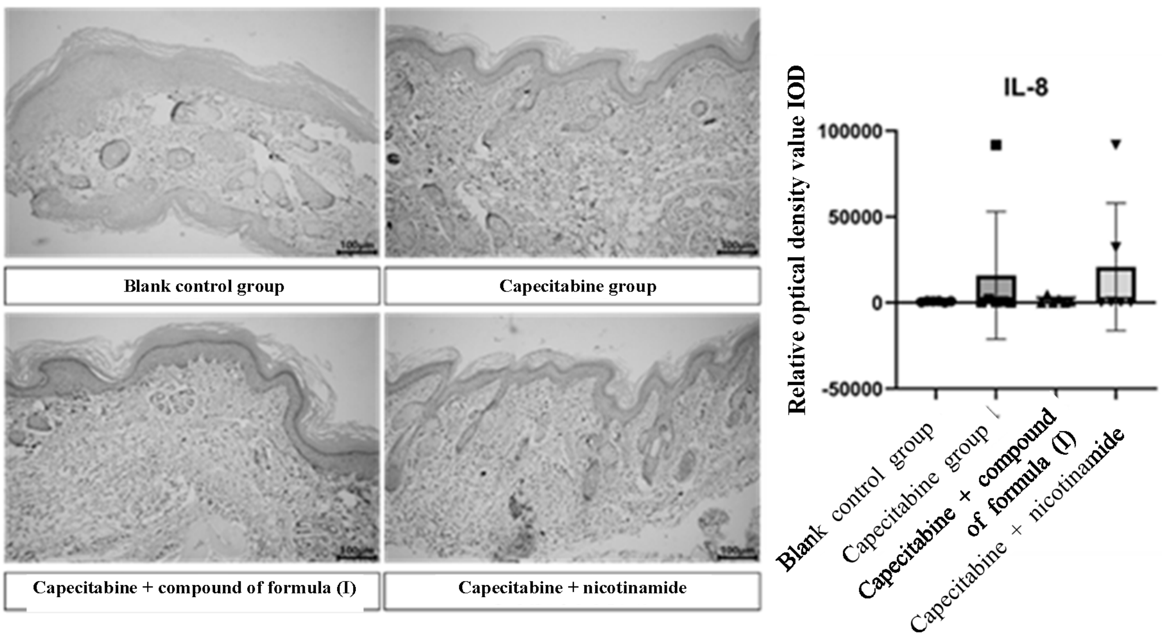
Blank control group
Capecitabine group
Capecitabine + compound of formula (I)
Capecitabine + nicotinamide
IL-8
Relative optical density value IOD
100000
50000
0
-50000
Blank control group
Capecitabine group
Capecitabine + compound of formula (I)
Capecitabine + nicotinamide

FIG. 14

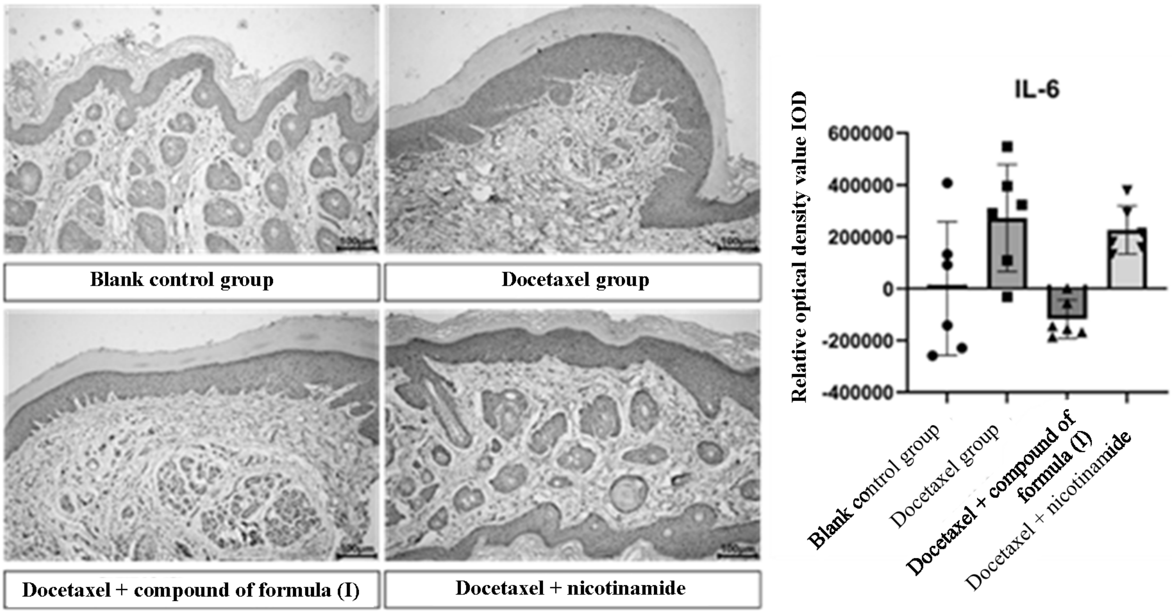
Blank control group
Docetaxel group
Docetaxel + compound of formula (I)
Docetaxel + nicotinamide
IL-6
Relative optical density value IOD
600000
400000
200000
0
-200000
-400000
Blank control group
Docetaxel group
Docetaxel + compound of formula (I)
Docetaxel + nicotinamide

FIG. 15

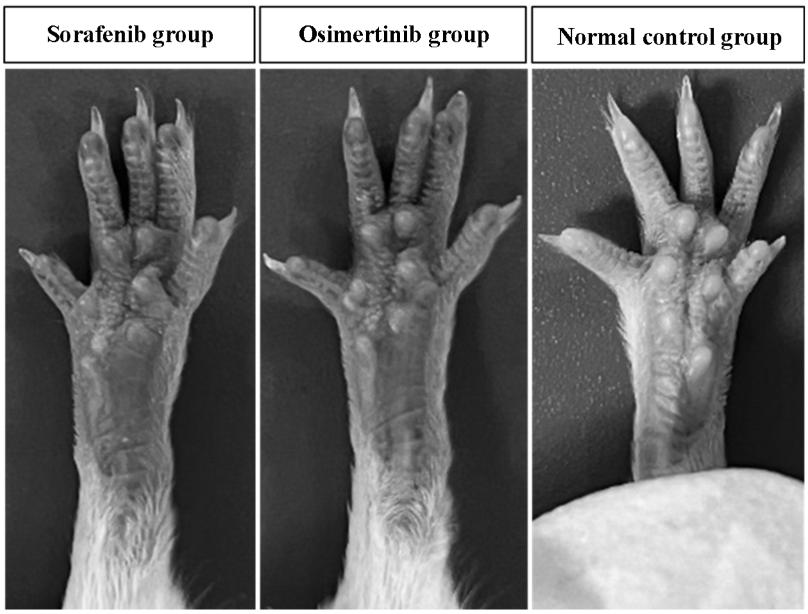
Sorafenib group
Osimertinib group
Normal control group

FIG. 16

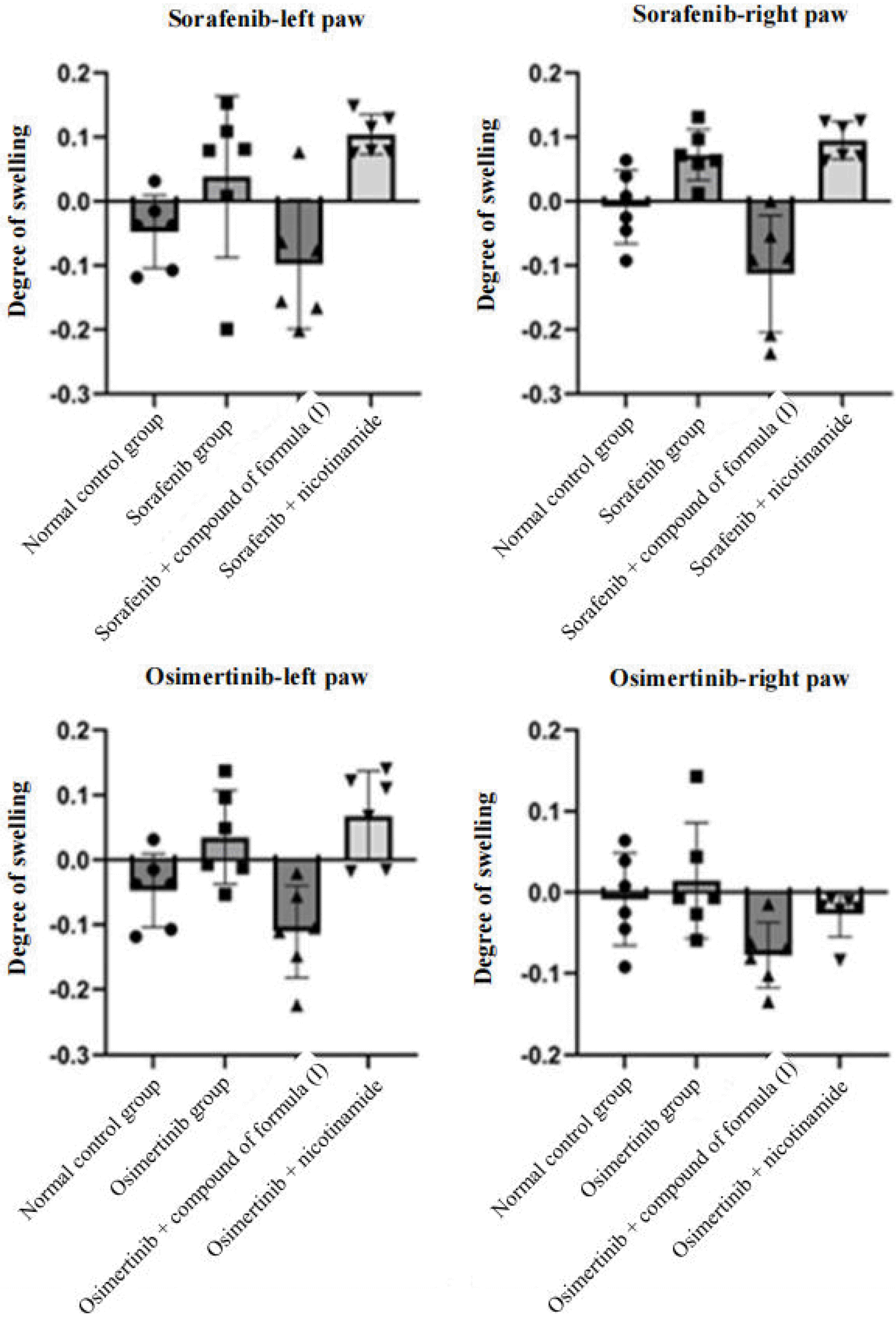
Sorafenib-left paw
Sorafenib-right paw
Degree of swelling
0.2
0.1
0.0
-0.1
-0.2
-0.3
Normal control group
Sorafenib group
Sorafenib + compound of formula (I)
Sorafenib + nicotinamide
Osimertinib-left paw
Osimertinib-right paw
Osimertinib group
Osimertinib + compound of formula (I)
Osimertinib + nicotinamide

FIG. 17

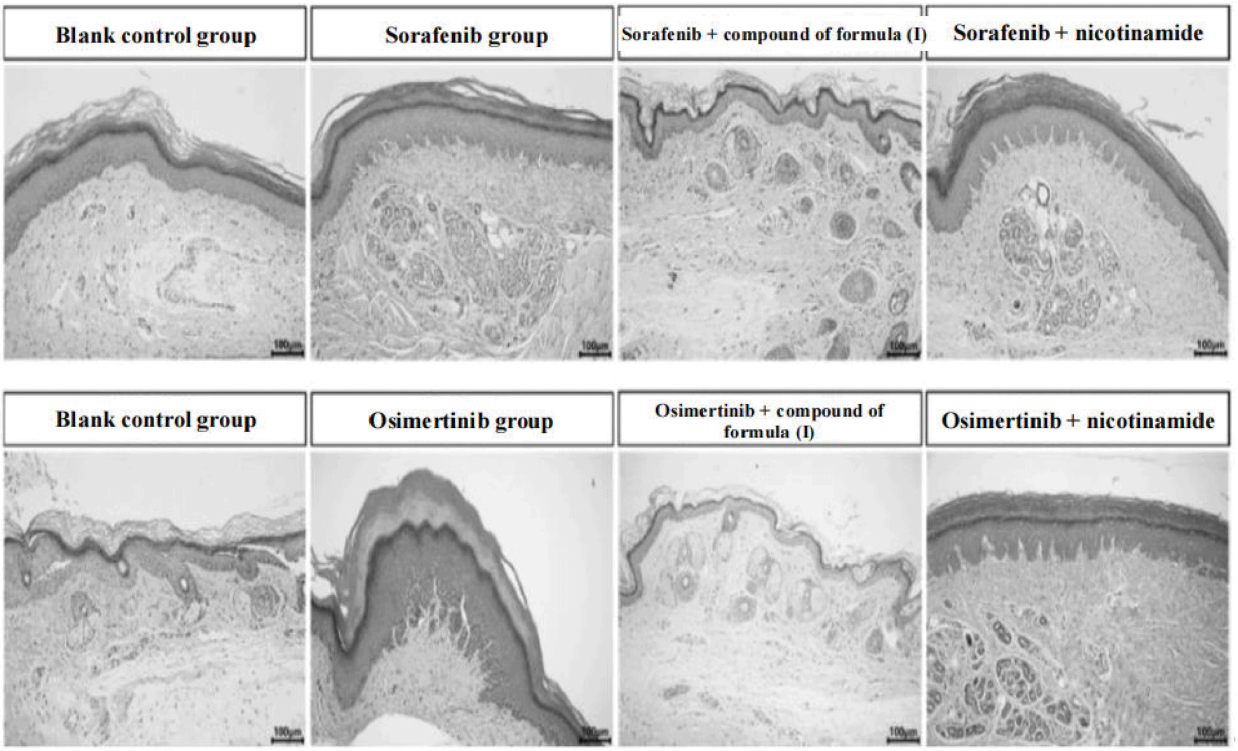
Blank control group
Sorafenib group
Sorafenib + compound of formula (I)
Sorafenib + nicotinamide
Blank control group
Osimertinib group
Osimertinib + compound of formula (I)
Osimertinib + nicotinamide

FIG. 18

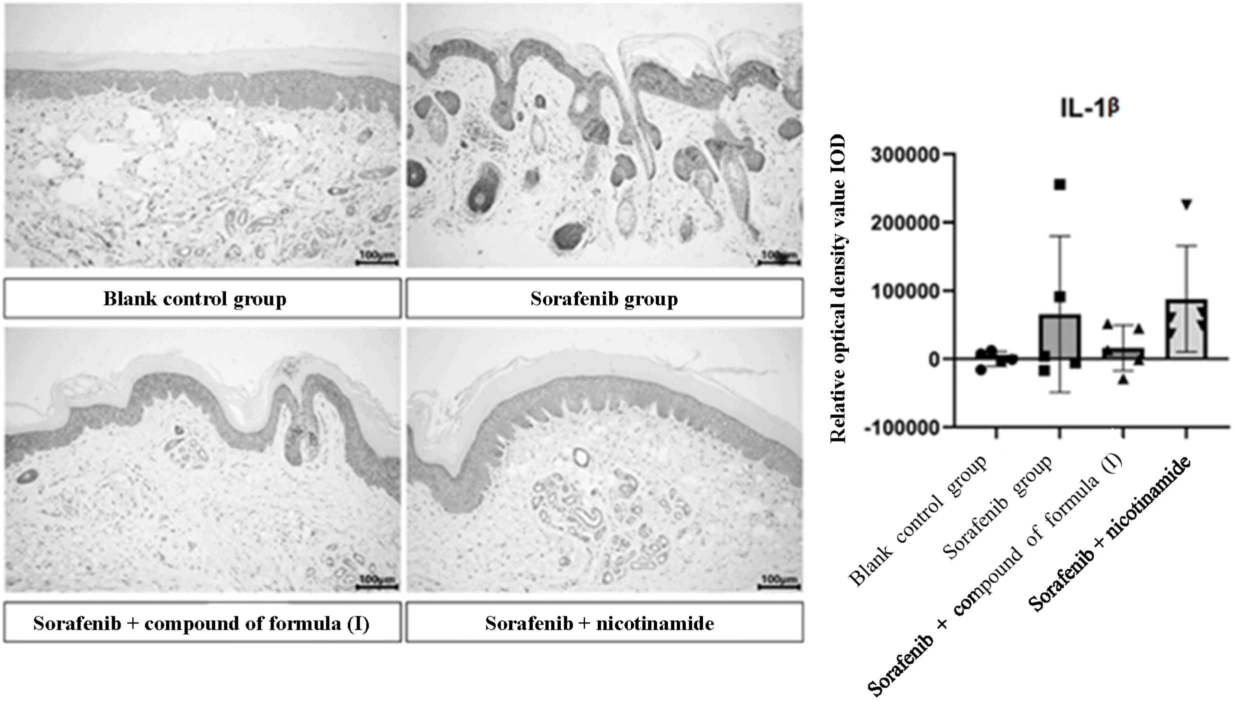
Blank control group
Sorafenib group
Sorafenib + compound of formula (I)
Sorafenib + nicotinamide
IL-1β
Relative optical density value IOD
300000
200000
100000
0
-100000
Blank control group
Sorafenib group
Sorafenib + compound of formula (I)
Sorafenib + nicotinamide

FIG. 19

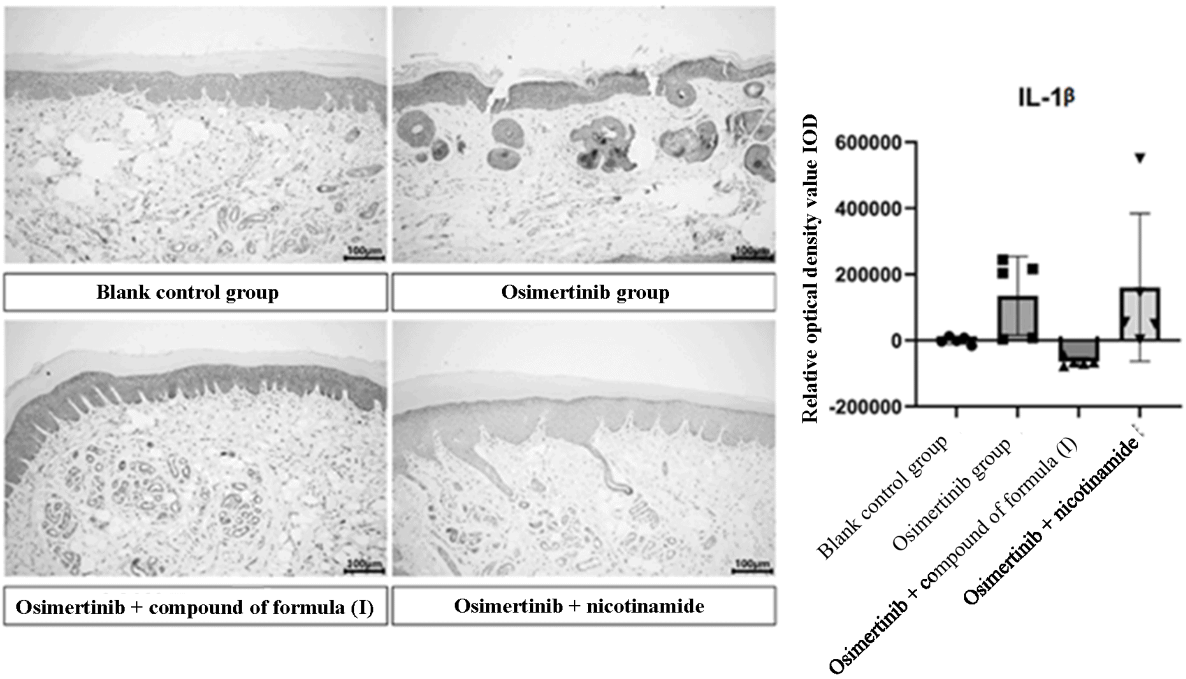
Blank control group
Osimertinib group
Osimertinib + compound of formula (I)
Osimertinib + nicotinamide
IL-1β
Relative optical density value IOD
600000
400000
200000
0
-200000
Blank control group
Osimertinib group
Osimertinib + compound of formula (I)
Osimertinib + nicotinamide

FIG. 20

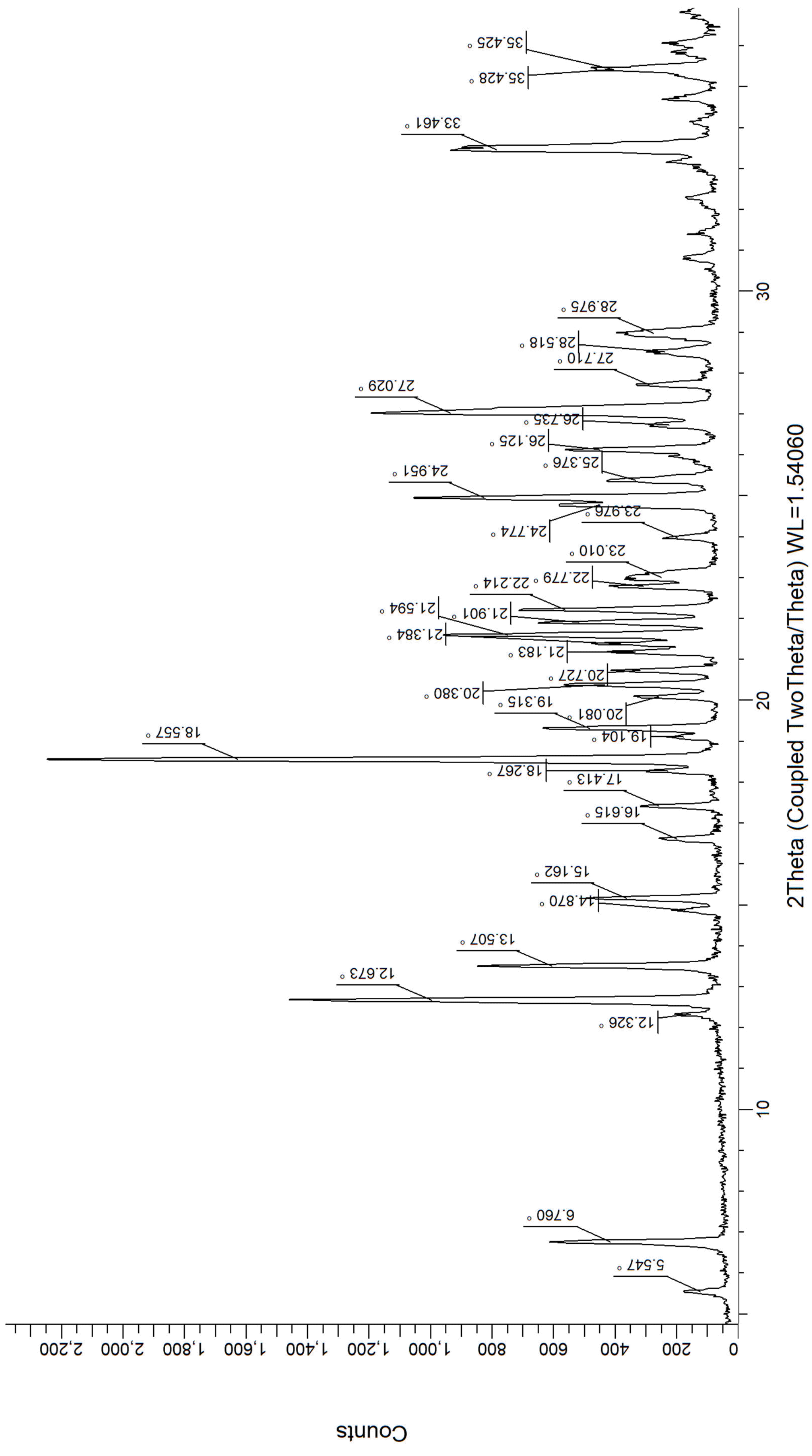
Counts
0
200
400
600
800
1,000
1,200
1,400
1,600
1,800
2,000
2,200
10
20
30
2Theta (Coupled TwoTheta/Theta) WL=1.54060
5.547 °
6.760 °
12.326 °
12.673 °
13.507 °
14.870 °
15.162 °
16.615 °
17.413 °
18.267 °
18.557 °
19.104 °
19.315 °
20.081 °
20.380 °
20.727 °
21.183 °
21.384 °
21.594 °
21.901 °
22.214 °
22.779 °
23.010 °
23.976 °
24.774 °
24.951 °
25.376 °
26.125 °
26.735 °
27.029 °
27.710 °
28.518 °
28.975 °
33.461 °
35.428 °
35.425 °

FIG. 21

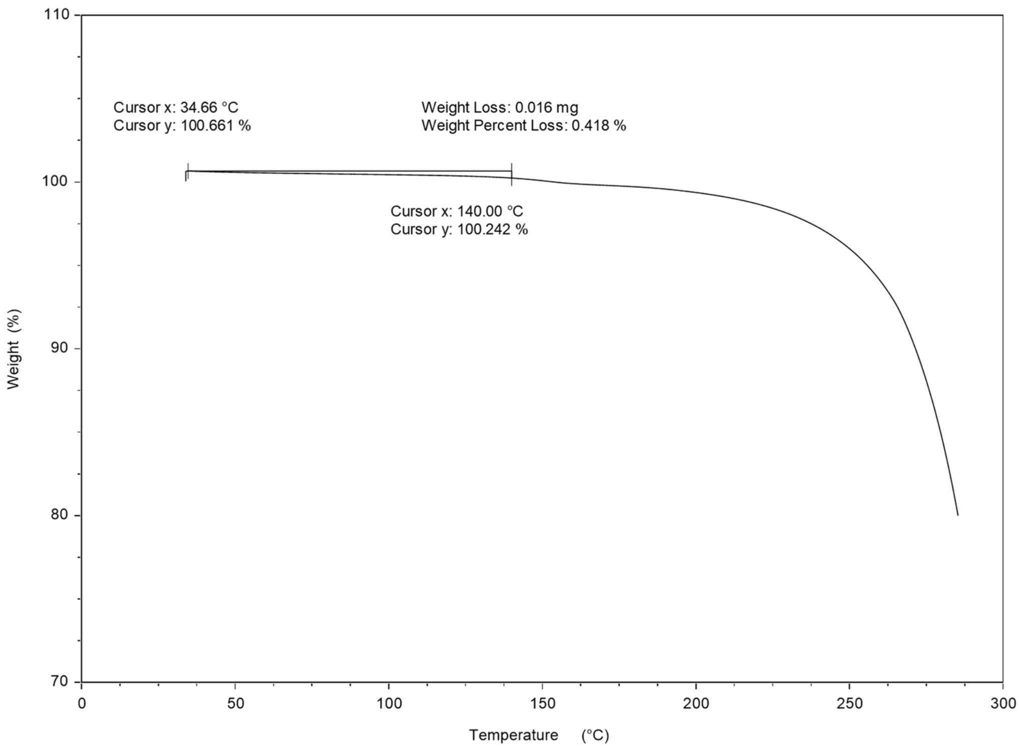

110
100
90
80
70
Weight (%)
Cursor x: 34.66 °C
Cursor y: 100.661 %
Weight Loss: 0.016 mg
Weight Percent Loss: 0.418 %
Cursor x: 140.00 °C
Cursor y: 100.242 %
0
50
100
150
200
250
300
Temperature (°C)


FIG. 22

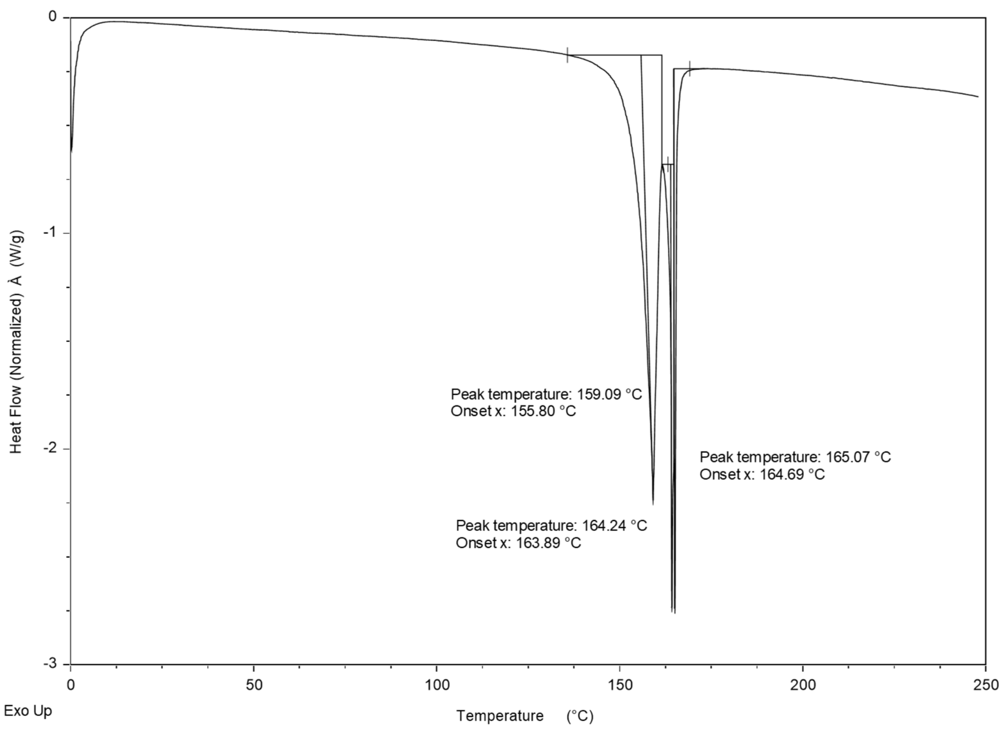

0
-1
-2
-3
Heat Flow (Normalized) Â (W/g)
Peak temperature: 159.09 °C
Onset x: 155.80 °C
Peak temperature: 165.07 °C
Onset x: 164.69 °C
Peak temperature: 164.24 °C
Onset x: 163.89 °C
0
50
100
150
200
250
Exo Up
Temperature (°C)


FIG. 23

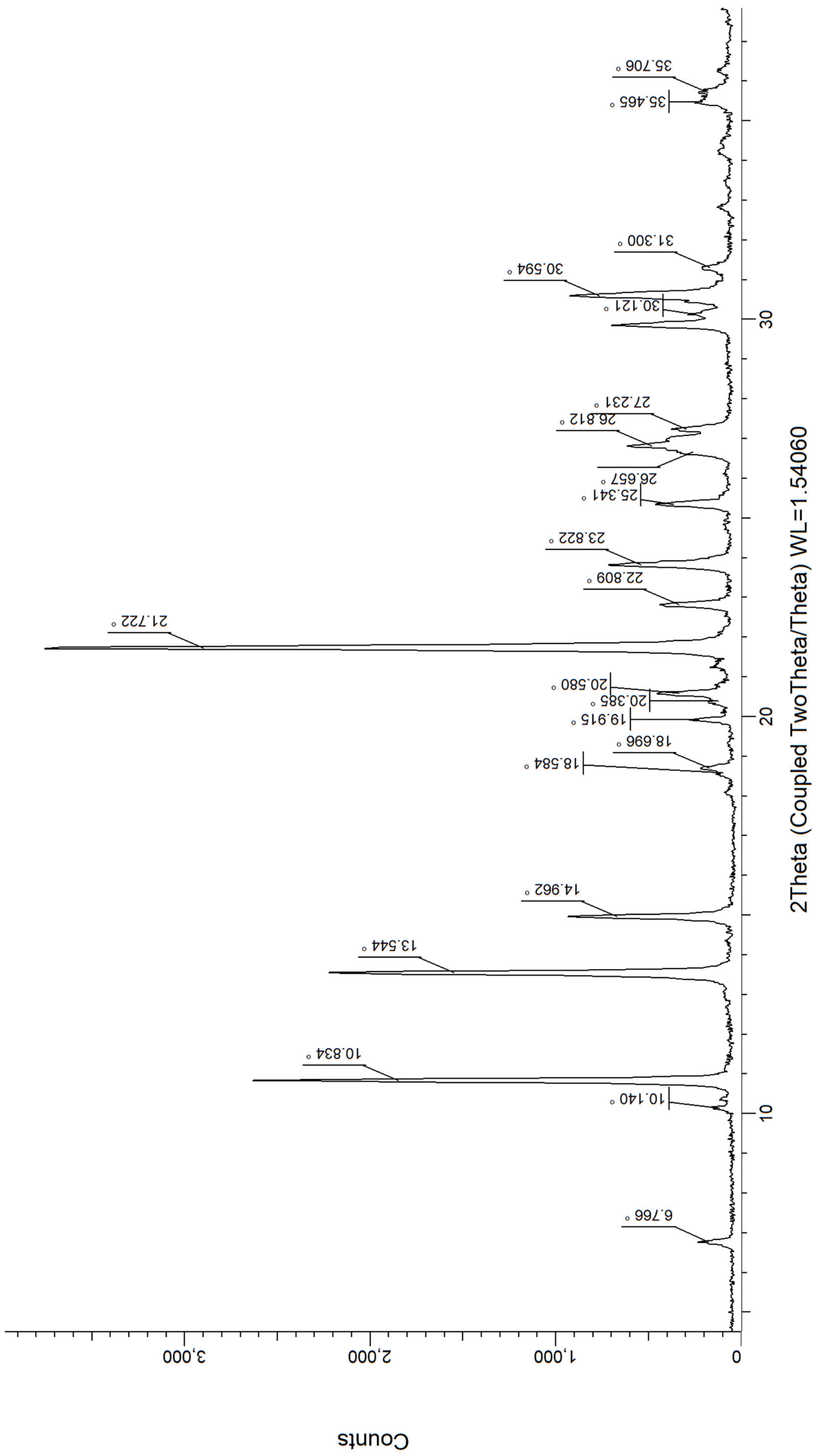
Counts
0
1,000
2,000
3,000
10
20
30
2Theta (Coupled TwoTheta/Theta) WL=1.54060
6.766 °
10.140 °
10.834 °
13.544 °
14.962 °
18.584 °
18.696 °
19.915 °
20.385 °
20.580 °
21.722 °
22.809 °
23.822 °
25.341 °
26.657 °
26.812 °
27.231 °
30.121 °
30.594 °
31.300 °
35.465 °
35.706 °

FIG. 24

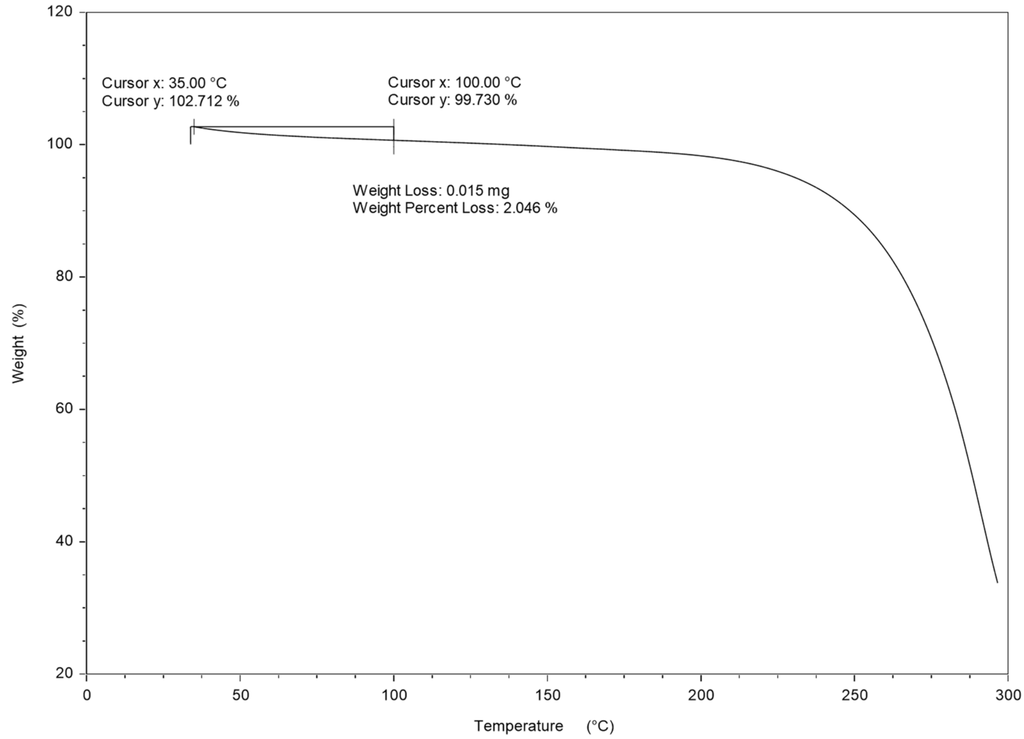
120
100
80
60
40
20
Weight (%)
Cursor x: 35.00 °C
Cursor y: 102.712 %
Cursor x: 100.00 °C
Cursor y: 99.730 %
Weight Loss: 0.015 mg
Weight Percent Loss: 2.046 %
0
50
100
150
200
250
300
Temperature (°C)

FIG. 25

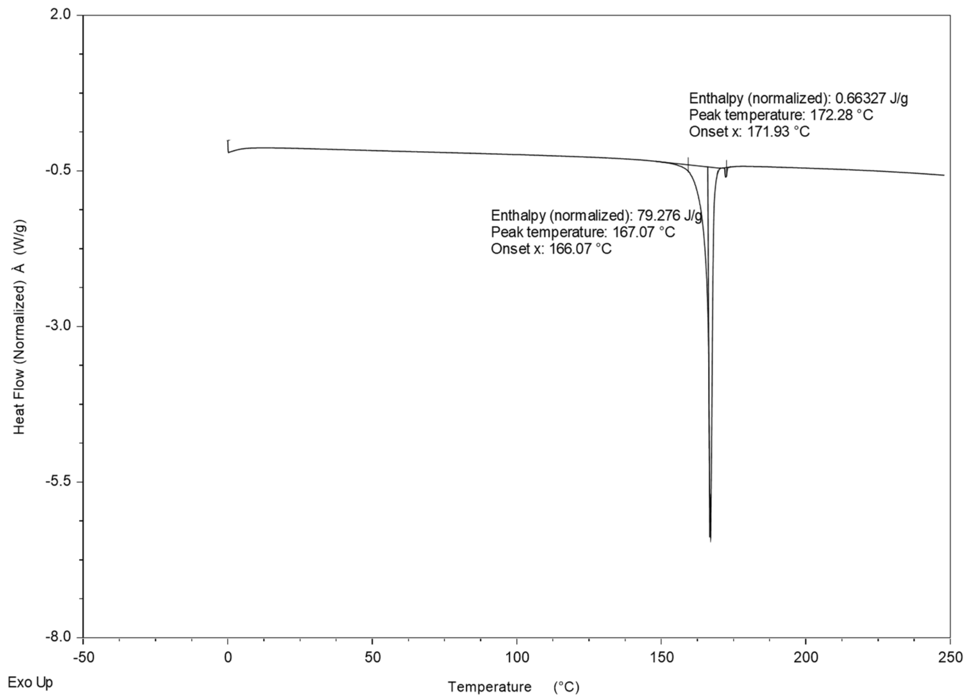
2.0
-0.5
-3.0
-5.5
-8.0
Heat Flow (Normalized) Â (W/g)
Enthalpy (normalized): 0.66327 J/g
Peak temperature: 172.28 °C
Onset x: 171.93 °C
Enthalpy (normalized): 79.276 J/g
Peak temperature: 167.07 °C
Onset x: 166.07 °C
-50
0
50
100
150
200
250
Exo Up
Temperature (°C)

FIG. 26

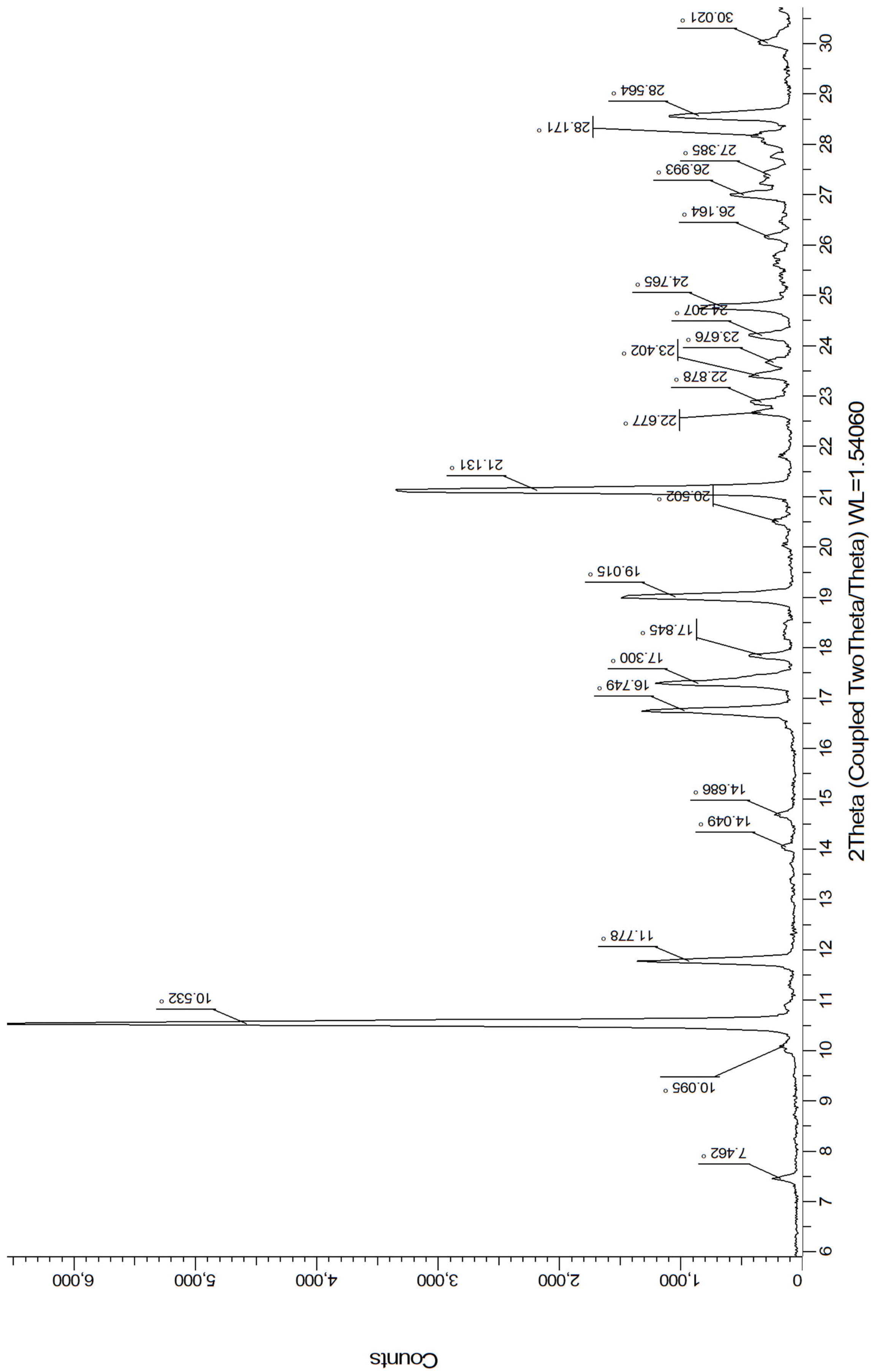
Counts
0
1,000
2,000
3,000
4,000
5,000
6,000
2Theta (Coupled TwoTheta/Theta) WL=1.54060
7.462 °
10.095 °
10.532 °
11.778 °
14.049 °
14.686 °
16.749 °
17.300 °
17.845 °
19.015 °
20.502 °
21.131 °
22.677 °
22.878 °
23.402 °
23.676 °
24.207 °
24.765 °
26.164 °
26.993 °
27.385 °
28.171 °
28.564 °
30.021 °

FIG. 27

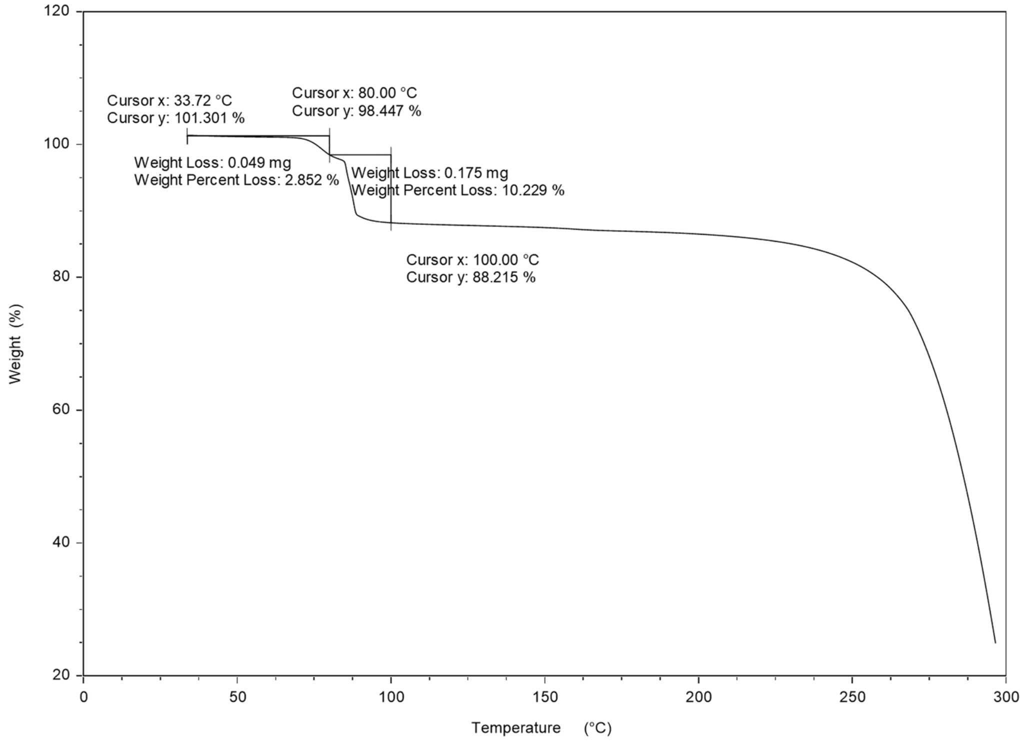
Cursor x: 33.72 °C
Cursor y: 101.301 %
Cursor x: 80.00 °C
Cursor y: 98.447 %
Weight Loss: 0.049 mg
Weight Percent Loss: 2.852 %
Weight Loss: 0.175 mg
Weight Percent Loss: 10.229 %
Cursor x: 100.00 °C
Cursor y: 88.215 %
Weight (%)
Temperature (°C)

FIG. 28

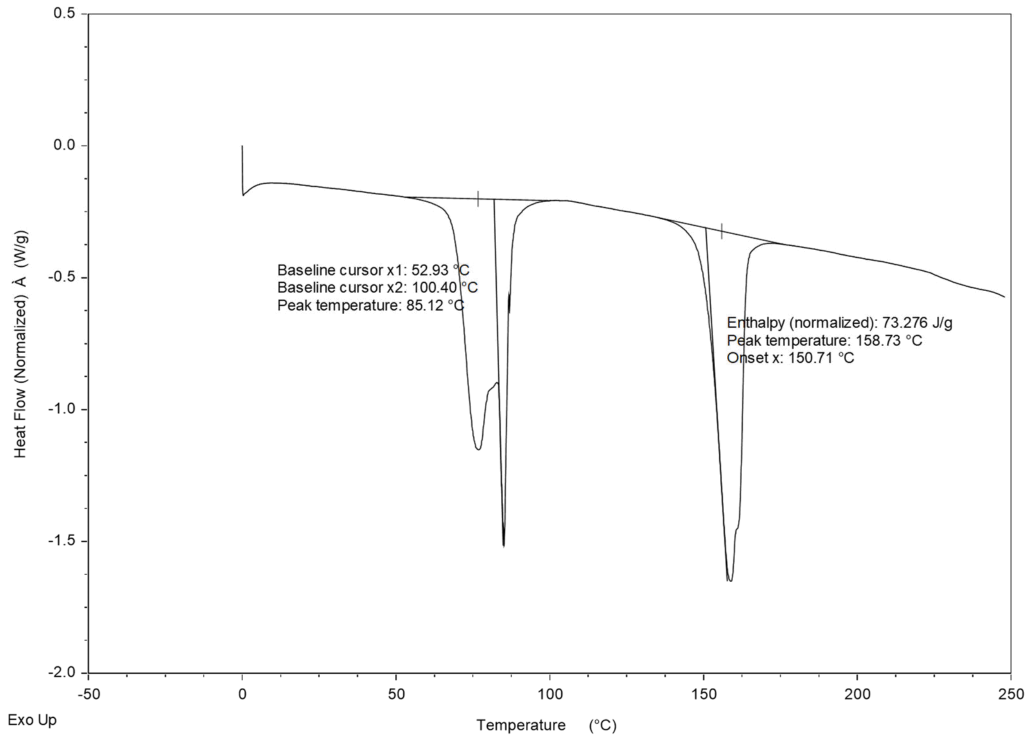
Baseline cursor x1: 52.93 °C
Baseline cursor x2: 100.40 °C
Peak temperature: 85.12 °C
Enthalpy (normalized): 73.276 J/g
Peak temperature: 158.73 °C
Onset x: 150.71 °C
Heat Flow (Normalized) Å (W/g)
Exo Up
Temperature (°C)

FIG. 29

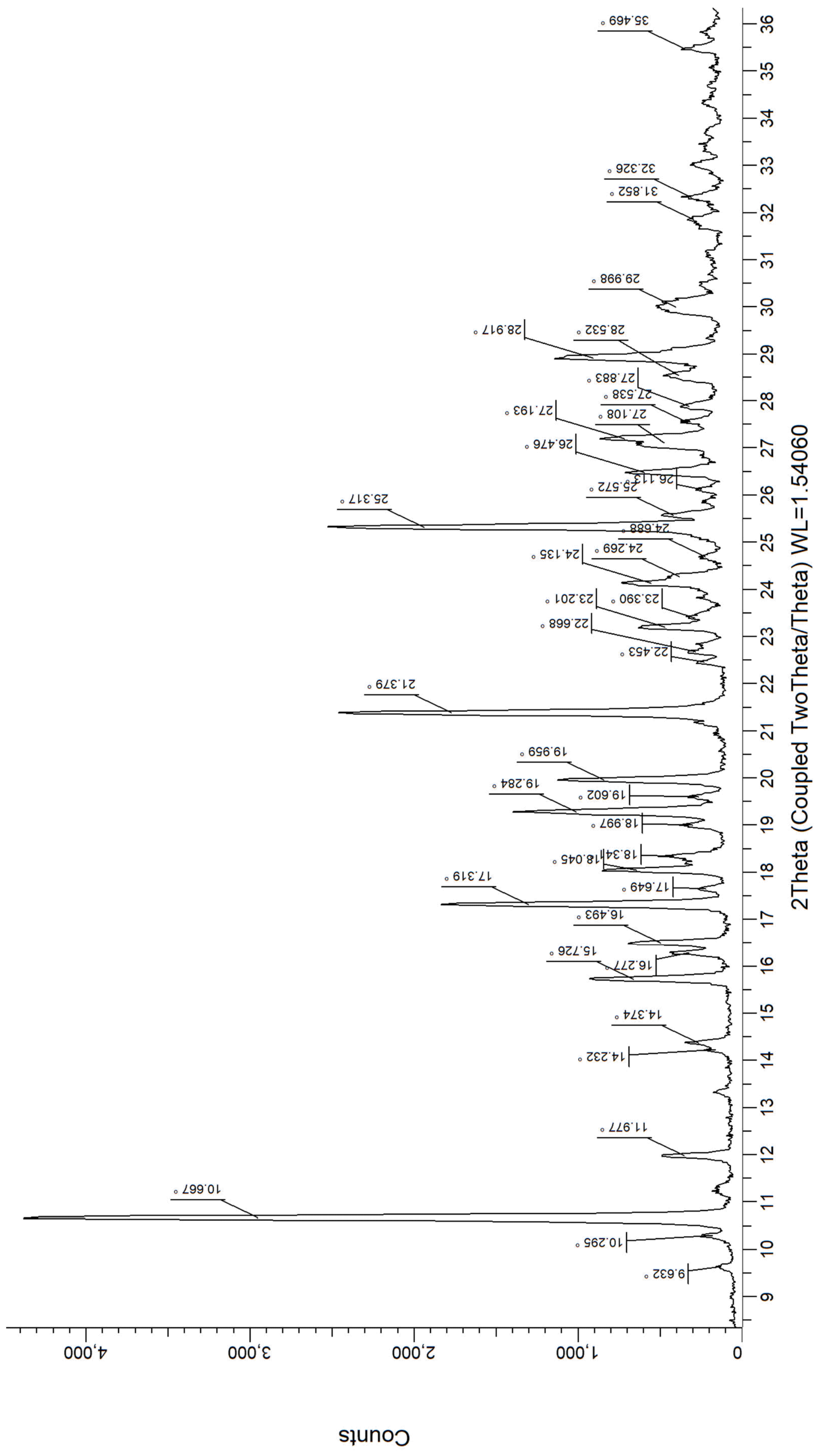
Counts
0
1,000
2,000
3,000
4,000
2Theta (Coupled TwoTheta/Theta) WL=1.54060
9.632 °
10.295 °
10.667 °
11.977 °
14.232 °
14.374 °
15.726 °
16.277 °
16.493 °
17.319 °
17.649 °
18.045 °
18.997 °
19.284 °
19.602 °
19.959 °
21.379 °
22.453 °
22.668 °
23.201 °
23.390 °
24.135 °
24.269 °
24.688 °
25.317 °
25.572 °
26.113 °
26.476 °
27.108 °
27.193 °
27.538 °
27.883 °
28.532 °
28.917 °
29.998 °
31.852 °
32.326 °
35.469 °

FIG. 30

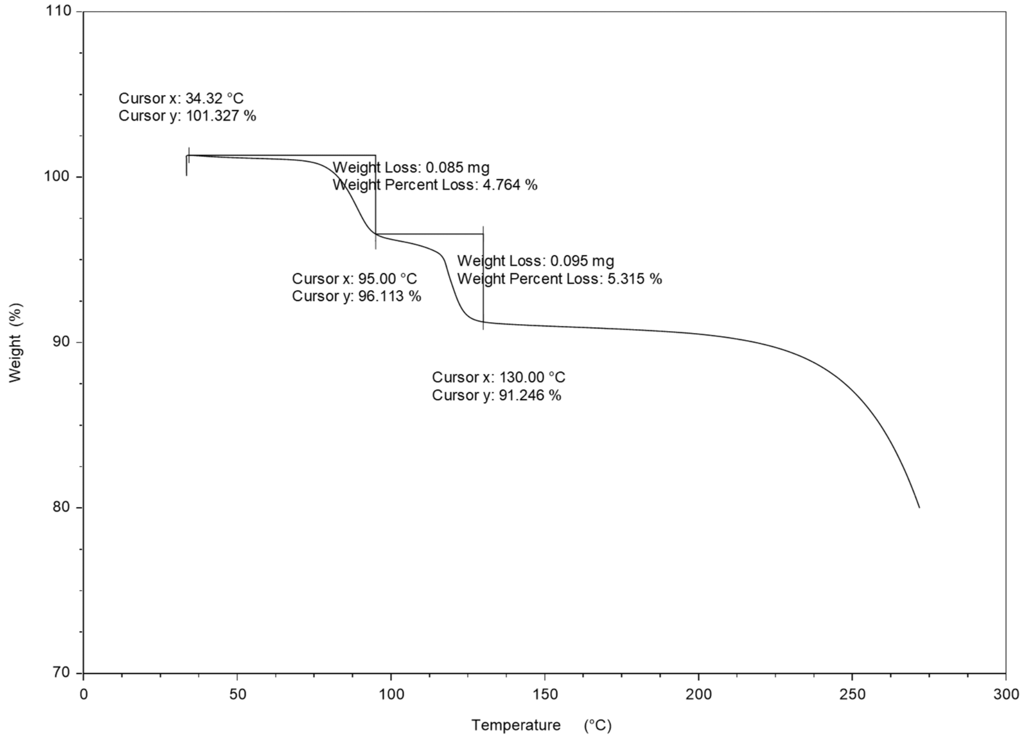
Cursor x: 34.32 °C
Cursor y: 101.327 %
Weight Loss: 0.085 mg
Weight Percent Loss: 4.764 %
Cursor x: 95.00 °C
Cursor y: 96.113 %
Weight Loss: 0.095 mg
Weight Percent Loss: 5.315 %
Cursor x: 130.00 °C
Cursor y: 91.246 %
Weight (%)
Temperature (°C)
110
100
90
80
70
0
50
100
150
200
250
300

FIG. 31

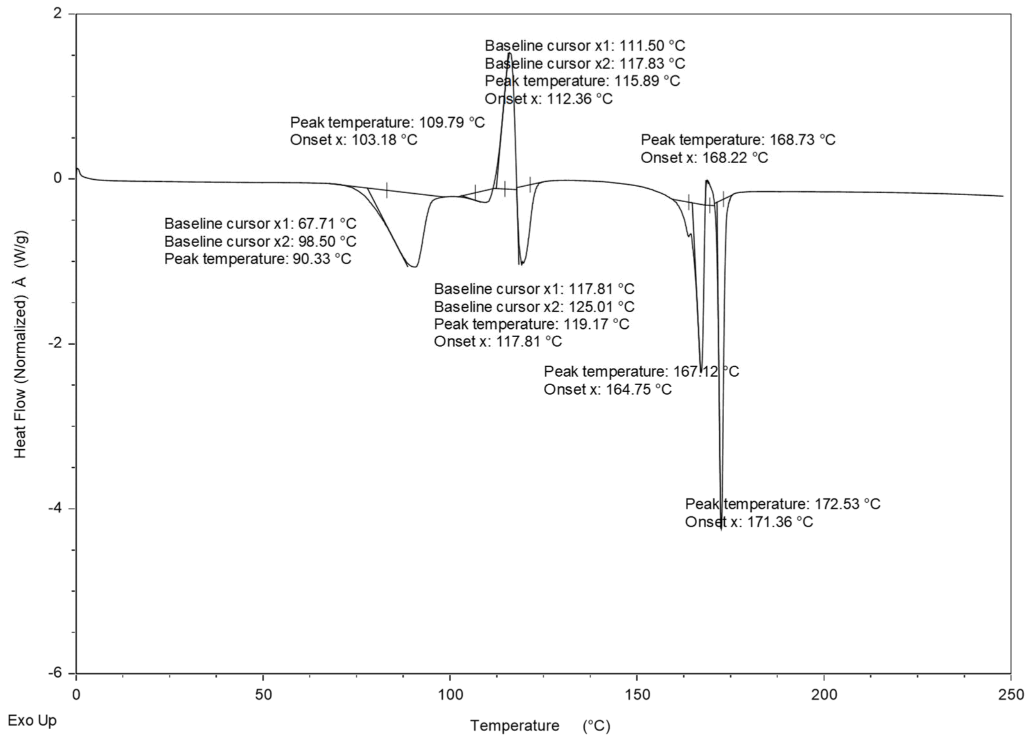
Baseline cursor x1: 111.50 °C
Baseline cursor x2: 117.83 °C
Peak temperature: 115.89 °C
Onset x: 112.36 °C
Peak temperature: 109.79 °C
Onset x: 103.18 °C
Peak temperature: 168.73 °C
Onset x: 168.22 °C
Baseline cursor x1: 67.71 °C
Baseline cursor x2: 98.50 °C
Peak temperature: 90.33 °C
Baseline cursor x1: 117.81 °C
Baseline cursor x2: 125.01 °C
Peak temperature: 119.17 °C
Onset x: 117.81 °C
Peak temperature: 167.12 °C
Onset x: 164.75 °C
Peak temperature: 172.53 °C
Onset x: 171.36 °C
Heat Flow (Normalized) Å (W/g)
Exo Up
Temperature (°C)
2
0
-2
-4
-6
0
50
100
150
200
250

FIG. 32

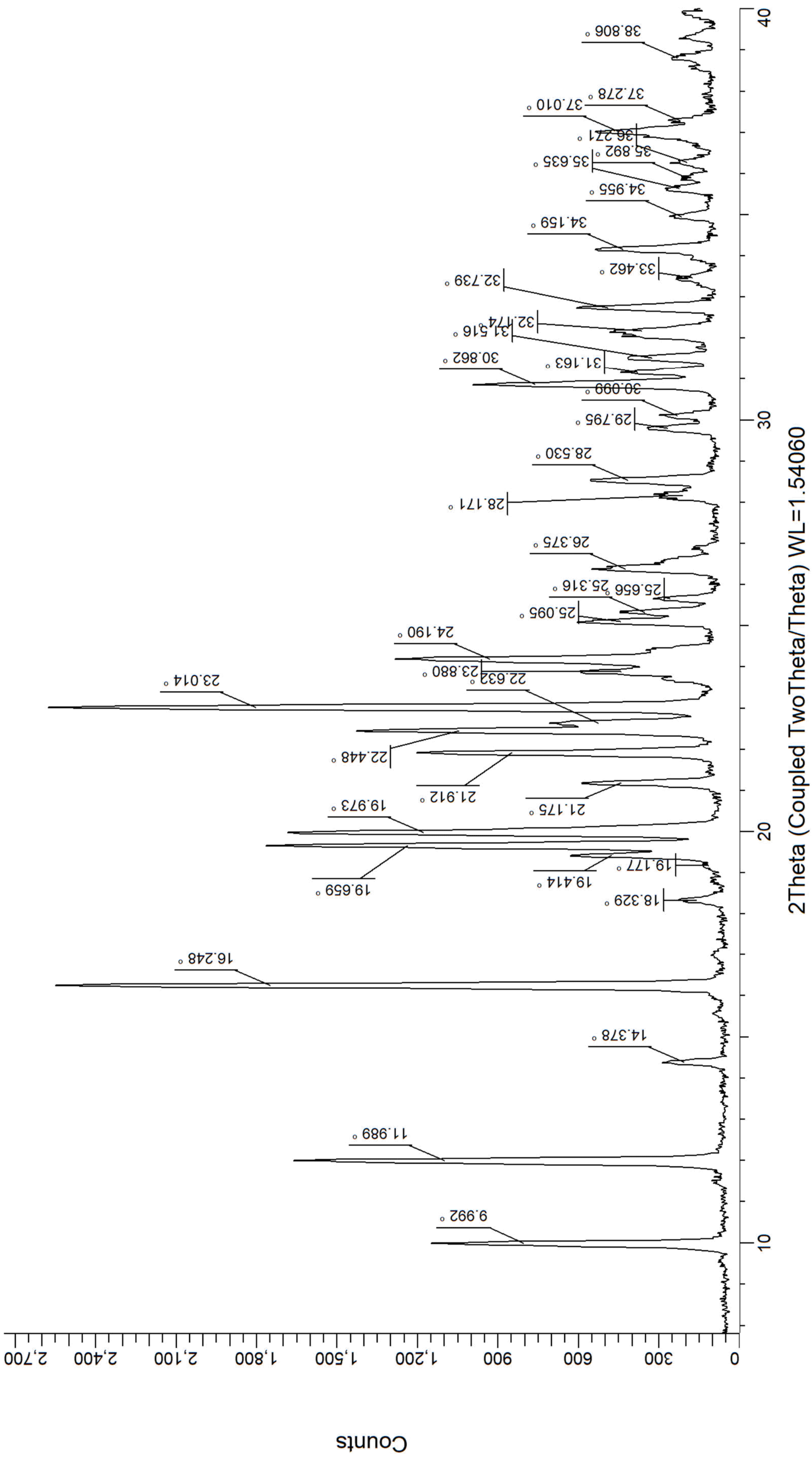

Counts
0
300
600
900
1,200
1,500
1,800
2,100
2,400
2,700
10
20
30
40
2Theta (Coupled TwoTheta/Theta) WL=1.54060
9.992 °
11.989 °
14.378 °
16.248 °
18.329 °
19.177 °
19.414 °
19.659 °
19.973 °
21.175 °
21.912 °
22.448 °
22.632 °
23.014 °
23.880 °
24.190 °
25.095 °
25.316 °
25.656 °
26.375 °
28.171 °
28.530 °
29.795 °
30.099 °
30.862 °
31.163 °
31.516 °
32.174 °
32.739 °
33.462 °
34.159 °
34.955 °
35.635 °
35.892 °
36.271 °
37.010 °
37.278 °
38.806 °


FIG. 33

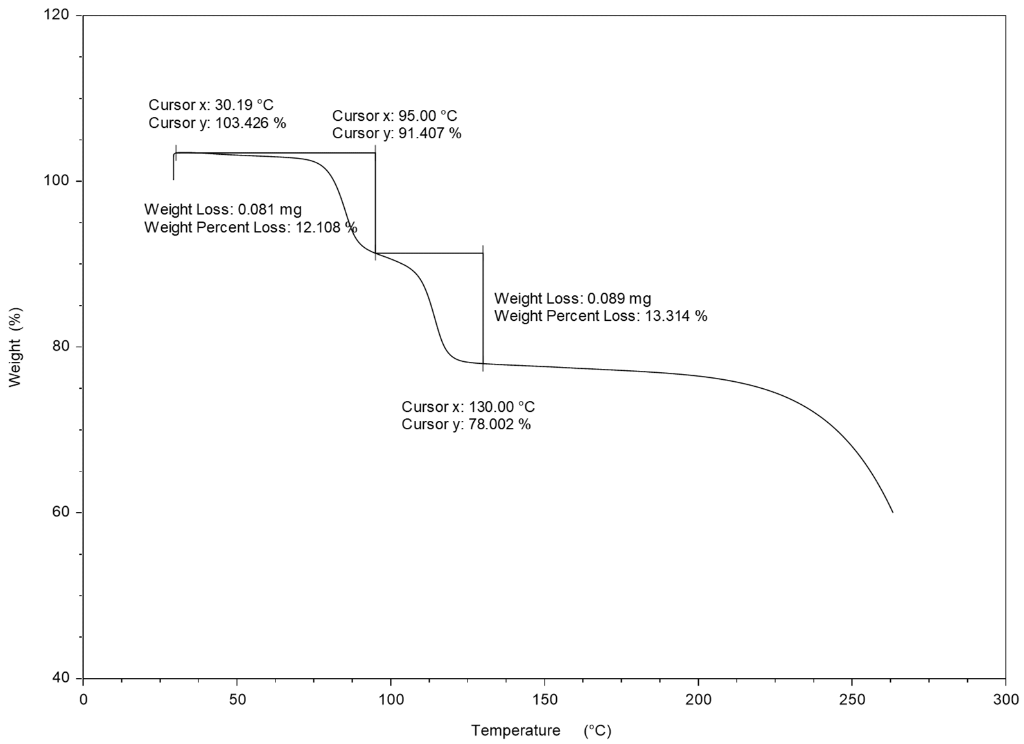
120
100
80
60
40
Weight (%)
0
50
100
150
200
250
300
Temperature (°C)
Cursor x: 30.19 °C
Cursor y: 103.426 %
Cursor x: 95.00 °C
Cursor y: 91.407 %
Weight Loss: 0.081 mg
Weight Percent Loss: 12.108 %
Weight Loss: 0.089 mg
Weight Percent Loss: 13.314 %
Cursor x: 130.00 °C
Cursor y: 78.002 %

FIG. 34

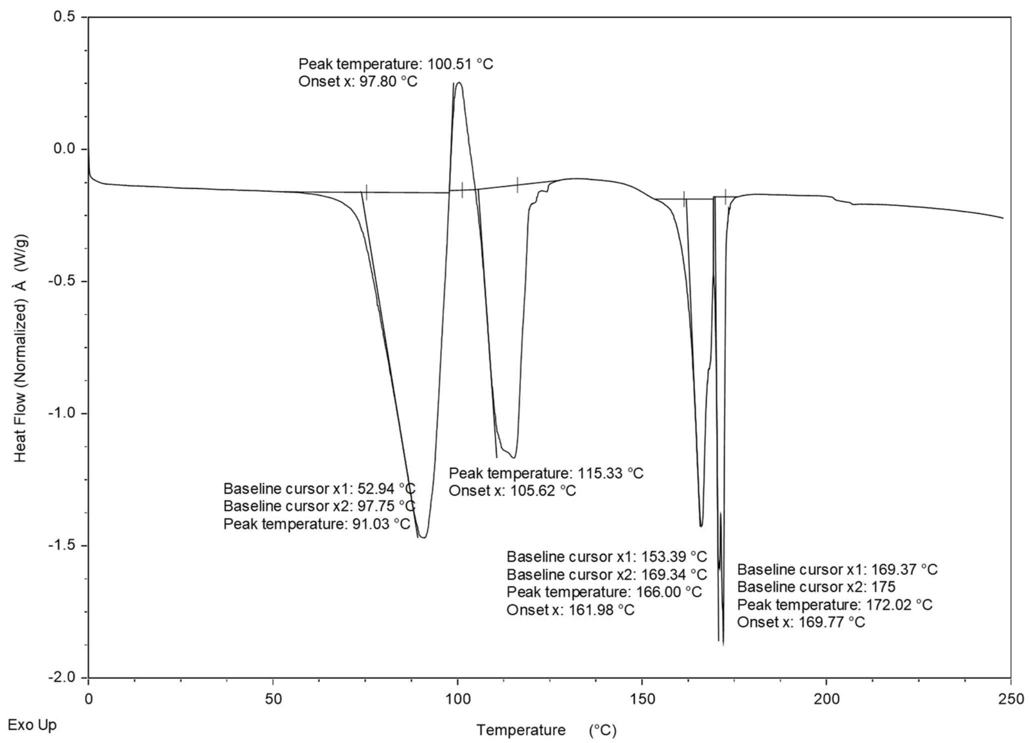
0.5
0.0
-0.5
-1.0
-1.5
-2.0
Heat Flow (Normalized) Â (W/g)
0
50
100
150
200
250
Exo Up
Temperature (°C)
Peak temperature: 100.51 °C
Onset x: 97.80 °C
Baseline cursor x1: 52.94 °C
Baseline cursor x2: 97.75 °C
Peak temperature: 91.03 °C
Peak temperature: 115.33 °C
Onset x: 105.62 °C
Baseline cursor x1: 153.39 °C
Baseline cursor x2: 169.34 °C
Peak temperature: 166.00 °C
Onset x: 161.98 °C
Baseline cursor x1: 169.37 °C
Baseline cursor x2: 175
Peak temperature: 172.02 °C
Onset x: 169.77 °C

FIG. 35

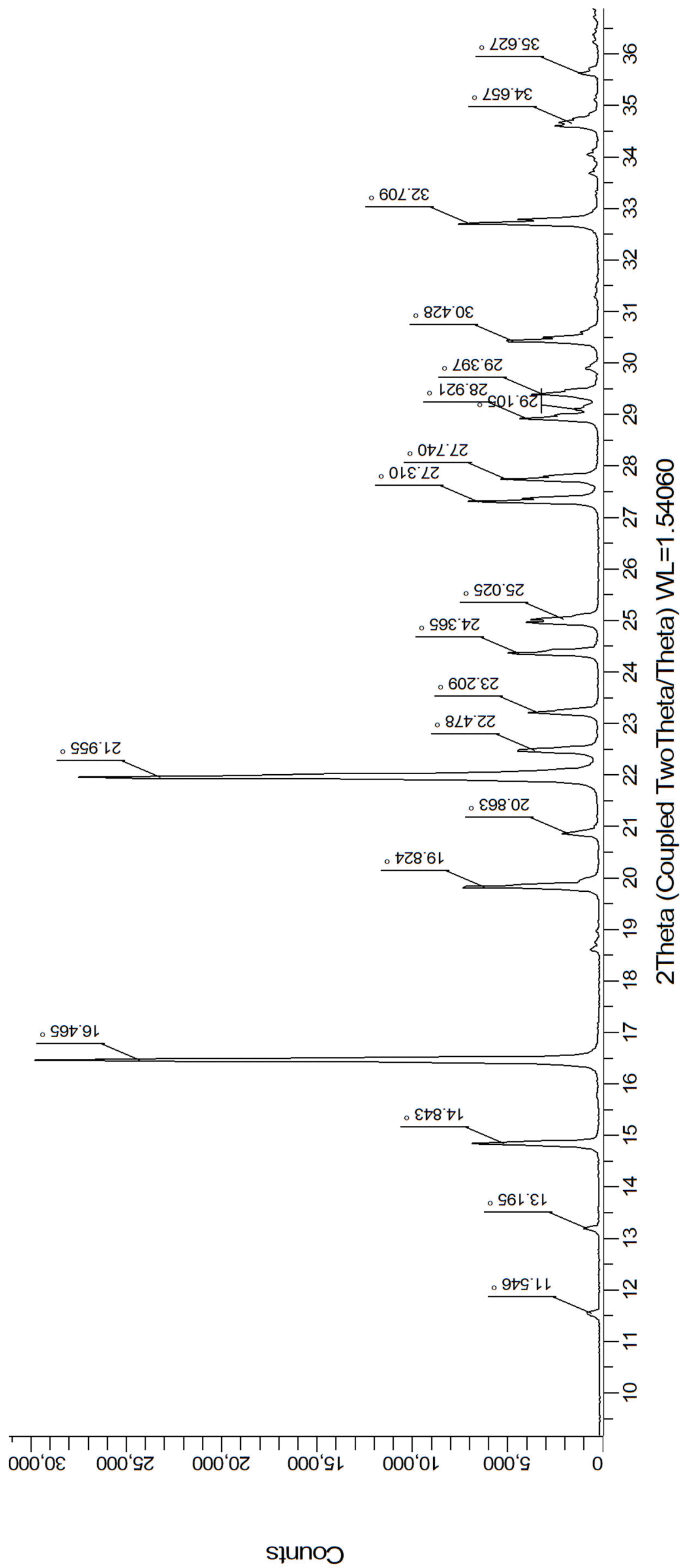
Counts
0
5,000
10,000
15,000
20,000
25,000
30,000
2Theta (Coupled TwoTheta/Theta) WL=1.54060
10
11
12
13
14
15
16
17
18
19
20
21
22
23
24
25
26
27
28
29
30
31
32
33
34
35
36
11.546 °
13.195 °
14.843 °
16.465 °
19.824 °
20.863 °
21.955 °
22.478 °
23.209 °
24.365 °
25.025 °
27.310 °
27.740 °
28.921 °
29.105 °
29.397 °
30.428 °
32.709 °
34.657 °
35.627 °

FIG. 36

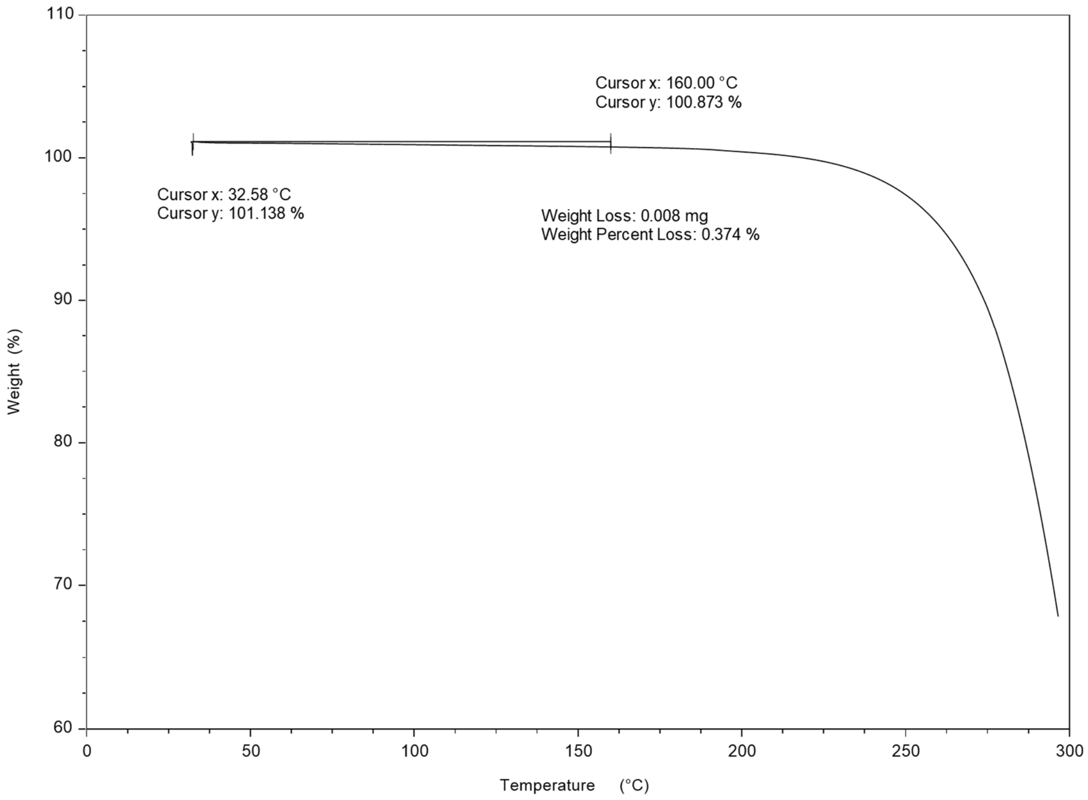
Cursor x: 160.00 °C
Cursor y: 100.873 %
Cursor x: 32.58 °C
Cursor y: 101.138 %
Weight Loss: 0.008 mg
Weight Percent Loss: 0.374 %
110
100
90
80
70
60
0
50
100
150
200
250
300
Weight (%)
Temperature (°C)

FIG. 37

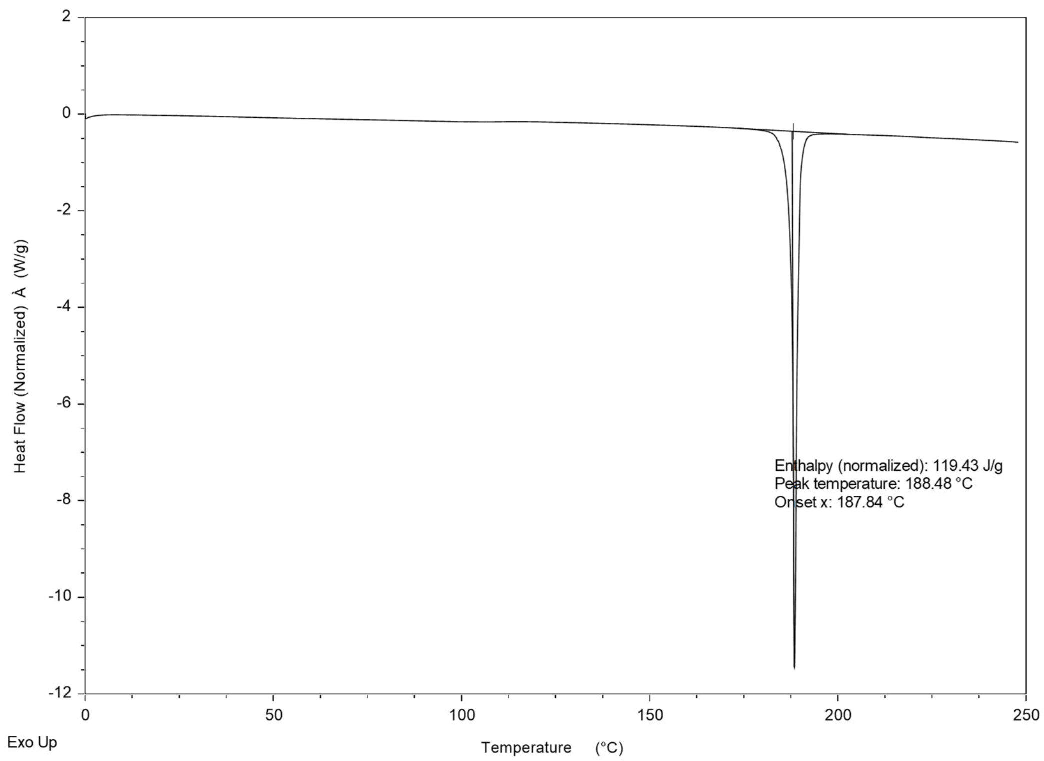
Enthalpy (normalized): 119.43 J/g
Peak temperature: 188.48 °C
Onset x: 187.84 °C
2
0
-2
-4
-6
-8
-10
-12
0
50
100
150
200
250
Heat Flow (Normalized) À (W/g)
Exo Up
Temperature (°C)

FIG. 38

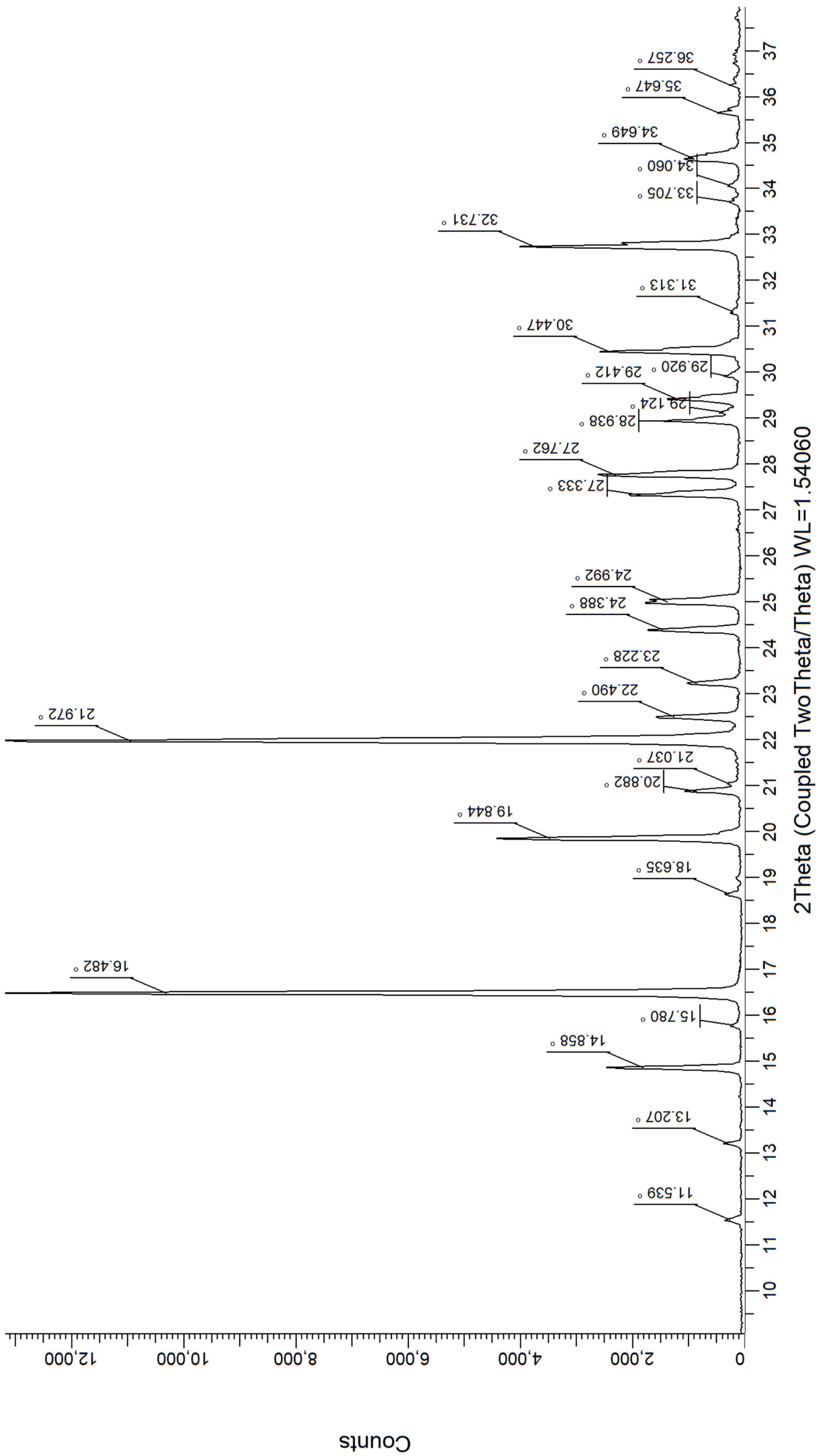
Counts
0
2,000
4,000
6,000
8,000
10,000
12,000
2Theta (Coupled TwoTheta/Theta) WL=1.54060
11.539 °
13.207 °
14.858 °
15.780 °
16.482 °
18.635 °
19.844 °
20.882 °
21.037 °
21.972 °
22.490 °
23.228 °
24.388 °
24.992 °
27.333 °
27.762 °
28.938 °
29.124 °
29.412 °
29.920 °
30.447 °
31.313 °
32.731 °
33.705 °
34.060 °
34.649 °
35.647 °
36.257 °

FIG. 39

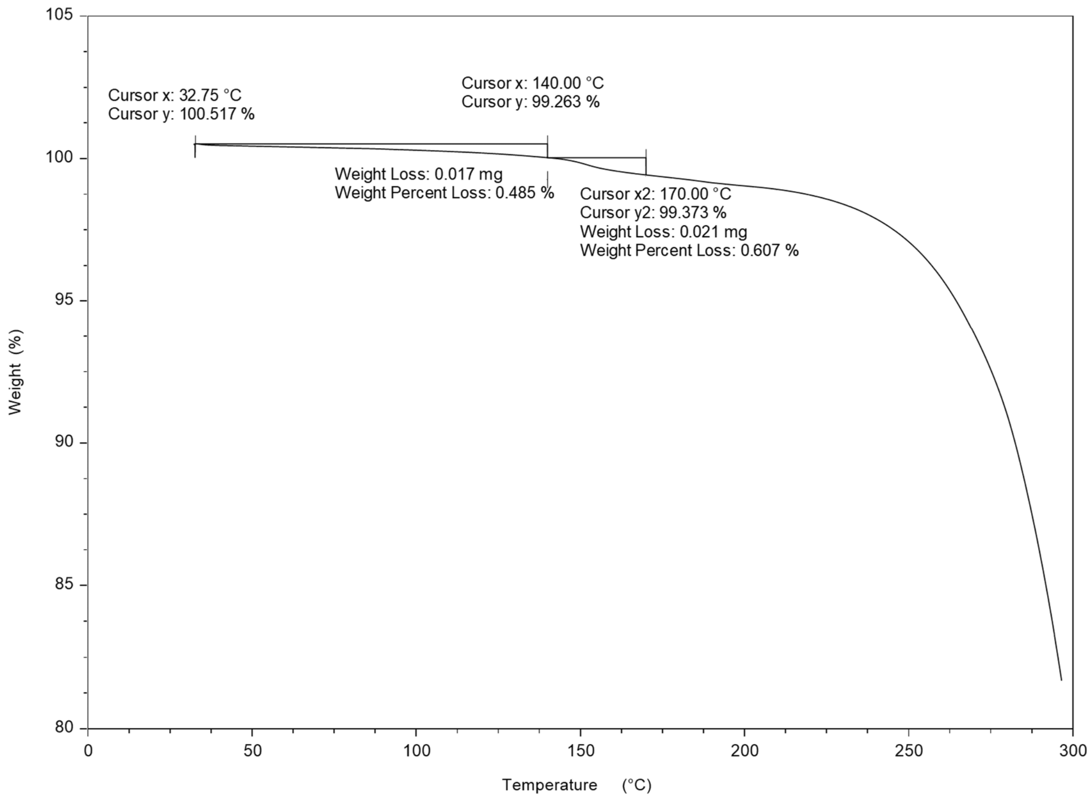
Cursor x: 32.75 °C
Cursor y: 100.517 %
Cursor x: 140.00 °C
Cursor y: 99.263 %
Weight Loss: 0.017 mg
Weight Percent Loss: 0.485 %
Cursor x2: 170.00 °C
Cursor y2: 99.373 %
Weight Loss: 0.021 mg
Weight Percent Loss: 0.607 %
105
100
95
90
85
80
Weight (%)
0
50
100
150
200
250
300
Temperature (°C)

FIG. 40

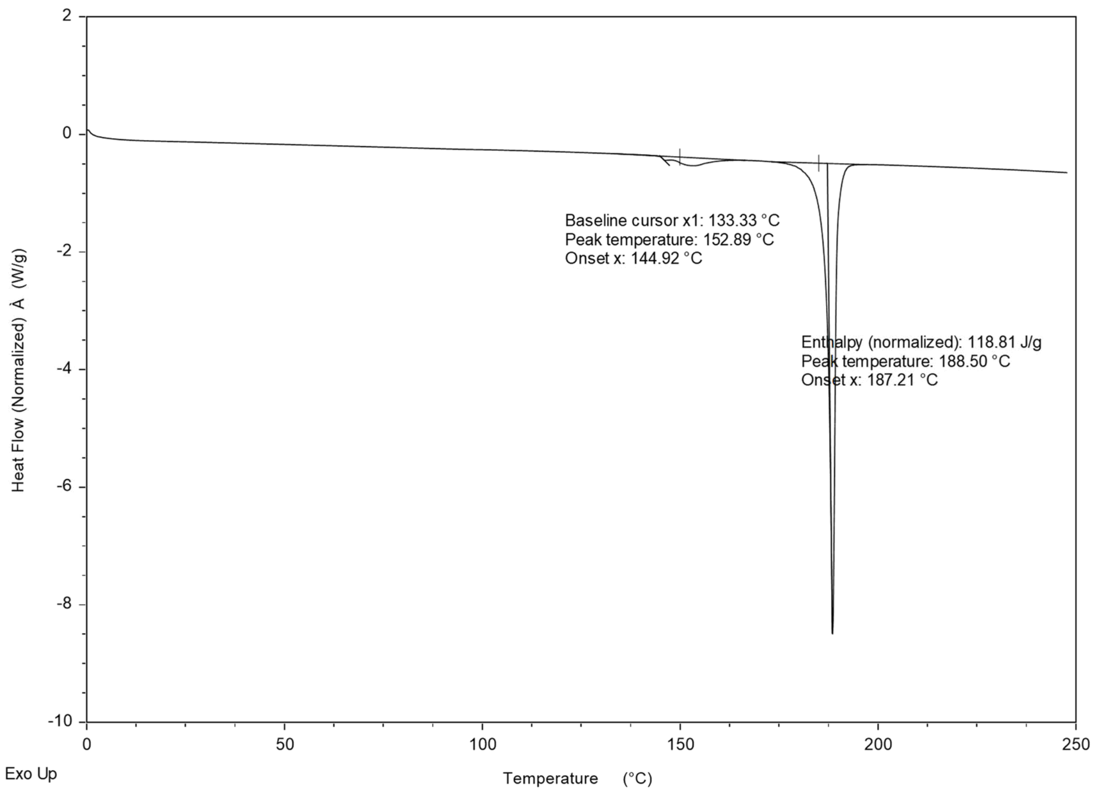
2
0
-2
-4
-6
-8
-10
Heat Flow (Normalized) Â (W/g)
Baseline cursor x1: 133.33 °C
Peak temperature: 152.89 °C
Onset x: 144.92 °C
Enthalpy (normalized): 118.81 J/g
Peak temperature: 188.50 °C
Onset x: 187.21 °C
0
50
100
150
200
250
Exo Up
Temperature (°C)

FIG. 41

**INTERNATIONAL SEARCH REPORT**

International application No.
**PCT/CN2024/137995**

**A. CLASSIFICATION OF SUBJECT MATTER**

C07D209/88(2006.01)i; A61K31/403(2006.01)i; A61P35/00(2006.01)i; A61P17/00(2006.01)i

According to International Patent Classification (IPC) or to both national classification and IPC

**B. FIELDS SEARCHED**

Minimum documentation searched (classification system followed by classification symbols)

IPC:C07D; A61K; A61P

Documentation searched other than minimum documentation to the extent that such documents are included in the fields searched

Electronic data base consulted during the international search (name of data base and, where practicable, search terms used)

DWPI, CNTXT, CNKI, REGISTRY (STN), CAPLUS (STN): 苏州湃芮生物, RNA修饰, 甲基化, m6A, 调控, 皮肤, 肿瘤, 结晶, 晶体, 晶型, 咔唑, 甲酰胺, RNA modification, methylation, regulat+, skin, tumor, tomour, cancer, crystalline, carbazolyl, carbamido, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2024236025 A1 (AOP ORPHAN IP AG) 21 November 2024 (2024-11-21) description, pages 5-16 | 1-18 |
| X | WO 2008019825 A1 (SANTHERA PHARMACEUTICALS (SCHWEIZ) AG et al.) 21 February 2008 (2008-02-21) description, pages 4, 6-7, 11, and 24-25 | 1-18 |
| A | WO 2008019825 A1 (SANTHERA PHARMACEUTICALS (SCHWEIZ) AG et al.) 21 February 2008 (2008-02-21) description, pages 4, 6-7, 11, and 24-25 | 19-20 |
| X | NAPPER, A. D. et al. "Discovery of Indoles as Potent and Selective Inhibitors of the Deacetylase SIRT1" *J. Med. Chem.*, Vol. 48, No. 25, 09 November 2005 (2005-11-09), pages 8045-8054 | 1-15 |
| A | WO 2010054382 A1 (ELIXIR PHARMACEUTICALS, INC. et al.) 14 May 2010 (2010-05-14) description, pages 2-3 and 5 | 1-20 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 February 2025** | **24 February 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/137995**

**C. DOCUMENTS CONSIDERED TO BE RELEVANT**

| Category* | Citation of document, with indication, where appropriate, of the relevant passages | Relevant to claim No. |
|---|---|---|
| A | WO 2006031894 A2 (ELIXIR PHARMACEUTICALS, INC. et al.) 23 March 2006 (2006-03-23)<br>description, pages 1-2 | 1-20 |
| A | CN 103958467 A (SIENA BIOTECH S.P.A.) 30 July 2014 (2014-07-30)<br>description, pages 6-8, and embodiment 5 and 7 | 1-20 |

Form PCT/ISA/210 (second sheet) (July 2022)

**INTERNATIONAL SEARCH REPORT**
**Information on patent family members**

International application No.
**PCT/CN2024/137995**

| Patent document cited in search report | Publication date (day/month/year) | Patent family member(s) | Publication date (day/month/year) |
|---|---|---|---|
| WO 2024236025 A1 | 21 November 2024 | None | |
| WO 2008019825 A1 | 21 February 2008 | None | |
| WO 2010054382 A1 | 14 May 2010 | None | |
| WO 2006031894 A2 | 23 March 2006 | None | |
| CN 103958467 A | 30 July 2014 | SI 2768807 T1 | 30 September 2016 |
| | | US 2016304455 A1 | 20 October 2016 |
| | | US 10329254 B2 | 25 June 2019 |
| | | AU 2012324824 A1 | 08 May 2014 |
| | | AU 2012324824 B2 | 11 August 2016 |
| | | WO 2013057258 A1 | 25 April 2013 |
| | | PL 2768807 T3 | 31 March 2017 |
| | | HRP 20160918 T1 | 23 September 2016 |
| | | HUE 029282 T2 | 28 February 2017 |
| | | IL 232058 A0 | 28 May 2014 |
| | | IL 232058 A | 29 February 2016 |
| | | ES 2585077 T3 | 03 October 2016 |
| | | HK 1200452 A1 | 07 August 2015 |
| | | EP 2768807 A1 | 27 August 2014 |
| | | EP 2768807 B1 | 04 May 2016 |
| | | BR 112014009297 A2 | 11 April 2017 |
| | | JP 2014530839 A | 20 November 2014 |
| | | JP 6142459 B2 | 07 June 2017 |
| | | PT 2768807 T | 08 August 2016 |
| | | US 2014303382 A1 | 09 October 2014 |
| | | CA 2852797 A1 | 25 April 2013 |
| | | CA 2852797 C | 04 February 2020 |
| | | AR 088377 A1 | 28 May 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311694967X


### Non-patent literature cited in the description


- **FRYE M. et al.** *Science*, 2018, vol. 361, 2073-2092
- **YANG C. et al.** *Cell Death & Disease*, 2020, vol. 11, 960
- **MEYER K.D.** ; **JAFFREY S.R.** *Nature Review Molecular Cell Biology*, 2014, vol. 15, 313-326
- **HSU P.J. et al.** *Journal of Biological Chemistry*, 2019, vol. 294, 19889-19895
- **YAO Y. et al.** *FASEB Journal*, 2019, vol. 33, 7529-7544
- **LE ZHANGHUI**. Preliminary Study on the Pathogenesis of LncRNA and RNA m6A Methylation in Acral Melanoma. *Dissertation*, 2019
- **LI T. D. et al.** *Cancer Cell*, 2020, vol. 20, 239
- **CUI Y. H. et al.** *Nature Communications*, 2021, vol. 12, 2183
- **KUMPER M. et al.** *Am. J. Physiol. Cell. Physiol.*, 2022, vol. 323 (4), 1290-1303